Europäisches Patentamt

European Patent Office

Office européen des brevets

(19)

(11) **EP 1 116 690 A2**

(12) ## EUROPÄISCHE PATENTANMELDUNG

(43) Veröffentlichungstag:
    **18.07.2001 Patentblatt 2001/29**

(51) Int Cl.[7]: **C01B 23/00**, A61K 49/00,
    F04B 37/14

(21) Anmeldenummer: **01100057.7**

(22) Anmeldetag: **10.01.2001**

(84) Benannte Vertragsstaaten:
    **AT BE CH CY DE DK ES FI FR GB GR IE IT LI LU MC NL PT SE TR**
    Benannte Erstreckungsstaaten:
    **AL LT LV MK RO SI**

(30) Priorität: **11.01.2000 DE 10000675**

(71) Anmelder: **Otten, Ernst-Wilhelm, Prof. Dr.**
    **55127 Mainz (DE)**

(72) Erfinder:
    • **Deninger, Anselm, Dr.**
      **55124 Mainz (DE)**
    • **Ebert, Michael, Dr.**
      **74379 Ingersheim (DE)**

    • **Schmiedeskamp, Jörg**
      **65195 Wiesbaden (DE)**
    • **Hasse, Jan**
      **55120 Mainz (DE)**
    • **Heil, Werner, Prof. Dr.**
      **55276 Oppenheim (DE)**
    • **Otten, Ernst-W., Prof. Dr.**
      **55127 Mainz (DE)**
    • **Surkau, Reinhard, Dr.**
      **82256 Fürstenfeldbruck (DE)**

(74) Vertreter: **Dr. Weitzel & Partner**
    **Friedenstrasse 10**
    **89522 Heidenheim (DE)**

(54) **Element, Vorrichtung und Verfahren zur hochproduktiven Erzeugung von hoch kernspinpolarisiertem Helium-3 Gas**

(57)     Die Erfindung betrifft ein Element mit wenigstens einem beweglichen Bauteil, insbesondere Kompressoren oder Ventile für eine Vorrichtung zur Erzeugung polarisierter Gase, insbesondere von $^3$He-Gas mit

einem Gehäuse
einem im Gehäuse ausgebildeten ersten Raum, der in leitender Verbindung
mit der Umgebung steht,

einem zweiten Raum, der in leitender Verbindung mit einem abgeschlossenen System umfassend das polarisierte Gas steht.

Die Erfindung ist dadurch gekennzeichnet, daß zwischen erstem und zweitem Raum mindestens ein Zwischenraum ausgebildet wird, so daß das Eindringen von Verunreinigungen in den zweiten Raum, der in Verbindung mit dem abgeschlossenen System steht, weitgehend vermieden wird.

Fig. 4a

EP 1 116 690 A2

## Beschreibung

Die Erfindung umfaßt ein Element, eine Vorrichtung sowie ein Verfahren zur Erzeugung von polarisierten Gasen, insbesondere kernspinpolarisiertem Helium-3 Gas ($^3$He) genannt "$^3$He-Polarisator". Sie zeichnen sich aus durch gleichzeitiges Erzielen hoher Produktionsraten an polarisierten Atomen und hohen Endpolarisationen.

Kernspinpolarisiertes $^3$He-Gas wird für eine große Vielfalt an physikalischen Forschungsexperimenten eingesetzt, wie sie beispielsweise in den Veröffentlichungen

J. Becker, H. G. Andresen, J. R. M. Annand, K. Aulenbacher, K. Beuchel, J. Blume-Werry, Th. Dombo, P. Drescher, M. Ebert, D. Eyl, A. Frey, P. Grabmayr, T. Großmann, P. Hartmann, T. Hehl, W. Heil, C. Herberg, J. Hoffmann, J. D. Kellie, F. Klein, K. Livingston, M. Leduc, M. Meyerhoff, H. Möller, Ch. Nachtigall, A. Natter, M. Ostrick, E. W. Otten, R. O. Owens, S. Plützer, E. Reichert, D. Rohe, M. Schäfer, H. Schmieden, R. Sprengard, M. Steigerwald, K.-H. Steffens, R. Surkau, Th. Walcher, R. Watson and E. Wilms; "Determination of the Neutron Electric Form Factor from the Reaction 3He(e; e'n') at Medium Momentum Transfer"; Eur. Phys. J. A 6, (1999) 329 - 344

D. Rohe, P. Bartsch, D. Baumann, J. Becker, J. Bermuth, K. Bohinc, R. Böhm, S. Buttazzoni, T. Caprano, N. Clawiter, A. Deninger, S. Derber, M. Ding, M. Distler, A. Ebbes, M. Ebert, I. Ewald, J. Friedrich, J. M. Friedrich, R. Geiges, T. Großmann, M. Hauger, W. Heil, A. Honegger, P. Jennewein, J. Jourdan, M. Kahrau, A. Klein, M. Kohl, K. W. Krygier, G. Kubon, A. Liesenfeld, H. Merkel, K. Merle, P. Merle, M. Mühlbauer, U. Müller, R. Neuhausen, E. W. Otten, Th. Petitjean, Th. Pospischil, M. Potokar, G. Rosner, H. Schmieden, I. Sick, S. Širca, R. Surkau, A. Wagner, Th. Walcher, G. Warren, M. Weis, H. Woehrle, M. Zeier; "Measurement of the neutron electric form factor Gen at 0.67 (GeV/c)2 via ", Phys. Rev. Lett. 83 (1999) 4257

W. Heil, J. Dreyer, D. Hofmann, H. Humblot, E.-Levievre-Berna, F. Tasset; "$^3$He neutron spin-filter"; Physica B 267-268 (1999) 328-335).

beschrieben werden.

Für medizinische Anwendungen, werden neuerdings neben dem üblichen Protonen ($^1$H) auch andere kernspinpolarisierte Isotope, wie $^3$He und Xenon-129 ($^{129}$Xe), für den Einsatz in der Kernspintomographie (MRT) diskutiert. Insbesondere zur Untersuchung der Ventilation der Lunge, wie sie beispielsweise in den Veröffentlichungen

P. Bachert, L.R. Schad, M. Bock, M.V. Knopp, M.Ebert, T. Großmann, W. Heil, D. Hofmann, R. Surkau, E.W. Otten; "Nuclear Magnetic Resonance Imaging of Airways in Humans with Use of Hyperpolarized $^3$He "; MRM 36 (1996) 192 - 196;

M. Ebert, T. Großmann, W. Heil, E.W. Otten, R. Surkau, M. Leduc, P. Bachert, M.V. Knopp, L.R. Schad, M. Thelen; "Nuclear magnetic resonance imaging with hyperpolarized $^3$He"; THE LANCET, 347 (1996) 1297- 1299;

H.-U. Kauczor, R. Surkau, T. Roberts; "MRI using hyperpolarized noble gases"; Eur. Radiol. (8) (1998) 820 - 827,

J.P. Mugler, B. Driehuys, J.R. Brookeman, G.D. Cates, S.S. Berr, R.G. Bryant, T.M. Daniel, E.E. de Lange, J.H. Downs Jr, C.J. Erickson, W. Happer, D.P. Hinton, N.F. Kassel, T. Maier, C.D. Phillips, B.T. Saam, K.L. Sauer, M.E. Wagshul; "MR Imaging and Spectroscopy Using Hyperpolarized $^{129}$Xe Gas: Preliminary Human Results"; MRM 37 (1997) 809 - 815

und den Anmeldungen

WO9527438A1 vom 4.4.1995, "Magnetic Resonance Imaging using hyperpolarized Noble Gases" und WO9737239A1 vom 28.2.97, "Enhancement of NMR and MRI in the presence of hyperpolarized Noble Gases" beschrieben werden.

Der Begriff "Polarisation" P bezeichnet den Anteil an orientierten Kernspins I (z.B. Isotop $^3$He mit Massenzahl 3 und Kernspinquantenzahl I = ½) und der damit verbundenen magnetischen Kern-Diplomomente $\mu_1$ entlang der Richtung eines äußeren Magnetfeldes B. Um gasförmige Isotope in der Kernspintomographie einsetzen zu können, wird eine Polarisation P benötigt, die 4 bis 5 Größenordnungen über $P_{Boltzmann}$ liegt, dem Grad der Polarisation des Gases im relaxierten thermischen Gleichgewichtszustand. Im äußeren Magnetfeld mit Flußdichte B des Experimentaufbaus ist $P_{Boltzmann}$ an die Boltzmannkonstante k gekoppelt und beträgt, ausgedrückt durch die magnetische Dipolenergie $-\mu_I$ B und der thermischen Energie kT :

$$P_{Boltzmann} = \tanh(\mu_I B_T / kT) \tag{1}$$

(mit T der absoluten Temperatur in Kelvin).

Die stärksten Flußdichten werden in medizinischen MR-Tomographen eingesetzt, wo dennoch $P_{Boltzmann} \ll 1$ ist und sich in guter Näherung durch $P_{Boltzmann} = \mu_I B / kT$ berechnen läßt. Typischerweise beträgt in Tomographen B = 1.5 T und T = 300 K, so dass sich eine $^3$He-Polarisation von nur $P_{Boltzmann} = 3{,}9 * 10^{-6}$ einstellen würde, aber Werte von $P \geq 1 * 10^{-2}$ benötigt werden, um den Signalverlust aufgrund der im Vergleich zum Gewebe sehr viel geringeren Atomzahldichte im gasförmigen Zustand auszugleichen. Gase mit solchen Polarisationsgraden werden auch als "hyperpolarisiert" bezeichnet. Die Präperation erfolgt über bekannte Verfahren, vorzugsweise durch Optisches Pumpen. Neben dem hohen Polarisationsgrad müssen für die Anwendungen relativ große Mengen an hyperpolarisiertem Gas bereitgestellt werden, so etwa für die Lungenventilation in der Größenordnung von 0,5 bis 1 liter pro Patientenuntersuchung.

Die Qualität des Gases als Tracer in der MRT orientiert sich an seinem Polarisationsgrad. Es steigt nämlich der Kontrast eines MRT-Bildes linear mit dem Polarisationsgrad an. Bei jeder Anregung der Kernresonanz wird der Polarisationsvektor um den sogenannten Flipwinkel $\alpha$ aus der Magnetfeldachse gekippt. Bei jeder Anregung wird die Hyperpolarisation P auf den Betrag $P * \cos \alpha$ irreversibel abbaut. Der Flipwinkel kann daher bei vorgegebenen, angestrebten Bildkontrast umso kleiner gewählt werden, je größer P ist, derart, dass das signalbestimmende Produkt $P * \sin \alpha$ konstant bleibt. Eine größere Hyperpolarisation ermöglicht daher eine größere Anzahl von Anregungen mit ein- und derselben Gabe an Tracergas. Das bedeutet einerseits die Möglichkeit der Steigerung der räumlichen Auflösung oder die Aquirierung einer größeren Anzahl an Bildern bei gleicher räumlicher Auflösung und pro Tarcergasgabe. Letzteres ist insbesondere für die funktionelle Lungenuntersuchung wichtig, der durch die Anwendung von kemspinpolarisiertem $^3$He Gas völlig neue Informationsquellen erschlossen werden. Solche Methoden sind beispielsweise in den Veröffentlichungen

W.G. Schreiber, K. Markstaller, B. Eberle, H.-U. Kauczor, N. Weiler, R. Surkau, G. Hanisch, M. Thelen; "Ultrafast MR-Imaging of Lung Ventilation Using Hyperpolarized Helium-3"; Eur. Radiol. 9, (1999) B28,

A.J. Deninger, B. Eberle, M. Ebert, T. Großmann, W. Heil, H.-U. Kauczor, L. Lauer, K. Markstaller, E. Otten, J. Schmiedeskamp, W. Schreiber, R. Surkau, M .Thelen, N. Weiler; "Quantification of regional intrapulmonary oxygen partial pressure evolution during apnea by $^3$He MRI"; im Druck in J. Magn. Res. (Nov 1999),

X.J. Chen, H.E. Möller, M.S. Chawla, G.P. Cofer, B. Driehuys, L.W. Hedlund, G.A. Johnson; "Spatially Resolved Measurements of Hyperpolarized Gas Properties in the Lung in vivo.Part I: Diffusion Coefficient"; MRM, in press (June 1999) beschrieben.

Andererseits kann auch die für einen bestimmten Bildkontrast notwendige Tracer-Gasmenge reduziert werden, so dass Störungen der normalen Ventilation der Lunge entsprechend der Viskosität und Diffusivität von Luft durch Beimischung von z.B. $^3$He reduziert werden kann. Je kleiner die Tracergasmenge, desto realistischer sind die Ergebnisse und wertvoller die Daten. Daher muß bei der Herstellung des kernspinpolarisierten Gases nicht nur auf die Menge sondern insbesondere auf einen hohen Polarisationsgrad geachtet werden.

Es ergibt sich nun das Problem, hohe Polarisationsgrade, beispielsweise P > 30 % zu erreichen. Dafür lässt sich die Methode des "Optischen Pumpens" von einem Alkalimetall-Dampf (meist Rubidium) und anschließender Spinaustausch-Polarisierung zwischen polarisierten Alkaliatomen und unpolarisierten $^3$He-Atomen einsetzen. Diese Verfahren und Anwendungen sind in den folgenden Publikationen beschrieben:

B. Driehuys, G.D.Cates, E. Miron, K. Sauer, D.K. Walter and W. Happer; "High-volume production of laser-polarized $^{129}$Xe"; Appl. Phys. Lett. 69:12 (1996) 1668 - 1670,

- Thad G. Walker, William Happer; "Spin-exchange optical pumping of noble-gas nuclei"; Review of Modern Physics 69:2 (1997) 629 - 642,

K.C. Hasson, P.L. Bogorad, B. Driehuys, G. Kameya, B. Wheeler, and D. Zollinger; "Polarized Helium-3 Production and Transportation System"; Eur. Radiol. 9 (1999) B16 ,

weitere Beschreibungen lassen sich finden in

WO9640585A1 vom 19.12. 1996 : "Method and System for producing polarized $^{129}$Xe Gas",
WO9639912A1 vom 7.6.1996: "Coatings For Production Of Hyperpolarized Noble Gases",
US05545396 vom 8.13. 1996: "Magnetic resonance imaging using hyperpolarized noble gases",
US05557199 vom 17.9.1996: "Magnetic resonance monitor",
US05612103 vom 18.3.1997: "Coatings for production of hyperpolarized noble gases",
US05617860 vom 8.4.1997: "Method and system for producing polarized 129 Xe gas",
US05642625 vom 1.7.1997: "High volume hyperpolarizer for spin-polarized noble gas",
US05789921 vom 4.8.1998: "Magnetic resonance imaging using hyperpolarized noble gases",
US05785953 vom 28.7.1998: "Magnetic resonance imaging using hyperpolarized noble gases",
US05809801 vom 22.9.1998: "Cryogenic accumulator for spin-polarized xenon-129",
US05860295 vom 19.1.1999: "Cryogenic accumulator for spin-polarized xenon-129".

Mit der leistungsfähigsten Appartur dieser Art, wie in Hasson et.al. (1999) (s.o.) beschrieben, lassen sich $^3$He Gasmengen von beispielsweise 2 bar liter / h mit P=10 % oder 0,38 bar liter / h mit P=30 % oder 0,11 bar liter / h mit P=40 % gewinnen.

Mit einem alternativen Verfahren, dem direkten "Optischen Pumpen" von $^3$He in einer Niederdruckgasentladung mit anschließender Kompression, lassen sich hohe Polarisationsgrade, bis dato beispielsweise P > 30 %, und gleichzeitig hohe Produktionsraten, beispielsweise 0,5 bar liter / h (entsprechend 12 bar liter / Tag) erzielen. Dafür wird $^3$He-Gas einem Reservoir entnommen und und bei einem Druck von ca. 1 mb in einer Plasmaentladung durch Absorption von zirkularpolarisiertem 1083,2 nm Laserlicht kernspinpolarisiert. Anschließende Kompression und Speicherung stellen das polarisierte Gas für eine Reihe sehr verschiedener Zwecke in der physikalischen Grundlagenforschung wie in der medizinischen Anwendung zur Verfügung. Dieses Verfahren ist in den folgenden Publikationen ausführlich beschrieben:

G. Eckert, W. Heil, M. Meyerhoff, E.W. Otten, R. Surkau, M. Werner, M. Leduc, P.J. Nacher, L.D. Schearer; "A dense polarized $^3$He target based on compression of optically pumped gas"; Nucl. Inst. & Meth. A 320 (1992) 53 - 65

J. Becker, W. Heil, B. Krug, M. Leduc, M. Meyerhoff, P.J. Nacher, E.W. Otten, Th. Prokscha, L.D. Schearer, R.Surkau; "Study of mechanical compression of spin-polarized $^3$He gas"; Nuc. Instr. & Meth. A (346) (1994) 45 - 51

W. Heil, H. Humblot, E.W. Otten, M. Schäfer, R. Surkau, M. Leduc; "Very long nuclear Relaxation times of spin polarized helium 3 in metal coated cells"; Phys. Lett. A 201 (1995) 337 - 343

R. Surkau; "Entwicklung und Test eines $^3$He-Neutronen-Spinfilters"; Dissertation an der Johannes Gutenberg-Universität Mainz (1995)

R. Surkau, J. Becker, M. Ebert, T. Großmann, W. Heil, D. Hofmann, H. Humblot, M. Leduc, E.W. Otten, D. Rohe, K. Siemensmeyer, M. Steiner, F. Tasset, N. Trautmann; "Realization of a broad band neutron spin filter with compressed, polarized $^3$He gas"; Nuc. Instr. & Meth. A 384 (1997) 444 - 450

Der Stand der Technik ist in der Veröffentlichung Surkau (1995), Surkau et.al. (1997) und

R. Surkau, A.J. Deninger, J. Bermuth, M. Ebert, T. Großmann, W. Heil, L. Lauer, E. Otten, J. Schmiedeskamp; "Highly polarized $^3$He for Lung MRI"; Eur. Radiol. 9 (1999) B15

beschrieben. Der Polarisator umfaßt 5 Baugruppen: Gas wird aus einem Gasversorgungssystem entnommen, in einer Polarisationseinheit bei Drücken zwischen 0,5 und 5 mb kernspinpolarisiert, in einer Kompressionseinheit auf Enddrücke von bis zu 10 bar verdichtet und schließlich in einer angeschlossenen Speichereinheit für längere Zeiträume zwecks Transports und Anwendung aufbewahrt. Eine Vorraussetzung zur Polarisationserhaltung über längere Zeiträume ist die Einbettung der polarisationsführenden Systeme in einem homogenen Magnetfeld, einer weiteren Baugruppe. Es werden Feldstärken von typischer Weise 0,8 mT verwendet.

Dieses Magnetfeld muß ein Mindesthomogenität aufweisen. Bei relativen, transversalen Gradienten von beispielsweise

$$\partial Br / \partial r \, / \, B_0 = G_r < 5 * 10^{-4} \, / \, cm \tag{2}$$

als Grenzwert werden bei Drücken von beispielsweise 200 mb und größer, wie sie beispielsweise beim Speichern von Gasen auftreten, nach dem in Veröffentlichung

L.D. Schearer, G.K. Walters; "Nuclear Spin-Lattice Relaxation in the Presence of Magnetic-Field Gradients"; Phys. Rev. 139:5A (1965) 1398- 1402

G.D. Cates, S.R. Schaefer, and W. Happer; "Relaxation of spins due to field inhomogeneities in gaseous samples at low magnetic fields and low pressures"; Phys. Rev. A 37 (1988) 2877 - 2885

erläuterten Zusammenhang

$$T_1 = p / (G_r^2 * 17000) \, h \, / \, (bar \, cm^2) \tag{3}$$

noch gradientenbedingte Relaxationszeiten von $T_1 > 47$ h eingehalten.

Um in dem geforderten Bereich die oben geforderte, für die Polarisationsspeicherung notwendige Homogenität aufzuweisen besteht das Magnetfeld aus 5 Ringspulen gleichen Durchmessers mit geeigneter Amper-Windungszahl und Abständen zueinander. Die jeweils beiden äußeren Spulenringe sind symmetrisch zur mittleren Spule axial angeordnet. Alle 5 Spulenringe sind in Serie geschaltet, sodass ein einheitlicher Strom alle Spulen durchfließt. Die benötigte Ampere-Windungszahl wird dann für jede Spule anhand geeignet gewählter Drahtwicklungszahlen eingestellt. Dies hat den Vorteil, den relativen Beitrag einjeder Spule zum Gesamtfeld trotz evt. kleiner Stromdrifts konstant zu lassen und damit verlässlich ein homogenen Feldbereich innerhalb des Spulenvolumens für die Apparatur aufzubauen.

Das Gasversorgungssystem umfaßt ein $^3$He-Reservoir und eine Schleuse, die periodisch Gas aus dem Reservoir entnimmt und in eine Reinigungsstufe einfüllt. Es wird dmit ein $^3$He-Fluß erzeugt, der dann von Fremdgasen außer Edelgasen gesäubert wird. Hierin wird hochporöses Gettermaterial St 707 der Firma SAES, Milano, verwendet, das in einem ersten Teil bei etwa 250°C vermehrt Moleküle adsorbiert, sie in ihre atomaren Bestandteile zerlegt und diese festhält. In einem zweiten Teil senkt Gettermaterial bei Raumtemperatur den Wasserstoffpartialdruck auf sehr kleine Werte ab, d.h. Wasserstoff wird effektiv im Getter adsorbiert. Nachfolgend wird das gereinigte $^3$He Gas durch eine Kapillare und eine Flüssigstickstoffkühlfalle in den Bereich des Optischen Pumpens geleitet. Die Kapillare formt über ein Druckgefälle einen kontinuierlichen Fluß in die Polarisationseinheit und verhindert andererseits eine Rückdiffusion von polarisiertem Gas in die Reinigungseinheit und Gebiete außerhalb des Magnetfelde, die die Polarisation schnell zerstören würden.

In der Polarisationseinheit wird in 4 zylinderförmigen, 1 Meter langen und 75 mm durchmessenden Zellen 320 bei Drücken zwischen 0,1 und 3 mbar über das Einstrahlen von elektrischen Hochfrequenzfeldern eine Gasentladung gezündet und metastabiles $^3$He Gas erzeugt ($^3$He$^*$). Dieses $^3$He$^*$ absorbiert resonant 1083,2 nm Licht aus einer geeigneten Lichtquelle (beispielsweise ein cw Nd:LMA Festkörperlaser), das über Polarisationsoptiken zirkular polarisiert und entlang des äußeren Magnetfeldes eingestrahlt wird. Der über die Lichtabsorption übertragene Drehimpuls richtet den Kernspin in Abhängigkeit der Rechts- oder Linkshändigkeit des Lichtes parallel (oder antiparallel) zum Magnetfeld aus. Näheres über die atom- und kernphysikalischen Prozesse lese man in der einschlägigen Literatur nach, z.B. in

F.D. Colegrove, L.D. Schearer, K. Walters; "Polarization of 3He Gas by Optical Pumping; Phys. Rev.(132) (1963) 2561-2572,

P.J. Nacher and M. Leduc; "Optical pumping in $^3$He with a laser"; J. Phys. (Paris) 46 (1985) 2057 - 2073.

G. Eckert, W. Heil, M. Meyerhoff, E.W. Otten, R. Surkau, M. Werner, M. Leduc, P.J. Nacher, L.D. Schearer; "A dense polarized $^3$He target based on compression of optically pumped gas"; Nucl. Inst. & Meth. A 320 (1992) 53 - 65

Das Laserlicht wird nach einmaligem Durchgang durch eine Zelle an einem dichroitischen Spiegel reflektiert, nichtabsorbiertes Licht steht damit für einen 2. Durchlauf zur Verfügung. 668 nm Fluoreszenzlicht kann dagegen durch diesen Spiegel hindurchtreten. In einem optischen Polarisationsnachweis, der in den Veröffentlichungen

N.P. Bigelow, P.J. Nacher, M. Leduc; "Accurate optical measurement of nuclear polarization in optically pumped $^3$He gas"; J. Phys. II France 2 (1992) 2159 - 2179

W. Lorenzon, T.R. Gentile, H. Gao, R.D. McKeown; "NMR calibration of optical measurement of nuclear polarization in $^3$He"; Phys. Rev. A 47:1 (1993) 468 - 479

und unten im Text näher beschrieben ist, wird der Zirkularpolarisationsgrad dieses Fluoreszenzlichtes gemessen und über zum Teil druckabhängige Eichfaktoren in die gerade vorherrschende, absolute Kernspinpolarisation umgerechnet. Die Zellen sind seriell miteinander verbunden, so dass das Gas langsam durch diese hindurchströmt und stetig an Polarisation gewinnt.

Über eine weitere Flüssigstickstoff-Kühlfalle wird das polarisierte $^3$He Gas periodisch z.B. alle 10 s über ein Einlaßventil in den Kompressionsraum der ersten Kolbenkompressors eingelassen, mittels Vorschub des Kolbens verdichtet und über ein Auslaßventil in eine Sammelzelle ausgestoßen. Der Antrieb des Kolbens erfolgt über einen Pressluft-Linearmotor, der über die Endplatte eines Balges die Bewegung über Kolbenstangen auf den Kolben im ultrahochvakuumdichten Kompressor überträgt. Alle Kompresorskomponenten sind aus Titan oder Bronze gefertigt, um Polarisationsverluste durch Wandkontakt mit magnetischem Material zu minimieren und andererseit ein Ultrahochvakuum taugliches und abriebfestes Material zu verwenden. Der Kolben mit Durchmesser 140 mm und Hub 110 mm ist durch einen Doppellippendichtring gedichtet und gelagert. Die Sammelzelle wird standardmäßig auf etwa 200 mb gefüllt bevor eine im Prinzip baugleiche zweite Kompressionsstufe mit Ventilen mit Kolbendurchmesser 60 mm und Hub 94 mm das Gas auf Enddrücke von 1 bis 10 bar verdichtet. Diese 2. Kompressionsstufe führt pro Sammelzellenfüllung 6 Kompressionszyklen aus mit einer Periode von ebenfalls 10 s und entleert damit die Sammelzelle.

Das von der 2. Kompressionsstufe hochverdichtete Gas wird in eine angeflanschte Speicherzelle gepumpt. Diese Speicherzelle kann über ein eignenes Ventil verschlossen und anschließend für Transportzwecke vom Polarisator gelöst werden. Solche transportable Zellen werden auch zur langen Aufbewahrung des Gases eingesetzt. Eine beispielhafte Ausführung für den medizinischen Einsatz ist daher z.B. aus geeignetem eisenarmen und diffusionsfestem Supremax Glas der Fa. Schott Glas gefertigt, worin Relaxationzeiten von bis zu 100 Stunden erreicht werden können. Weiterhin können auch durch geeignete Innenbeschichtungen die Relaxationszeit verlängert und Speicherzeiten von bis zu 200 Stunden erreicht werden, wie in den Veröffentlichungen

W. Heil, H. Humblot, E.W. Otten, M. Schäfer, R. Surkau, M. Leduc; "Very long nuclear relaxation times of spin polarized helium 3 in metal coated cells"; Phys. Lett. A 201 (1995) 337 - 343

W. Heil, J. Dreyer, D. Hofmann, H. Humblot, E.- Levievre-Berna, F. Tasset; "$^3$He neutron spin-filter"; Physica B 267-268 (1999) 328-335

ausgeführt wird.

Das Verfahren des direkten Optischen Pumpens von $^3$He in einer Niederdruckgasentladung mit anschließender Kompression ist zwar theoretisch gut untersucht, aber daraus abgeleitete Vorrichtungen und ihre Entwicklung zur Reife, die den geforderten Produktionsdaten nach Menge und Polarisationsgrad ebenso wie den Ansprüchen an Zuverlässigkeit und Qualitätssicherung genügt, stellt dagegen eine sehr hohe und vielseitige physikalisch technische Herausforderung dar. Die Produktion, Speicherung und der anschließende Transport des hyperpolarisierten $^3$He-Gases zum Anwendungsort bedarf neuartiger Verfahren und Vorrichtungen. Deshalb wurde die Produktion von hyperpolarisierten Gasen bislang meist nur im kleinen Stil vorgenommen. Die für die Herstellung und Operation von Apparaturen zur effizienten Produktion von hochpolarisiertem $^3$He-Gas verlangte Expertise betrifft die Gebiete der Atom- und Laserphysik, der Kernresonanz, der Technik des ultrahohen Vakuums, der Handhabung spezieller Materialien wie beispielsweise Titan oder Glas. Speziell für die klinische Anwendung wird eine Gasversorgung für viele Einsatzorte notwendig, die eine gute Gas-Qualität zuverlässig garantiert. Qualitätssicherung ist am Besten durch eine zentrale Herstellung des Gases durch Spezialisten mit leistungsfähigen Apparaturen darstellbar. Das Gas wird anschließend gespeichert und dem Kunden angeliefert.

Aufgabe der Erfindung ist es somit, ein Produktionsverfahren und - vorrichtung anzugeben, das auf der Methode des direkten Optischen Pumpens von $^3$He basiert und gegenüber dem Stand der Technik die Steigerung der Produktionrate um etwa eine Größenordnung bei gleicher Endpolarisation gewährleistet. Das Verfahren umfaßt eine Reihe von Prinzipien, um die hohen Produktionsraten und Endpolarisationen gleichzeitig verwirklichen zu können. Kern der Aufgabe ist die möglichst polarisationsverlustfreie Verdichtung von $^3$He Gas, das zuvor möglichst hoch kernspinpolarisiert wurde. In der Methode des direkten Optischen Pumpens von $^3$He anfallende höhere Investitionskosten pro Gerät, lohnen sich aufgrund des weit höheren Produktionspotentials im Vergleich zu der Spinaustausch-Polarisierung, deren Produktionspotential pro Gerät durch physikalische Gesetzmäßigkeiten limitiert ist.

Ein wesentliches Ziel der Erfindung ist die Dichtung von Bewegungsdurchführungen in den $^3$He-Gasraum. Dabei muß beachtet werden, dass in den Teilen der Apparatur mit $^3$He-Gas möglichst Dichtheiten und Charakteristiken der Fremdgasabgabe innerhalb der Rezipienten entsprechend Ultrahochvakuum-Bedingungen (UHV) gefordert werden. Fremdgasbeimischungen in der Größenordnung von ppm beeinträchtigen bereits empfindlich die für effizientes optisches Pumpen notwendigen Plasmabedingungen und damit die erreichbare Endpolarisation. Weiterhin müssen Ventile mit hohen Standzeiten und angepaßter Bauform bzgl. Leitungsquerschnitt und Ventilvolumen aus nichtmagnetischen oder nur sehr schwach magnetichem Materialien eingesetzt werden. Gleiches gilt für den Kompressor.

Bewegungsdurchführungen in den Vakuumbereich werden am geeignetsten - da technisch am einfachsten zu realisieren - mittels Linearantrieben realisiert. Eine UHV taugliche Durchführung von Linearbewegung von außerhalb in den $^3$He-Gasbereich hinein, wurde bislang mit Hilfe von hochelastischen Metallbälgen realisiert, die das bewegte mit dem unbewegten Teil vakuumdicht verbinden. Geeignete Bälge können z.B. in der Bauform als Wellbälge aus Tombak oder Bronze oder in der Bauform als Membranbalg z.B. aus Titan gefertigt werden. Ihnen gemein ist eine endliche Lebenszeit, ausgedrückt in einer maximalen Zahl an Streckungen oder Stauchungen mit bestimmter Hubstrecke, die von Wellen- oder Membrananzahl und geometrischer Dimensionierung abhängt. Danach führen Materialermüdungen zu Brüchen und damit zu Undichtigkeiten. Die gewünschte Zahl an Streck- oder Stauch-Zyklen liegt in der Größenordnung von einigen Hundertausend bis wenige Millionen, was je nach gewünschter Hublänge sehr platzaufwendige und kostenträchtige Balgausführungen erfordert.

Gemäß einem ersten, wesentlichen Gedanken der Erfindung wird die Methode der Dichtung von Bewegungsdurchführungen in den $^3$He-Gasraum platz- und kostensparend gemäß Anspruch 1 gelöst, und insbesondere für alle Dimensionen ein zuverlässig arbeitendes Prinzip der UHV-tauglichen Linearbewegungsdurchführung angegeben. Innerhalb eines Gehäuses wird ein bewegliches Bauteil linear bewegt. Das bewegliche Bauteil trennt dabei einen ersten Raum, der in leitender Verbindung mit der Umgebung steht, von einem einem zweiten Raum ab, der in leitender Verbindung mit einem abgeschlossenen System steht. Das abgeschlossene System umfaßt das polarisierte Gas. Das bewegliche Bauteil ist dabei derart ausgeführt, das zwischen dem ersten und dem zweiten Raum ein Zwischenraum ausgebildet ist, so dass ein direktes Eindringen von Fremdgasen aus der Umgebung in das abgeschlossene System weitgehend vermieden wird.

Gemäß eines weiteren Gedankens der Erfindung wird der Zwischenraum über einen Pumpenanschluß evakuiert, so dass mit hoher Sicherheit aus der Umgebung in den Zwischenraum eindringende Gase abgepumpt werden und nicht in das geschlossene System eindringen können. Dieses Dichtungsprinzip mit selektivem Bepumpen von Zwischenvolumen wird im Folgenden "fraktioniertes Pumpen" genannt.

Diese Ausführung der Durchführung von Linearbewegungen in geschlossene Systeme kann nun mit Vorteil für die Realisierung von Kompressoren einerseits und/oder Ventilen andererseits genutzt werden. Bei Kompressoren ist das bewegliche Bauteil der Kolben, der durch seine Bewegung das Kompressionsvolumen verdrängt und ein darin befindliches Medium komprimiert. Bei Ventilen stellt das bewegliche Bauteil einen Ventilstößel dar, der zum Schließen einer Verbindung zwischen zwei Öffnungen im Gehäuse dient.

Die Länge des Bauteils kann so groß gewählt werden, dass die Längsstreckung des beweglichen Bauteils stets größer ist als sein Hub. Mit Vorteil wird dabei ausgeschlossen, dass Medien der Umgebung Oberflächen des Gehäuses benetzen, die nach der Bewegung des beweglichen Bauteils anschließend Teil der Oberfläche des abgeschlossenen Systems darstellen. Damit wird der Eintrag von Fremdmedien durch zwischenzeitliche Benetzung der Gehäuseoberfläche vermieden.

Da prinzipiell mehrere solche Baugruppen, bestehend aus Gehäuse, einem beweglichem Bauteil und einem bepumpten Zwischenraum in einer Vorrichtung zur Anwendung kommen können, können diese Gehäuse zu einem einzigen mit mehreren beweglichen Bauteilen zusammengefaßt werden, worin die Zwischenräume in leitender Verbindung stehen. Mit Vorteil ergibt sich eine platzsparende Anordnung, worin speziell alle Zwischenräum über nur einen einzigen Pumpanschluß evakuiert werden können.

Gemäß eines weiterführenden Gedankens werden Dichtmaterialen verwendet, die in Nuten untergebracht sind, welche wiederum um den Kolben oder Stößel umlaufen. Diese Dichtmaterialien umfassen beispielsweise Gleitdichtungen in Form von Ringen und bewirken, dass oben genannten Räume möglichst gut voneinander abgetrennt sind und dennoch eine Bewegung der Kolben oder Stößel zugelassen wird.

Für den Einsatz solcher Baugruppen in Anlagen zur Herstellung von polarisierten $^3$He-Gas muß beachtet werden, dass ein äußeres, homogenes Magnetfeld nicht verzerrt und die Oberflächen möglichst keine paramagnetischen Zentren aufweisen, um hohe Polarisationsgrade zu erreichen und zu erhalten. Weiterhin sollten die Oberflächen selbst möglichst keine Fremdgase freisetzten, um die Effizienz des fraktionierten Pumpens nicht zu schwächen. Daher werden für Gehäuse, bewegliche Bauteile und Dichtmaterialien in unmittelbarem Kontakt oder der Nähe von polarisiertem $^3$He nur aus möglichst gering ausgasenden Werkstoffen gefertigt, die zudem aus un- oder nur schwach magnetisiertem Materialien bestehen.

Insbesondere Oberflächen, die von Gleitdichtungen überstrichen werden, müssen zusätzlich zu den zuvorgenannten Eigenschaften noch eine ausreichende Abriebfestigkeit aufweisen. Mit Vorteil werden dafür Materialien wie Titan oder Bronze eingesetzt.

Wie bereits zuvor ausgeführt, werden in oben beschriebenen Kompressoren mit Vorteil funktionsbedingt lange Kolben eingesetzt - er muß länger als sein Hub sein. Dies führt nun im allgemeinen zu einer erheblichen Verlängerung des gesamten Kompressors. Besonders bei großen Hubwegen, wie sie in der hier besprochenen Anwendung bevorzugt werden, ist es von Vorteil, wenn beim Hydraulikantrieb des Kompressors durch Integration der Druckkammern in den Innenraum des Kolbens selbst die Baulänge verkürzt wird.

Der Kolbenkompressor und sein Antrieb dürfen weiterhin das homogene Magnetfeld nicht verzerren. Für platzsparende Apparaturen muß daher der Antrieb aus nichtmagnetischen Materialien dicht am eigentlichen Kompressor aufgebaut sein. Ein weiterer Gedanke der Erfindung ist daher die Verwendungs eines in dem Stößel oder Kolben integrierten, hydraulischen Antriebs aus denselben Materialien, die auch zum Bau des Kompressors oder Ventils eingesetzt werden.

Um bei gegebenem Kolbendurchmesser den gewünschten Enddruck von beispielsweise 10 bar im Kompressionsvolumen aufbauen zu können muß der Antrieb genügend Kraft aufbringen. Mit Vorteil lassen sich hier hydraulische Antriebe einsetzen, da bei Öl-Drücken von typischerweise bis zu 250 bar im Vergleich zur Stirnfläche des $^3$He-Gasraums nur kleine Stirnflächen für das Ölvolumen im Kolben oder Stößel notwendig sind. Damit lassen sich dennoch die notwendigen Kräfte aufbauen, um den erwünschten Kolben- oder Stößelvortrieb zu realisieren. Auch aus diesem Grund kann der Antrieb innerhalb des Kolbens untergebracht werden, womit gleichzeitig mit Vorteil der Hub des Kolbens maximiert werden kann, wenn die maximaler, äußerer Kompressorbaulänge vorgegeben ist.

Um den Kolben in beide Richtungen bewegen zu können werden als weiterer erfinderische Gedanke im Kolben oder Stößel zwei Druckkammern realisiert, so dass bei Druckbeaufschlagung der einen oder anderen Druckkammer der Kolben oder Stößel in das Gehäuse ein- oder ausfährt. Gleichzeitig wird das Öl aus der nichtdruckbeaufschlagten Kammer wieder abgelassen, so dass die Bewegungsrichtung beliebig häufig umgekehrt werden kann. Insbesondere lassen sich auch Stellungen des Kolbens- oder Stößels zwischen den Endlagepunkten anfahren und nach Verschließen der Ölkammern auch stabilisieren. Solange die Ölkraft größer als die Kräfte sind, die durch Gase im geschlossenen System auf den Kolben oder Stößel ausgeübt werden, läßt sich damit der Kolben oder Stößel wie gewünscht bewegen. Dieser Fall läßt sich für die Anwendung im $^3$He-Kompressor im Allgemeinen immer erreichen.

In einer Variante des hier vorgestellten Prinzips eines Reinstgaskompressors wird statt des oben beschriebenen, langsam laufenden Kolbenkompressors mit großem Hubvolumen und großem Hubweg ein schnell laufender Kolbenkompressor mit kleinerem Hubvolumen und kürzerem Hubweg bei gleicher Förderleistung gewählt. Dabei wird das Prinzip des fraktionierten Pumpens über Zwischenraumvolumina beibehalten. Der Antrieb des Kolbens kann dann mit Vorteil auf traditionelle Weise über Kurbelwelle und Pleuel erfolgen.

Allerdings muß man bei schnellaufenden Kolben höhere Leckraten über die Dichtringe tolerieren, weil hermetisch dichtende Ringe einen hohen Anpressdruck erfordern, der bei schnellem Lauf zu einer unverträglich hohen Entwicklung von Reibungskräften und -wärme führt. Die auf die komprimierte Gasmenge bezogenen relativen Leckverluste wachsen aber im Vergleich zu einem langsam laufenden hermetisch dichtenden Kolben, nicht im gleichen Maße wie die Leckraten selbst, weil andererseits auch der Kompressionsvorgang in kürzerer Zeit abläuft, bekannt als Prinzip der dynamischen Dichtung. Dennoch darf man sich bei der Kompression von beispielsweise seltenen Edelgasen, wie die hier genannten $^3$He und $^{129}$Xe-Isotope, aus Kostengründen keine Leckverluste erlauben.

Das hier beschriebene Prinzip des fraktionierten Pumpens löst dieses Problem auf folgende Weise, welches für jedes System mit bepumpten Zwischenvolumen angewandt werden kann: Die aus dem geschlossenen System in das Zwischenvakuum einleckende Edelgasmenge wird über den Pumpenanschluß abgepumpt. Nach Abtrennung der Fremdgase, beispielsweise kryotechnisch, steht das gereinigte Edelgas wieder zur Verfügung.

Ein weiterer erfinderische Gedanke ist nun die Verwendung des fraktionierten Pumpens als Prinzip der Bewegungsdurchführung in den Gasraum für eine Einrichtung zum Kernspinpolarisieren von $^3$He-Gas nach der Methode des direkten "Optischen Pumpens". Diese Einrichtung umfaßt die gleichen Baugruppen wie im Stand der Technik: Eine Polarisationseinheit, in der das $^3$He-Gas in einem Niederdruckplasma bei Drücken zwischen 0,5 und 5 mb kernspinpolarisiert wird; eine Kompressionseinrichtung zur Kompressions des polarisierten Gases auf Enddrücke von bis zu 10 bar und schließlich ein Speichervolumen, in dem das Gas für längere Zeiträume zwecks Transport und Anwendung aufbewahrt wird. Die Verwendung des oben beschriebenen "fraktionierten Pumpens" hat den Vorteil, eine erhebliche Steigerung der Effizienz der Einrichtung zu erreichen. Dies betrifft insbesondere die Erhaltung der einmal erzeugten Polarisation während der Kompression.

Ein weiterer erfinderische Gedanke betrifft die Unterstützung des "fraktionierten Pumpens" durch nur geringausgasende Wandmaterialien. Weiterhin muß bedacht werden, dass mit Vorteil alle Vakuumleitungen in dem homogenen Magnetfeld untergebracht sind, welches für die Erzeugung der Polarisation wichtig ist. Um die Feldhomogenität zu erhalten, müssen die Leitungen aus nichteisenhaltigen Materialien gefertigt sein. Allgemein kann daher als Wandmaterial für Leitungen Aluminium gewählt werden. Als Dichtmaterialien werden Alu-Schneidkantringe eingesetzt. Alu-Schneidkantringe haben gegenüber normalen Dichtungen aus Polymeren (Kunststoff, Gummi wie Viton, NBR etc.) den Vorteil, nicht mehr als das Rohrmaterial selbst auszugasen, so dass Vakua von unter $10^{-6}$ mb leicht erreicht werden. Im Niederdruckbereich der Einrichtung werden Glas-Metall Übergänge ebenfalls metallisch mit weichem Indium gedichtet. Auf der Hochdruckseite werden Viton Dichtringe eingesetzt. Viton eignet sich von allen Polymeren am besten aufgrund seiner dichtesten Matrix und den damitverbunden relativ geringen Ausgasraten an Fremdgasen wie z.B. $N_2$, $O_2$, $CO_2$, oder Kohlenwasserstoffen.

In einem solchermaßen gefertigten und gedichteten System lassen sich vor dem Pumpstutzen Vakua von < $5^{*}10^{-9}$ mb erreichen. Dies bedeutet einen großen Fortschritt im Vergleich zum Stand der Technik, wo alle Vakuumsysteme aus Glas gefertigt sind und nur Glashähne mit kleinem Querschnitt (max. 5 mm Durchmesser) und folglich geringem Leitwert eingesetzt werden konnten. Insbesondere unterstützt die Strategie guter Pumpquerschnitte die Arbeitsweise des fraktionierten Pumpens.

Ein weiterer Gedanke der Erfindung betrifft die Erweiterung des "fraktionierten Pumpens" auch auf den Bereich des $^{3}$He-Plasmaraumes mit Vorteil zum Erreichen besserer Betriebswerte, d.h. höheren Gasdurchsatz und höherer Endpolarisation. Durch gasselektives Pumpen wird der $^{3}$He-Plasmaraum von eingedrungen oder desorbierten Fremdgasen gereinigt. Hohe Gasreinheiten mit Fremdgasatomkonzentrationen von kleiner 10 ppm in den Optischen Pumpvolumen sind notwendig, um hinreichende Dichten an angeregtem, metastabilem Helium Atomen für eine effiziente Absorption des optischen Pumplichts zu erreichen. Fremdgasatome bauen im Stoß die Anregungsenergie der He-Atome ab.

Mit Vorteil können Getterpumpen zur selektiven Reinigung von Edelgasen in dem fraktionierten Pumpprozess eingesetzt werden, da diese sich weder chemie- noch physisorbieren lassen. Solange die Diffusionszeiten an die Getteroberflächen kleiner sind als die Zeit des Vorbeiströmens an der Getterpumpe, besteht aufgrund der hohen Affinität der Oberfläche eine hohe Wahrscheinlichkeit, dass das Fremdgas adsorbiert wird. Mit Vorteil eignen sich daher selektive Sorptionspumpen zum weiteren fraktionierten Pumpen am Ein- und/oder Ausgang des Volumens zum optischen Pumpen. Das Eindringen von Fremdgasen in dieses Optische Pumpvolumen wird somit effektiv verhindert. Solche Fremdgase können beispielsweise von Dichtmaterialien des Kompressor freigesetzt werden. Der Einsatz von Gettern ermöglicht damit das Erzielen höchster Endpolarisationen und Produktionsraten.

Dafür kommen Getterpumpen zum Einsatz, bei denen Physi- oder Chemiesorption von Gasen an oder in eine meist metallische Matrixstruktur erfolgen. Reinste metallische Getteroberflächen können direkt im Vakuum durch Verdampfen oder durch Kathodenzerstäubung ständig oder im geeigneten Rhythmus neu erstellt werden und die durch gesättigte Absorption passivierten Schichten ersetzen. Alternativ finden nicht verdampfbare Getter "NEG" wie "Non evaporable getters" genannt Verwendung. Ihre Poriosität bietet großes Oberflächen- zu Volumenverhältnis und damit eine hohe Kapazität.

In der Vorrichtung nach dem Stand der Technik werden ein NEG betehend aus in Tablettenform gesintertem Metallstaub der Legierung St707 der Fa. SAES Getters, Milano, Italien, eingesetzt, um Gas aus einem Vorratsbehälter zu reinigen. Diese Legierung enthält 5,4 % Eisen, welches bzgl. der $^{3}$He Polarisation stark relaxierend wirkt. Um daher das Eindringen von magnetischem Staub in die Apparatur und damit den Eintrag einer starken Relaxationsquelle zu verhindern, wurde das Gettermaterial im Stand der Technik von Glasfritten mit Porendurchmessern von 40 - 100 µm Durchmesser eingefaßt. Dennoch blieb im Stand der Technik das Risiko der Verunreinigung mit ferromagnetischem Staub.

In einem weiterführenden Gedanken der Erfindung ist daher in der Vorrichtung Reinigungseinheiten mit Verdampfergettern basierend auf nichtferromagnetischen Gettersubstanzen zu verwenden. Zum Beispiel kann Titanmetall als Gettermaterial eingesetzt werden. Auch Wismutmetall ist hierfür ein gutes Gettermaterial. Ein Titan-Sublimationsgetter kann durch Erhitzen eines Titandrahts mittels hohem Stromfluß aktiviert werden. Es dampfen dann reaktive Titanatome ab, die in Form einer reaktiven Schicht auf Oberflächen in Gasreinigungseinheiten als aktiver Adsorber für Nichtedelgase und Dämpfe arbeiten. Durch die Fremdgasbindung wird das frisch sublimierte Titan passiviert, so dass für eine Aktivierung stets neues Titan abgedampft werden muß. Die Abdampfrate ergibt sich aus der anfallenden Verunreinigungsmenge pro Zeit. Um genügend aktive Getterfläche zum selektiven Pumpen zur Verfügung zu haben, wird in geeigneten zeitlichen Abständen eine frische Titanoberfläche abgeschieden.

Bei einem Rohrdurchmesser von beispielsweise einigen cm beträgt die mittlere Diffusionszeit zur getterbelegten Innenwand bei einem Gasdruck von 1 mb und Raumtemperatur Größenordnungsmäßig 1 ms, bei höherem Druck entsprechend mehr. Die Verweilzeit des zu reinigenden Gases im Getter sollte mindestens 1 bis 2 Größenordnungen über dieser mittleren Diffusionszeit liegen. Tatsächlich beobachtet man auch bei Drücken von 40 mb ein instantanes Ausbleichen von heliumfremden Spektrallinien in einem Gasentladungsspektrum, wenn die Titanverdampfung aktiviert wird.

Weitere Gettermaterialen wie Cäsium-oder Rubidium weisen ähnlich gute Sorptionseigenschaften wie Titan oder Wismut auf. Alkalimetalle sind aber aber aufgrund ihres relativ hohen Dampfdrucks bei Raumtemperatur leichter beweglich und greifen Polymere, wie sie als Dichtmaterialien eingesetzt werden, an.

Titan und Wismut, wie auch Cäsium- und Rubidium, tragen zusätzlich die Eigenschaft $^3$He nur schwach zu relaxieren. Wismutbeschichtung von Glaszellen führt sogar zu einer entscheidenden Verlängerung der Relaxationszeiten in Speichervolumen auf 50 h dank seines diamagnetischer Charakters. Die Gefahr, dass beispielsweise magnetische Getterpartikel wie im Falle des Einsatzes von porösem ST 707 Material im Stand der Technik als Aerosole in der Apparatur verteilt werden, ist bei Titan- oder Wismutgettern nicht gegeben. Daher kann auf eine Abgrenzung des Titan- oder Wismutgetterbereichs etwa durch Glasfritten von Volumen in dem polarisiertes $^3$He enthalten ist, verzichtet werden. Hierdurch kann ein guter diffusiver Kontakt von Gas mit dem Gettermaterial und damit eine gute Reinigungseigenschaft sichergestellt werden. Solche Titan- oder Wismutverdampfungsgetter können in folgendem allgemein als nichtmagnetische Verdampfungsgetter charakterisiert werden.

Bei der Auswahl von Wandmaterialien und Formgebung der polarisationsführenden Volumen muß bedacht werden, dass für die effektive Erzeugung höchster Endpolarisationen bei höchsten Produktionsraten eine möglichst gute Polarisationserhaltung nach ihrer Erzeugung erreicht wird. Dies ist gleichbedeutend mit der Minimierung der Relaxation, die durch Oberflächenrelaxation dominiert wird. Die wandbedingte Relaxationsrate $\Gamma_W$ wächst linear mit dem Verhältnis aus Oberfläche zu Volumen gemäß

$$\Gamma_{1,W} = 1 / T_1 = \gamma_W \, O / V \tag{4}$$

worin $\gamma_W$ der für die betreffende Oberfläche spezifische Relaxationskoeffizient ist. Es kommt daher zunächst einmal auf die richtige Auswahl der Materialien an.

In einer Einrichtung zum Kernspinpolarisieren von $^3$He-Gas nach der Methode des direkten "Optischen Pumpens" werden daher vorteilhafterweise Gläser, Titan und Aluminium eingesetzt, als Dichtmaterialien kommen je nach Einsatzort Indium, OHC-Kupferringe, Aluringe und in sehr beschränktem Umfang und kleinstmöglichen Abmessungen Gummiringe aus Viton oder NBR, z.B. als Ventildichtungen, zur Anwendung. Alle Materialien sind nichtmagnetisch und erhalten die Homogenität des äußeren Führungsfeldes. Im Bereich des $^3$He-Plasmas werden - wie oben aufgeführt - mit Vorteil nur geringausgasende, metallische Dichtmaterialien eingesetzt. Umgebungen von bewegten Dichtungen sind aus abriebfestem Titan gefertigt, z.B. Ventile und Kompressor, können aber auch aus Bronze, insbesondere Phosphorbronze hergestellt werden. Diese Materialien zeichnen sich ebenfalls durch ausreichend lange Relaxationszeiten für polarisiertes $^3$He aus. Sammel- oder Speicherzellen werden aus gering relaxierenden Gläsern gefertigt, die sich einerseits gut evakuieren und damit von Fremdgasen wie relaxierendem Sauerstoff gut reinigen lassen. Andererseits zeigen sie geringe Wandrelaxationen und eignen sich daher für lange Polarisationsspeicherungen.

Mit Vorteil zeigen die zuvor beschriebenen Verdampfungsgetter neben den guten materialbedingten Relaxationseigenschaften kleine Gasvolumen mit minimaler intrinsischer Oberfläche, d.h. gute O/V Verhältnisse im Vergleich zu porösen, auch nichtmagnetischen Sorptionsgettern (etwa Aktivkohle oder sogenannte Molekularsiebe, d.h. hochporöse Silikatschichten). Im Gegensatz zu üblichen Sorptionspumpen mit riesigen, aktiven Oberflächen aufgrund poröser Strukturen, wird über die Betriebsweise des Titangetters für genügend aktive Fläche gesorgt. Adsorbierte Fremdgase oder -dämpfe werden mit einer neuen Titanschicht zugedeckt und so konserviert. Mit Vorteil eignen sich daher Verdampfungsgetter als selektive Sorptionspumpen zum weiteren fraktionierten Pumpen im Bereich polarisierter Gase wie z.B. $^3$He. Insbesondere können somit Titan- oder Wismutgetter ganz besonders auch als Reinigungseinheit im Bereich von polarisiertem $^3$He Gas selbst verwendet werden.

Solch nichtmagnetische Verdampfungsgetter zum fraktionierten Pumpen können auch als Reinigungstufe zwischen Gasvorratsvolumen und Optischem Pumpvolumen für eine Vorreinigung eingesetzt werden, um die Fremdgasdichte aus dem Vorratsbereich zu minimieren und damit in der Gasentladung die Erzeugung einer möglichst hohen Dichte an metastabilen $^3$He Atomen zum Erreichen höchster Endpolarisationen zuzulassen.

Mit Vorteil werden mehrere fraktionierte Pumpstufen eingesetzt. Zum einen können größere Volumen zum Optischen Pumpen mit mindestens einer Reinigungsstufe z.B. an Verbindungsrohren zwischen zwei Teilvolumina versehen werden, um bei begrenzter Diffusion, beispielsweise aufgrund höheren Gasdrucks, die Effizienz der Methode zu steigern. Weiterhin kann $^3$He-Gas aus einem Vorrat zunächst in in eine erste Reinigungseinheit geführt werden.

Nach ausreichender Verweildauer wird ein Ventil zum Optischen Pumpvolumen geöffnet, welches, wie oben beschrieben mindestens eine weitere Reinigungseinheit am Eingang des Volumens umfaßt. Diese hat nun die Aufgabe, eindringende Verunreinigungen (Fremdgase) zurückzuhalten und das Gas nachzureinigen.

Stehen Sorptionsgetter im direkten diffusiven Kontakt zum $^3$He-Plasma, so kann mit Vorteil die Effizienz der Sorptionsgetter weiter gesteigert werden, indem die Sorptionsfläche als Kathode negativ zum Plasma vorgespannt wird. Aufgrund des geringeren Ionisationspotentials von Fremdgasatomen gegenüber $^3$He werden diese vorzugsweise ionisiert und als positiv geladene Ionen dann im elektrischen Feld mit Vorteil besonders schnell auf die Sorptionsflächen gezogen.

Ein weiterer Gedanke der Erfindung ist eine Steigerung der Effektivität von Sorptionsgettern durch Kühlung der adsorbierenden Oberfläche, etwa durch eine Flüssig-Stickstoff Kühlfalle. Während die Kühlfalle als Kühlfinger ausgeführt werden kann, besteht der Sublimationsgetter z.B. aus einer darum herum angeordneten Titandraht-Wendel. Der Kühlfinger wird durch Füllen mit flüssigem Stickstoff auf eine Temperatur von 77 Kelvin gekühlt und friert auch unabhängig von der Gettersubstanz Gase mit niedrigem Sättigungsdampfdruck aus. Dazu gehören dominant Kohlenwassersoffe, $CO_2$ und Wasserdampf. Durch die Kühlung der aktiven Metallschicht erfolgt auch die Bindung der flüchtigen Gase wie $N_2$, $O_2$ und vor allem $H_2$ noch effektiver.

Während durch gute Präperation der Optischen Pumpzellen bei Gasdrücken von 1 mb Polarisationswerte von mindestens 50 % erreicht werden können, lassen sich durch Einsatz dieser zusätzlichen Gasreinigungseinheiten die Polarisationswerte weiter steigern. Dabei können jeweils Kühlfinger oder Titansublimations-Getter einzeln oder gemeinsam aktiviert werden. Insbesondere blocken sie aus dem Kompressor oder Ventilen eindiffundierende Fremdgase ab und garantieren einen langen Betrieb ohne Absenkung der Polarisationseffizienz.

Für eine effektive Polarisationserhaltung ist - wie ausgeführt - neben der Auswahl geeigneter Materialien mit kleinem $\gamma_W$ für und innerhalb der polarisationsführenden Leitungen und Behälter die Formgebung derselben wichtig.

Alle Leitungen, die polarisiertes Gas führen, sind aufgrund ihres relativ großen Oberflächen-zu-Volumen-Verhältnisses (O/V) nach Gl. (4) verhältnismäßig starke Relaxationsquellen im Vergleich zu den weiten Räumen wie beispielsweise die Volumina zum Optischen Pumpen, dem Volumen des Kompressors und den Speicherzellen. Allgemein gilt es, die Aufenthaltszeit $T$ einer bestimmten Gasmenge in einem Volumen klein gegenüber der dort angetroffenen Relaxationszeit $T_1$ zu halten, so dass die Polarisation beim Verlassen dieses Volumens

$$P(\tau)=P_0 \cdot \exp(-\tau / T_1) \approx P_0 \qquad (5)$$

also noch nahe der ursprünglichen Polarisation $P_0$ ist.

Insbesondere gilt es gasleitende Volumina $V_L$ klein zu halten gegenüber den durch sie pro Zeit transportierten Gasvolumen $V_G / \tau$. Bei einem diskontinuierlichen Gasaustausch, wie er mit Vorteil in dieser Vorrichtung praktiziert wird, kann $V_G / \tau$ als die innerhalb eines Zyklus' mit Dauer $\tau$ transportierten Gasmenge $V_G$ identifiziert werden. Gemäß dem zuvor beschriebenen Gedanken der Erfindung wird der relative Anteil an Gas $V_L / V_G$, der zwischen dem Gastransport für die Zeitspanne $T$ der Relaxation mit der Rate $\Gamma_1 = 1/ T_1$ dem Totvolumen der Leitung ausgesetzt ist, minimiert. Relaxation durch Wandkontakt während des diskontinuierlichen Flusses kann schon bei Relaxationszeiten von mehreren Minuten vernachlässigt werden. Ziel ist daher eine Minimierung des Leitungstotvolumens $V_L$.

Dieses Ziel wird am besten - auch im Hinblick auf ihren Strömungs- und Vakuumleitwert - durch möglichst kurze Zuleitungen erreicht. Meist ist aber die Länge 1 aufgrund baulicher Vorgaben mit einer minimalen Länge vorgegeben. Für große Flüsse ist der Radius r=d/2 (vgl. Gl. (18) unten) groß zu wählen, andererseits wächst damit das möglichst klein zu haltende Volumen der Leitung. Das verlangt eine optimierte Anordnung der einzelnen Funktionsbereiche zueinander. Andererseits müssen die Radien r der Zuleitungen - im Kompromiss mit den erforderlichen Strömungs- und Vakuumleitwerten - knapp bemessen werden, da das schädliche O/V nur mit r, das schädliche Volumen aber mit $r^2$ skaliert.

Für die Verwendung eines Kompressor zur Kompression von Gasen müssen allgemein einige wichtige Funktionsparameter beachtet werden, die auch bei der Dimensionierung der Zuleitungen eine wichtige Rolle spielen. Das Produkt aus Kolbenquerschnitt und Kolbenhub wird als Hubvolumen $V_{hub}$ bezeichnet. In einer beispielhaften Ausführung wurde ein Kompressorvolumen von $V_{Hub}$ = 15,3 litern gewählt. Dazu im Verhältnis steht das Volumen $V_{L1}$ beispielhafter Zuleitung aus dem Optischen Pumpvolumen, es wurde $\kappa_E = V_{L1} / V_{Hub}$ = 0,0062 erreicht. Gegenüber einem Wert $\kappa_E$ = 0,101 der Vorgänger-Version ist dieser stark geschrumpft, d.h. verbessert; da evt. Relaxationsverluste von angesaugtem Gas in diesem Bereich nun nicht mehr zu Buche schlagen.

Bei vorgezogenem Kolben bei maximaler Kompression wird das restliche Volumen im Arbeitsraum einschließlich dem der Zuleitungen zu den Ventilen Totvolumen $V_{tot}$ des Kompressors genannt. Als effektiver Kompressionsfaktor $K_{eff}$ sei das Verhältnis aus Enddruck $p_{aus}$, der beim Auspressen des Gases in der vorgezogenen Endstellung des Kolbens erreicht wird, und dem Einlaßdruck $p_{ein}$, der sich beim Gaseinlaß in der rückwärtigen Kolbenstel-

lung einstellt, bezeichnet als

$$K_{eff} = P_{aus} / P_{ein}. \tag{6}$$

Als konstruktionsbedingter Kompressionsfaktor $K_0$ sei das Verhältnis aus Hubvolumen und Totvolumen bezeichnet:

$$K_0 = V_{hub} / V_{tot}. \tag{7}$$

Es gilt die Ungleichung

$$K_{eff} \leq K_0. \tag{8}$$

Das Gleichheitszeichen wird erreicht, wenn in der Kompressionsphase auch das Auslaßventil geschlossen bleibt oder ausgangsseitig im Rezipienten der maximal mit dem Kompressor errichbare Druck $p_{aus} = K_0 \, p_{ein}$ erreicht ist. Der relative Anteil $\in$ einer eingelassenen Gasmenge, die beim Kompressionsvorgang nicht in den Rezipienten ausgestoßen wird, sondern im Totvolumen des Kompressors verbleibt, ist gegeben durch

$$\in \, = K_{eff} \, V_{tot} / V_{hub} = K_{eff} / K_0. \tag{9}$$

$\in$ erreicht den Wert 1 bei $K_{eff} = K_0$, d.h. der Gasausstoß ist dann auf Null abgesunken.

Dies muß insbesondere bei der Auslegung der Verbindungsleitung von Kompressor zur Speicherzelle berücksichtigt werden. Mit steigendem Kompressionsdruck, d.h. wachsendem $K_{eff}$, sinkt das Volumen $V_G$ der ausgestoßenen Gasmenge und steigt damit das Verhältnis

$$\kappa_A = V_L / V_G = V_L / V_{hub} \cdot ( 1/K_{eff} - 1/K_0 )^{-1}. \tag{10}$$

In einer beispielhaften Ausführung mit $V_{hub} = 15{,}3$ liter wurde für die den ersten Kompressionsschritt betreffende Leitungen ein Volumen $V_L \approx 8 \, cm^3$ erreicht, sodaß bei typischen Verdichtungen von $K_{eff} \approx 200$ bzw. 500 und dabei auftretenden $K_0 > 10000$ nach Gl.(xx) ein Verhältnis von $\kappa_A = 0{,}10$ bzw. 0,27 erreicht wird. Stand der Technik sind Werte $\kappa_A = 0{,}5$ bzw. 2,5 (mit $V_L \approx 3{,}5 \, cm^3$, $V_{hub} = 1{,}87$ liter und $K_0 = 800$). Die Fraktion $\kappa_A$ der in der Zuleitung für einen Zyklus gefangenen Gasmenge ist in der beispielhaften erfinderischen Ausführung in Folge des viel größeren Kompressionsraumes bei kleiner Auslaßleitung stark reduziert worden. In einem zweiten Verdichtungsschritt von einigen hundert mbar auf wenige bar spielen diese Überlegungen eine sehr viel geringere Rolle, da aufgrund des hier auftretenden kleineren $K_{eff} \approx 10$ bis 100 $K_A$ im Prozentbereich oder gar darunter liegt.

Das Totvolumen $V_{tot}$ im Kompressor ist die gefährlichste Relaxationquelle im Kompressionszyklus, da darin das Oberflächen-zu-Volumenverhältnis Größenordnungsmäßig 1000 mal größer ist als im vollen Hubvolumen $V_{hub}$ des Kompressors und folglich nach Gl. (4) die wandbedingte Relaxationszeit $T_{1,W}$ 1000 mal kürzer ist. Sie fällt damit in den Bereich der Zykluszeit von typisch 20 s oder ist bei ungünstigen $\gamma_W$ noch wesentlich kürzer. Es ist also damit zu rechnen, dass dieser durch Gl. (9) gegebene Anteil des Gases voll relaxiert ist bevor er im nächsten Zyklus frisch eingelassenem Gas beigemischt und mit diesem in eine Speicherzelle transportiert wird. Er beeinträchtigt damit den Transportfaktor $\eta$ zwischen eingangsseitige und ausgangsseitiger Polarisation $P_{ein}$ bzw. $P_{aus}$ auf Werte kleiner 1:

$$\eta = P_{aus} / P_{ein} = 1 - \in \, < 1. \tag{11}$$

Da das Verfahren ausgehend von dem Druck im Polarisationsvolumen ($p \approx 1$ mbar) eine starke Kompression $K_{eff}$ vorgibt, ist eine Maximierung von $K_0 = V_{hub} / V_{tot}$ vorteilhaft. Das Totvolumen resultiert hauptsächlich aus restlichen Spalten vor dem vorderen Kolbendichtring und einer Golddraht-Vakuumdichtung zwischen Zylinder und Zylinderkopf und skaliert daher mit dessen Umfang wie

$$V_{tot} = 2\pi \, r \, \alpha. \tag{12}$$

Typische Werte für den Koeffizienten $\alpha$ bei Verwendung von Doppellippen-Dicht-Gleitringen sind $\alpha \geq 0{,}17\ cm^2$. Aus Gl. (9) und (12) ergibt sich somit für $\in$ die Formel

$$\in = K_{eff}\ 2\ \pi\ r\ \alpha\ /\ V_{hub}. \tag{13}$$

Demnach sollte bei vorgegebenen Hubvolumen $V_{hub} = \pi\ r^2\ h$ der Kolbenradius r zugunsten seines Hubs h klein gehalten werden um das Totvolumen $V_{tot}$ und damit die Polarisationsverluste zu minimieren. Anders ausgedrückt bedeutet dies, das Verhältnis

$$\beta = 2\ \pi\ r\ /\ V_{hub} = (K_0\ \alpha)^{-1} \tag{14}$$

des Umfangs $2\ \pi\ r$ der Kompressorzylinders zum Hubvolumen $V_{hub}$ bei gegebenem $\alpha$ möglichst klein zu gestalten. Beispielsweise sollten mindestens Werte von $\beta < 1/30\ cm^{-2}$, besser $\beta < 1/100\ cm^{-2}$ unterschritten und mit besonderem Vorteil Werte von $\beta = 1/300\ cm^{-2}$ erreicht werden. In einer beispielhaften Ausführung wurde daher bei einem Kompressionsvolumen von 15,3 litern ein langer Kolbenhub von 1,0 m gewählt, resultierend in einem Wert $\beta = 1/350\ cm^{-2}$.

Zunächst hat der schnelllaufende Kompressors mit kürzerem Hubweg gegenüber einem mit längerem Hubweg bei gleichem Durchmesser einen weiteren Nachteil: Nach der Kompression bleibt nach dem oben Gesagten im Totvolumen des Kompressors ein relativer Anteil der eingelassenen Gasmenge gefangenen. Im ersten Fall ist diese gegenüber der ins Sammel- oder Speichergefäß transferierten Menge größer, d.h. ungünstiger. Innerhalb dieses Totvolumens muß, wie obenstehend ausgeführt, aufgrund seines großen Oberflächen zu Volumenverhältnisses mit einer sehr kurzen Relaxationszeit gerechnet werden. Handelt es sich bei der gefangenen Gasmenge also um hyperpolarisiertes Gas, so wird diese Eigenschaft des Kurzhubkompressors zumindest bei kleinen Hubfrequenzen zu erheblichen Polarisationsverlusten führen. Die höhere Hubfrequenz des kleineren Kompressors gleicht aber diesen Nachteil zumindest teilweise wieder aus, weil auch die Verweilzeit im Totvolumen entsprechend kürzer ist, im gleichen Sinne, wie dies schon zuvor bezüglich der Leckverluste diskutiert wurde.

Gemäß einem weiteren Gedanken der Erfindung ist es daher vorteilhaft, beim Pumpen von Gas aus dem Kompressor in eine Speicherzelle ein rechtzeitiges Öffnen des betroffenen, dazwischengeschalteten Ventils anzustreben. D.h., dass das Ventil genau dann geöffnet wird, wenn gleicher Druck im Kompressor und in der Volumen, in das das Gas ausgestoßen wird, herrscht. Würde zu spät geöffnet, so würde unnötig lange unnötig viel Gas in dem gefährlichen Totvolumen stehen bevor es ausgestoßen wird.

Aber auch ein vorzeitiges Öffnen des Ventils muß vermieden werden. Dies träte ein, wenn der Kolben noch nicht genügend komprimiert hätte, und Gas mit höherem Druck aus der Speicherzelle in das Kompressionsvolumen durch das zu früh geöffnete Ventil einströmen könnte. Mit weiterer Kompression wird dieses Gas zwar sofort wieder zurückgepumpt, durch das Hin- und Herströmen durch Volumina mit ungünstigen, d.h. stärker relaxierenden Materialien und/oder ungünstigem O/V-Verhältnissen kann unnötig Polarisation verloren gehen. Mit Vorteil wird daher ein bei Überdruck im Kompressor automatisch öffnendes Ventil eingesetzt. An dem Stößel des Ventils gemäß der Erfindung ist daher ein weiterer Hilfsstößel angebracht. Vor dem Ausstoßen in eine Speicherzelle wird der Stößel des Ventils nur in die mittlere Stellung gefahren, so daß der Hilfsstößel aufgrund eines Überdrucks in der Speicherzelle immer noch in geschlossener Ventilstellung gehalten wird. Überschreitet schließlich der Druck im Kompressionsraum den der Speicherzelle, so öffnet das Gas aus dem Kompressor den Hilfsstößel. Einfahren des Stößels verschließt nach Auströmen des Gases das Ventil zwangsläufig wieder, bevor der Kolben zurück fährt. Zum Ansaugen von Gas aus der Speicherzelle kann der Stößel mit Hilfsstößel in eine garantiert geöffnete Position gefahren werden.

Mit diesem Verfahren sind mittelbare Polarisationsverlustquellen aufgrund von unzeitigem Ventilschaltens ausgeschlossen. Mit Vorteil vereinfacht diese die Prozesssteuerung des Kompressionszyklus', die anderen Falls auf eine exakte zeitliche Steuerung aller Schritte mit Toleranzen in der Größenordnung von Millisekunden gemessen wäre. Eine solche stenge Synchronisierung ist mit der verwendeten Pneumatik oder Hydraulik nicht zu erreichen.

Einem weiteren Gedanken der Erfindung nach kann bei der zuvor dargestellten größeren Baulänge des Kompressors, parallel die gleiche Länge für ein langes Optisches Pumpvolumen genutzt werden und/oder ggf. auf mehrere schmale, parallelliegende Zellen aufgeteilt werden. Die eingangs schon angesprochene Polarisationmethode des Gases beruht auf dem Prozess des Optischen Pumpen (OP). Beim direkten $^3He^*$-OP wird resonantes 1083 nm Laserlicht von metastabilen $^3$He Atomen im $^3S_1$-Zustand absorbiert. Resonant bedeutet, dass das Laserlicht spektral an die dopplerverbreiterte $^3He^*$ Absorptionslinie angepaßt ist, da ansonsten das Licht nicht absorbiert und genutzt werden kann. Bei Drücken um 1 mb wird in dem $^3$He-Plasma nur eine $^3He^*$-Dichte von größenordnungsmäßig $10^{10}\ /\ cm^3$ erreicht. Bei dieser relativ geringen Absorberdichte wird auf die Länge einer OP-Zelle nur der kleinere Teil der eingestrahlten Laserleistung absorbiert, der zudem mit wachsendem Polarisationsgrad rasch abnimmt, z.B. bei P=50 % schon fast um eine Größenordnung. Typischerweise beobachtet man bei Drücken von 1 bis 2 mb und P = 0 % Absorptionlängen $I_0$ von 3 bis 5 m innerhalb der die resonante Laserlichtleistung exponentiell mit dem Weg durch das Gas auf 1/e = 36 % abgefallen ist. Bei Polarisationsgraden von 50 % werden $I_0$ von 15 bis 20 m und bei 60 % $I_0$ von über 60 m beobachtet.

Dem Gedanken der Erfindung folgend kann das OP-Volumen sehr gestreckt werden beispielsweise auf 2,4 m Länge bei Durchmessern von 5 bis 7 cm, um lange Absorptionstrecken zu erhalten und möglichst viel Laserlicht zu absorbieren. Das Licht wird nach einem Durchgang mittels eines Spiegels zurück reflektiert, so dass bei Verwendung von beispielsweise 5 Zellen im Vergleich zur Stand der Technik eine nun dreifache Absorptionstrecke von insgesamt 24 m zur Verfügung steht. Dies resultiert in der Erzeugung einer möglichst großen absoluten Zahl an polarisierten Atomen pro Zeit. Mit Vorteil wird durch die große Streckung der Pumpzellen (bei konstantem Volumen kleinerer Radius) auch die Reabsorption von ausgestrahltem Fluoreszenzlicht minimiert. Dieses ist anzustreben, da Fluoreszenzlicht mit unter den gegebenen Umständen relativ isotrop und stark depolarisiert emittiert wird, und Licht mit der falschen Händigkeit bei einer erneuten Absorption sehr stark depolarisierend wirkt.

Der Spiegel für die Rückreflexion des Laserlichtes ist als dichroitischer Spiegel ausgeführt; hoch reflektierend für 1083 nm, hochtransmittierend für 668 nm. Er wirft einerseits das Pumplicht hinter der OP-Zelle zurück, Fluoreszenzlicht der Wellenlänge $\lambda$=668 nm kann dagegen durch diesen Spiegel hindurchtreten. In einem optischen Polarisationsnachweis hinter dem Spiegel wird der Zirkularpolarisationsgrad dieses Fluoreszenzlichtes gemessen und über Eichfaktoren in die in der Zelle herrschende, absolute Kernspinpolarisation momentan umgerechnet. Das Standardmeßverfahren und die Eichfaktoren sind in den Veröffentlichungen

W. Lorenzon, T.R. Gentile, H. Gao, R.D. McKeown; "NMR calibration of optical measurement of nuclear polarization in $^3$He"; Phys. Rev. A 47:1 (1993) 468 - 479
N.P. Bigelow, P.J. Nacher, M. Leduc; "Accurate optical measurement of nuclear polarization in optically pumped $^3$He gas"; J. Phys. II France 2 (1992) 2159 - 2179
beschrieben.

Der Zirkulationpolarisationsgrad des austretenden Fluoreszenzlichts, welches entlang der optischen Achse aus den Zellen zum Optischen Pumpen emittiert wird, wird im Stand der Technik mittels einer mit Frequenz f rotierenden $\lambda$/4-Platte in linear polarisiertes Licht umgesetzt, dessen Polarisationsrichtung ebenfalls mit Frequenz f rotiert. Nach Durchgang durch einen statischen Linearpolarisator, ein Interferenzfilter und einen Kollimator wird das Licht in einem Photodetektor, beispielsweise einer Photodiode, in ein mit Frequenz 2f amplituden-moduliertes Stromsignal umgewandelt. Nach Verstärkung iin einem Verstärker wird der Modulationshub von einem Lock-In Verstärker nachverstärkt, phasenempfindlich gleichgerichtet und als Meßwert gespeichert. Das aufgrund des unpolarisierten Fluoreszenzlichtanteils vorhandene, von Null verschiedene Mittelwertsignal wird mittels eines Tiefpasses gemessen und als zweiter Meßwert gespeichert. Der Quozient aus beiden Werten ist der gesuchte Zirkularpolarisationsgrad und gibt, dividiert durch einen bekannten druckabhängigen Eichfaktor, den Wert der Kernspinpolarisation an.

Gemäß einem Gedanken der Erfindung vermeidet eine neue Variante des Verfahrens zur Bestimmung des Zirkularpolarisationsgrades mit Vorteil mechanisch rotierende Elemente. Der alternative Aufbau generiert in Abhängigkeit der Fluoreszenzzirkularpolarisation einen maximalen und einen minimalen Spannungswert, deren Differenz, geteilt durch ihre Summe und den Druckeichfaktor wieder die Kernspinpolarisation ergibt. Dazu durchquert das 668 nm Licht zunächst eine statische $\lambda$/4-Platte, anschießend einen Flüssigkristallelement, einen Linearpolarisator, einen 668 nm Interferenzfilter und einen Kollimator bevor es in einem Photodetektor nachgewiesen und verstärkt wird. Ein Rechtecksignalgenerator erzeugt ein positives oder negatives Spannungssignal, das das Flüssigkristallelement bei einer der beiden Spannungen veranlaßt, als $\lambda$/2-Platte zu wirken, bei der anderen nicht. Dadurch wird die Polarisationsebene periodisch um 90° gedreht und vom Analysator abwechselnd der links- und der rechtspolarisierte Anteil des Fluoreszenzlichtes transmittiert und in Abhängigkeit des Steuerspannungssignals des Generators als zweite unterschiedliche Meßsignalwerte gespeichert. Aus diesen beiden Werten kann nun das Kerspinpolarisationssignal wie oben angegeben berechnet werden.

Neben dem Vorteil fehlender rotierender Elemente zeichnet sich dieses Analyseverfahren durch seine hohe Stabilität und Zuverlässigkeit aus, da nur elektronische Veränderliche eingesetzt werden. Gegenüber leichten Verkippungen zur Optischen Achse reagieren beide hier besprochenen Verfahren unempfindlich. Ein weiteres Verfahren der Stand der Technik, das ebenfalls auf rotierende Elemente verzichtet, benutzt anstatt der statische $\lambda$/4-Platte und anschießendem Flüssigkristallelement einen Polarisationsstrahlteilerwürfel und mißt die beiden hierdurch getrennten Polarisationskomponenten auch in getrennten Fotodetektoren und Vertärkerketten. Der gesuchte Meßwert, nämlich der Quotient aus diesen beiden getrennt gewonnenen Signalen, reagiert aber empfindlich auf jegliche Dejustage, wie etwa relative Driften des Koeffizienten der Lichtumwandlung in den beiden Detektionszweigen etc. . Das hier an zweiter Stelle genannte neu Verfahren hat aber wie das zuerst genannten den Vorteil eines gemeinsamen Detektionskanals für beide Polarisationskomponenten und ist daher frei von den genannten systematischen Fehlerquellen.

Wenn das Optische Pumpvolumen in mehrere Bereiche geteilt wird, jeweils mit Volumen des Kompressors, dann kann mit weiter optimierten Produktionsraten polarisiert werden:

Der zeitlichen Verlauf der Polarisationsentwicklung in einer Zelle, in die zunächst unpolarisiertes Helium-3 Gas eingefüllt wurde und dann das resonante, zirkular polarisierte 1083 nm Laserlicht zugeschaltet wurde, zeigt zunächst einen sehr steilen Anstieg der Polarisation, der mit steigender Polarisation zunehmend flacher wird und schließlich in einen Gleichgewichtswert ausläuft. Dieser Wert liegt aufgrund konkurierender Relaxationsprozesse unterhalb von 100 %. Die Relaxationsprozesse bewirken im Fall, dass kein Optisches Pumpen stattfindet, einen exponentiellen Abfall der Polarisation. Je langsamer diese Relaxationsprozesse sind, desto schneller ist der Polarisationszuwachs und desto höher stellt sich die Gleichgewichtspolarisation ein. Zu diesen Relaxationsprozessen zählen im Bereich der Optischen Polarisierung dominant

die Relaxation durch Gasentladungsanregung,

Ionisation,

Excimer- und Molekülbildung,

Abstrahlung polarisierten Fluoreszenzlichts und

Relaxation durch Diffusion in und aus Bereichen mit unterschiedlicher Polarisation.

Letztere spielt damit, ähnlich wie bei der unten besprochenen Diffusion zwischen unterschiedlich polarisierten Bereichen, nach Gl. (17) die Rolle einer effektiven zusätzlichen Relaxationsrate $\Gamma_{1,diff}$. Wandrelaxation und Relaxation durch Diffusion in Feldgradienten (s. Gl. (4),(2) und (3)) spielen in diesem Bereich dagegen eine untergeordnete Rolle.

Da selbst mechanisch langsame Kolbenpumpen mit großem Hub Pumpzykluszeiten von einer halben Minute unterschreiten können, teilt man mit Vorteil das Optische Pumpvolumen in mehrere, seriell verbundene Abschnitte, wobei jeder Abschnitt wieder aus mehreren langen Zellen besteht. Jeweils das Gas aus dem "letzten" Abschnitt mit der höchsten Polarisation wird bei einem diskontinuierlichen Gasaustausch in den Kompressor gespült und anschließend verdichtet. Gas aus dem "vorletzten" Abschnitt kann dann in den "letzten" Abschnitt transferiert und auf höchste Polarisationswerte gebracht werden, was auch "fraktioniertes Optisches Pumpen" genannt wird. Der erste Abschnitt wird gerade mit der weitertransportierten Gasmenge an unpolarisiertem Gas aufgefüllt.

Beispielsweise können zwei Abschnitte gewählt werden. Im ersten Abschnitt wird das Gas vorpolarisiert, im zweiten Abschnitt dann auf hohe Endpolarisationen gebracht. Zum Aufpolarisieren steht somit die doppelte Kompressor-Zykluszeit zur Verfügung.

Wird ein Bereich auf mehrere Zellen aufgeteilt, dann sollte in den jeweiligen Bereichen jeweils ähnliche Bedingungen herrschen, d.h. eingekoppelte Laserleistung wie auch der sich ergebende Polarisationsgrad sollten gleich sein. Letzteres kann durch einen guten diffusiven Gasaustausch innerhalb eines Bereiches begünstigt werden, d.h. zwischen den dazugehörigen Zellen. Um auch einen möglichst schnellen Gaswechsel über hohe Gasflüsse zu ermöglichen sind die jeweiligen Zellen untereinander jeweils mit kurzen Rohrleitungen verbunden. Ein langes Verbindungsrohr zwischen zwei Bereichen sorgt dagegen für einen verminderten diffusiven Gasaustausch und damit für eine weitgehende Entkopplung beider Bereiche. Man vermeidet damit den hohen Aufwand eines Extraventils im Abschitt des Optischen Pumpens und erreicht trotzdem die Minimierung einer zusätzlichen Relaxation durch Diffusion unterschiedlich polarisierter Gase zwischen den Bereichen. Ist

$$T_{diff} = IV / AD \qquad (15)$$

die mittlere Diffusionzeit, charakterisiert für den Austausch von (1-1/e) = 64 % des Gases aus einem Volumen V über eine Verbindungsleitung mit Länge I und Querschnitt A, sowie der Diffusionskonstante D(p) für $^3$He bei gegebenen Druck p, so muß $\tau_{diff} \gg \tau_{zykl}$ mit $\tau_{zykl}$ Dauer eines Kompressionszyklus' gelten. Wählt man beispielsweise zwischen Bereich 1 und 2 je mit Volumen V = 17,5 liter eine Verbindungsrohrlänge I = 60 cm mit Querschnitt A= 3,1 cm$^2$, so ergibt sich mit D = 1700 cm$^2$ / s bei 1 mbar und T = 300 Kelvin eine Diffusionszeit $\tau_{diff}$ = 310 s. Obige Bedingung ist somit erfüllt bei $\tau_{zykl}$ = 20 s . Mit der Zeitkonstanten $\tau_{diff}$ gleicht sich die Polarisationsdifferenz $\Delta P = P_2 - P_1$ zwischen den beiden Bereichen aus gemäß

$$d(\Delta P) / dt = - \Delta P / \tau_{diff} . \qquad (16)$$

Für den Bereich mit der höheren Polarisation spielt diese Diffusion die Rolle einer effektiven Relaxationrate

$$\Gamma_{1,diff} = 1/P_2 \, dP_2/dt = - 1/(2 \, P_2) \, d(\Delta P)/dt = \Delta P/(2 \, P_2) \, 1/\tau_{diff}. \qquad (17)$$

Übliche Werte sind $P_1$ =30 % und $P_2$=50 %, so dass sich in etwa ein $\Gamma_{1,diff}$ von ca. (1500 s)$^{-1}$ ergib. Dies ist eine relativ schwache Relaxationsrate im Vergleich zu der durch die Gasentladung verursachten, die je nach Stärke derselben im

Bereich um $(500s)^{-1} < \Gamma_{Plasma} \leq (200s)^{-1}$ liegt.

Umgekehrt muss beim diskontinuierlichen Gasaustausch für einen hohen Fluß gesorgt werden. Nimmt man laminare Flüsse f an, so skalieren diese bei gegebener Druckdifferenz $(p_2 - p_1)$, mit dem Durchmesser d und der Länge l einer Verbindungsleitung wie $f \propto d^4 / l$ gemäß dem Hagen Poiseullschen Gesetz

$$f = \pi \, d^4 \, (p_2{}^2 - p_1{}^2) / ( 256 \, \eta \, l ) \tag{18}$$

wobei $\eta$ die dynamische Viskosität des Mediums bedeutet. Die Länge ist aus Gründen des maximalen Flusses, also des maximalen Gasaustauschs pro Zeit wiederum zu minimieren.

Zwischen den Zellen eines Bereiches kann ein Rohrlänge von beispielsweise l = 30 cm realisiert werden, stark verkürzt gegenüber von 60 cm im Stand der Technik. Mit steigendem Querschnitt wächst der Fluß quadratisch, reziprok dazu verkürzt sich die benötigte Zeit für Druckausgleich zwischen zwei Volumen. Der Volumenzuwachs der Leitung ist aber nur linear an den Querschnitt gekoppelt. Es läßt sich also ein passender Querschnitt finden, der beispielsweie zu d = 2 cm gewählt werden kann. Selbst bei Differenzdrücken $\Delta p = 1{,}2 - 1{,}0$ mb zwischen Optischen Pump- und Kompressor-Volumen werden gemäß Gl. (18) mit $\eta(^3He) = 19{,}6 \cdot 10^{-6}$ kg/(ms) Spitzenflüsse von f = 15 mbar liter / s in den Kompressor erreicht. Anzusaugende Gasmengen $q = V_{komp} \cdot p_2 = 15$ mbl sind damit sekundenschnell eingelassen. Mit steigendem Druck $p_2$ in dem OP-Volumen wächst der Fluß quadratisch, während die anzusaugende Gasmenge nur linear wächst. Der gewählte Leitungsquerschnitt ist damit ausreichend.

Für die Leitung zwischen den Bereichen ist eine Länge von l=60 cm angenommen worden bei gleichem Leitungsquerschnitt. Da beim diskontinuierlichen Betriebsmodus gleiche Gasmengen bewegt werden, die Leitung aber doppelt so lang gewählt wird, muß ein um Faktor $\sqrt{2}$ größerer Differenzdruck zwischen den Bereich 1 und 2 eingestellt werden. Dies kann aber ohne Abstriche an dem Verfahren sichergestellt werden.

Ein weiterer Gedanke der Erfindung ist die Optimierung durch Anpassung der Zellendurchmesser an die vorhandene Laserleistung und an die in dem entsprechenden Bereich vorherrschende mittlere Polarisation und daran geknüpfte Absorptionslänge.

So läßt sich beispielsweise das Volumen des ersten Abschnitts auf zwei Zellen aufteilen, das Volumen des zweiten Bereichs auf drei Zellen. Bei gleicher Zellenlänge bedeutet dies einen $\sqrt{3/2}$ größeren Zellendurchmesser im ersten Abschnitt verglichen mit dem zweiten. Für einen optimale Polarisierung werden die Zellen möglichst vollständig mit Laserlicht ausgeleuchtet, wofür das Licht im Bereich 1 weiter aufgeweitet werden muß als im zweiten. Dies bedeutet hier aber bei gleicher, in Bereich 1 und 2 eingekoppelter Lichtleistung eine geringere Intensität, in dieser beispielhaften Ausführung nur 2/3 derjenigen von Bereich 2. Eine im Mittel geringe Polarisation von beispielsweise 30 % bis 40 % und damit verknüpfte kürzere Absorptionslänge $I_0$ von 5 bis 10 m in Bereich 1 führt aber zu einer zu Bereich 2 vergleichbaren, absoluten Absorption von Laserlicht und damit zu einem ähnlichen Gewinn an polarisierten Atomen pro Zeit. Dort werden bei mittleren Polarisationen von 50 % Absorptionlängen von 15 bis 20 m angetroffen. Nach der Passage der jeweiligen Bereiche, durch die das Licht sequenziell hindurchgespiegelt wird, ist die ursprüngliche Lichtleistung in beiden Fällen auf größenordnungsmäßig 1/e gefallen. Beide Bereiche sind daher für die im Betrieb angetroffenen mittleren Polarisationen auf effektive Nutzung des Laserlichts optimiert.

Bei gegebener Laserlichtleistung bestimmt der Durchmesser der Zellen letztendlich, welche Gleichgewichtspolarisationen darin erreicht werden können. Hier werden höhere Werte angestebt, wie im Stand der Technik, weshalb beispielsweise für Bereich 1 Zelleninnendurchmesser von 70 mm und für Bereich 2 von nur 54 mm gewählt werden können. Entsprechend ergibt sich ein Gesamtvolumen von 35 litern. Ein korrespondierendes Kompressorvolumen betrüge beispielsweise 17,5 liter, ähnlich dem der beispielhaften Ausführung von 15,3 liter. Damit ist sichergestellt, dass einerseits die Zellendurchmesser an die unterschiedlichen Polarisationsniveaus angepaßt sind, andererseits auch der Zelldurchmesser nicht zu gering für hohe Laserleistungen gewählt wurde. Ist die Intensität sehr groß, so gehen der optische Übergang und damit die absorbierte Leistung in Sättigung und der überschüssige Teil der Lichtleistung läuft ungenutzt durch die Zellen hindurch. Mit der Optimierung gemäß der Erfindung hingegen, entsteht ein optimal großes Optisches Pumpvolumen, wobei dort die Verweilzeit bei gegebener Fördermenge und damit sein Polarisationsgrad maximiert werden.

Zelldurchmesser, wie sie oben angegeben sind, erfordern das Aufweiten und Parallelisieren des Laserlichts, welches vor Eintritt in die Volumen zum Optischen Pumpen vollständig zirkular polarisiert sein muß. Mit Vorteil wird der Laserstrahl zunächst durch einen Polarisationsstrahlteilerwürfel von hoher Qualität linear polarisiert und anschließend mittels einer passend justierter $\lambda/4$-Platte zirkularpolarisiert. Ein nachfolgendes Teleskop weitet den Strahl auf den geforderten Durchmesser auf und bildet den Zwischenfokus auf den gegenüberliegenden Spiegel ab, dabei das Zellenvolumen möglichst gut ausfüllend, d.h. schwach konvergent. Bei langen Wegstrecken durch Zellen, beispielhaft zu $2^* \, 240$ cm gewählt, ist es vorteilhaft, den Strahl nach Hin und Herspiegelung durch eine Zelle wieder durch Linsen zu kollimieren, bevor er in eine weitere Zelle eingespiegelt wird. Durch eine solche Linsenstraße wird trotz an die Zelldurchmesser angepaßter Strahldurchmesser eine Berührung des Lichts mit den Zellwänden vermieden. Mit Vorteil wird dabei eine ebenfalls möglichst weite Strahltaille an den Ort des rückwärtigen Spiegels gelegt. Ansonsten würde Reflexion des Lichts an dem Zellenrohr den Zirkularpolarisationsgrad vermindern und zusätzlich relaxierend wirken.

Vorteilhafterweise besteht das Teleskop aus einer Konkavlinse und einer Konvexlinse, was die Baulänge des Teleskops verkürzt und die Divergenz auf relativ kleine Winkel beschränkt. Auf letztere ist zwecks Erhaltung der Zirkularpolarisation trotz Entspiegelung der Linsen zu achten.

Vorteilhafterweise wird der Laserstrahl nacheinander durch alle Zellen eines Bereichs justiert, um die angebotene Absorptionslänge wie oben erläutert voll auszunutzten. So wird möglichst viel Licht genutzt und insbesonderen dem Bereich mit der höchsten Polarisation und der damit verbunden geringsten Absorptionswahrscheinlichkeit zu einer höchsten Polarisationsrate verholfen.

Die OP-Zellen sind aus geeignetem Material gefertig, was zumindest an den Ein- und Austrittsfenstern für 1083 nm Licht transparent ist. Um Radiofrequenzfelder zur Erzeugung des Helium-Plasmas extern einkoppeln zu können, werden nichtleitende Materialien bevorzugt. Mit Vorteil werden die Zellen aus Glas, mit weiterem Vorteil aus unempfindlichem Duranglas (von Fa. Schott Glas) oder Pyrex (von Fa Corning) gefertigt. Glas läßt sich zusätzlich zu den vorgenannten Eigenschaften bei hohen Temperaturen (Duran von bis zu 520°C) ausheizen und evakuieren, was Fremdgase aus den oberflächennahen Schichten entfernt. Zusätzlich können durch Einfüllen einiger mb an Helium Gas und Brennen einer HF- oder Mirkowellenentladung letzte Fremdgase von der inneren Zellenoberfläche gelöst und durch Abpumpen entfernt werden. Während des Polarisationsbetriebes wird dann das $^3$He Gas nicht mehr durch Fremdatome verunreinigt, die durch Plasmaentladung oder Ausgasen aus der Wand freigesetzt werden. Im Plasma wird dann eine optimal lange Lebensdauer resp. Dichte an metastabilen $^3$He-Atomen erreicht, eine Grundvoraussetzung für hohe Endpolarisationen und Polarisationsraten.

Alternativ sind auch nichtmagnetische, metallische Plasmazellen mit transparenten Fenstern möglich. Die Fenster werden dabei über Metalldichtungen gedichtet (z.B. Golddraht), die auch für den Ausheizprozess hitzebeständig sind. Für diese Art von Zellen werden innenliegende Elektroden verwendet, um ein Plasma zu brennen. Alternativ kann auch eine elektrodenlose Mikrowellenentladung mit Vorteil in solche metallische Zellen eingekoppelt werden, die dann als Hohlleiter fungieren.

Um Lichtverluste durch Absorption in den Fenstern zu vermeiden sind mit weiterem Vorteil die Ein- und Austrittsfenster der OP-Zellen aus optischem Glas, z.B. ZKN 7, gefertigt, welches sich mit Vorteil mittels Übergangsgläsern mit Duranglas verschmelzen läßt. Eine weitere Ausführung besteht aus "Borofloatglas", welches sich direkt als optisch planes Fenster an Duranröhren ansintern läßt.

Mit Vorteil sind die Polarisations-Optiken ebenfalls entspiegelt, um Lichtleistungsverluste durch Reflexion an den Ein- und Austrittsflächen zu vermeiden. Mit weiterem Vorteil sind die Fenster zumindest auf der Außenseite entspiegelt, was die sonst üblichen Reflexionsverluste von typisch 4 % der Lichtleistung an jeder Grenzfläche vermeidet.

Auch die Linsen in den Teleskopen sind mit Vorteil für 1083 nm Licht entspiegelt. Dies dient nicht nur der Vermeidung von Reflexionsverlusten, sondern auch der Erhaltung des Lichtpolarisationsgrades. Bei Brennweiten von -100 mm für die erste Linse und 300 mm für zweite Linse z.B. errechnet man ohne Entspiegelung zwar nur eine Depolarisation um ca. 0,5 %, jedoch reagiert die maximal erreichbare Gleichgewichtspolarisation sehr empfindlich auf kleine Beimischungen von zirkular polarisiertem Licht mit umgekehrten Drehsinn (Details sind in der schon oben zitierten Veröffentlichung von Eckert et. al. 1992 beschrieben). Tatsächlich konnte in der beispielhaften Ausführung keine Änderung der Gleichgewichtspolarisation beobachtet werden, wenn die Positionen des Teleskops und der Polarisationsoptik vertauscht wurden, sodass jegliche depolarisierende Wirkung des Teleskops ausgeschlossen wurde. Das erforderte allerdings eine sehr teure Polarisationsoptik mit großem, auf den Zellendurchmesser angepaßten Querschnitt. Die in der beispielhaften Ausführung eingesetzen, kostengünstigen kleinen Polarisations-Optiken mit nur 20 mm Kantenlänge erlauben daher in Verbindung mit einem Teleskop mit Vorteil eine polarisationserhaltende Aufweitung des Strahls auf die für optionales Optisches Pumpen erforderliche Querschnitte.

Nicht nur in den Volumen des Optischen Pumpens, sondern auch in den Speicherzellen für polarisiertes $^3$He-Gas müssen kleinste Verunreinigungen an Sauerstoff vermieden werden, da dieser die erzeugte Hyperpolarisation mit einer Rate $\Gamma_1$ proportional zu seinem Partialdruck $p_{O2}$ abbauen würde, welche nach

B. Saam, W. Happer and H. Middleton; "Nuclear relaxation of $^3$He in the presence of $O_2$"; Phys. Rev. A 52 (1995) 862 - 865

bei 20°C Raumtemperatur gegeben ist durch die Gleichung

$$\Gamma_{1,O2} = 1 / T_1 = p_{O2} / (\text{bar } 2{,}25 \text{ s}). \tag{19}$$

Danach wird z.B. bei $p_{O2}$ = 2·10$^{-6}$ mbar, entsprechend einem mittleren Hochvakuumbereich, die Sauerstoffbedingte Relaxationszeit schon auf $T_{1,O2}$ = 312 h beschränkt. Die entsprechende Relaxationsrate von $\Gamma_{1,O2}$ = (312 h)$^{-1}$ trüge schon beträchtlich zu einer mindestens angestrebten Gesamtrelaxationsrate von $\Gamma_1$ = (100 h)$^{-1}$ bei. $T_1$ bezeichnet diejenige Zeit, nach der die Polarisation gemäß einem zeitlichen Exponentialgesetz auf einen Bruchteil von 1/e ihres Ausgangswertes gefallen ist.

Mit Vorteil werden in einer Vorrichtung mehrere Pumpsystem eingesetzt, um das fraktionierte Pumpen und die Apparaturpräparation vakuumtechnisch zu trennen. Damit wird die Diffusion von Fremdgasen oder - dämpfen zwischen den jeweiligen Vakuumbereichen der Apparatur vermieden und höchste Gasreinheiten in den Volumen für Optische Polarisierung von $^3$He und dessen Speicherung erreicht. Zu unterscheiden sind die Bereiche, die im regulären Betrieb der Anlage das $^3$He enthalten von den Zwischenvolumina der Ventile und des Kompressors und dem Rückraum des Kompressors, der den Öldämpfen aus dem Bereich des Antriebs ausgesetzt ist. Zweckmäßigerweise wird das Zwischenvolumen des Kompressors zusammen mit den Zwischenräumen der Ventile von einer Pumpe abgepumpt. Diese Anordnung erlaubt es zusätzlich, Zwischenräume der Ventile und des Kompressors ständig zu bepumpen, unabhängig vom Bedarf der Evakuierung relevanter $^3$He Arbeitsvolumen. Weiterhin werden das Leitungssystem für die Evakuation des $^3$He Arbeitsvolumen niemals mit Fremdgasen aus den Zwischenräumen der Ventile und des Kompressors verunreinigt. Dies steigert die Zuverlässigkeit, mit der die erforderliche Reinheit des $^3$He im $^3$He-Arbeitsvolumen erreicht und auf längere Sicht aufrecht erhalten werden kann, was Vorraussetzung für lange, störungsfreie Betriebsphasen und damit für hohe mittlere Produktionraten ist.

Die Erfindung soll nunmehr anhand der nachfolgenden Figuren beispielhaft beschrieben werden, ohne dass hierin ein Einschränkung zu sehen ist.

Es zeigen:

**Fig.** 1 : eine prinzipielles Schema des Polarisations- und Kompressionsprozesses auf Basis der Methode des direkten Optischen Pumpens von $^3$He ;

**Fig.** 2 : ein prinzipielles Schema des Polarisators und Kompressors auf Basis der Methode des direkten Optischen Pumpens von $^3$He nach Stand der Technik;

**Fig.** 3 : die für den Polarisationsprozess wesentlichen Elemente des erfindungsgemäßen Polarisators und Kompressors auf Basis der Methode des direkten Optischen Pumpens von $^3$He;

**Fig.** 4a,b : einen Schnitt durch eine erfindungsgemäßes Element mit einem beweglichen Bauteil, ausgeführt als alleinstehendes Ventil - in geschlossener und geöffneter Position;

**Fig.** 4c : einen Schnitt durch eine erfindungsgemäßes Element mit mehreren beweglichen Bauteilen, ausgeführt als Ventilgruppe - in geschlossener und geöffneter Position;

**Fig.** 5a,b : einen Schnitt durch eine erfindungsgemäßes Element mit einem beweglichen Bauteil, ausgeführt als einstufiger Kolbenkompressor, mit Illustration von Dichtelementen und äußeren Pumpanschluß;

**Fig.** 6a,b : einen Schnitt durch eine erfindungsgemäßes Element mit einem beweglichen Bauteil, ausgeführt als einstufiger Kolbenkompressor, mit Illustration eines hydraulischen Antriebs mit Dopplekammersystem;

**Fig.** 7 : ein prinzipielles Schema des erfindungsgemäßen Polarisators und Kompressors mit optimiertem Pumpensystem;

**Fig.** 8a,b : einen Schnitt durch eine erfindungsgemäßes Element mit einem beweglichen Bauteil, ausgeführt als Ventil, wie es Teil einer Baugruppe im Zylinderkopf des Kompressors als Einlaßventil und Ventil zum Pumpanschluß dient - in geschlossener und geöffneter Position;

**Fig.** 9a-d : einen Schnitt durch eine erfindungsgemäßes Element mit einem beweglichen Bauteil, ausgeführt als Ventil, wie es Teil einer Baugruppe im Zylinderkopf des Kompressors als Auslaßventil dient. Es kann automatich bei Überdruck öffnen was durch die gezeigten unterschiedlichen Positionen illustriert wird;

**Fig.** 10 : die maximal erreichbare Kompression $K_0$ in Abhängigkeit des Ausgangsdrucks in einer Speicherzelle;

**Fig.** 11a,b : schnell laufende Kolbenkompressoren mit kleinerem Hubvolumen und kürzerem Hubweg in zwei Versionen mit außen- und innenliegendem Antrieb;

**Fig.** 12 : den zeitlichen Verlauf der Polarisationsentwicklung in einer der OP-Zelle, wenn zunächst unpolarisiertes Helium-3 Gas eingefüllt und dann das resonante, zirkular polarisierte 1083 nm Laserlicht zugeschaltet wurde;

**Fig.** 13 : das Schema eines Berereichs eines Optischen Pumpvolumens, mit Polarisationsoptiken, Linsenstraßen und serieller Laserstrahlführung durch zwei OP-Zellen;

**Fig.** 14 : das Schema des Meßaufbaus zur Analyse des Zirkularpolarisationsgrades von 668 nm Fluoreszenzlichts, zur Bestimmung des Kernspinpolarisationsgrades in einer alten und einer erfinderischen neuen Realisierung;

**Fig.** 15 : den zeitlichen Verlauf der Polarisation in OP-Zellen, wenn das Gas diskontinuierlich oder kontinuierlich ausgetauscht wird;

**Fig.** 16 : die beispielhafte Abhängigkeit erreichbarer Endpolarisationen in OP-Zellen in Abhängigkeit des $^3$He-Gasdruckes und des benutzten optischen $^3$He$^*$-Übergangs C8 oder C9;

**Fig.** 17 : die mit einer erfinderischen Vorrichtung beispielhaft erreichbare Polarisation und beispielhaft erreichbare Menge an polarisiertem $^3$He-Gas in Abhängigkeit des Gasdurchsatzes.

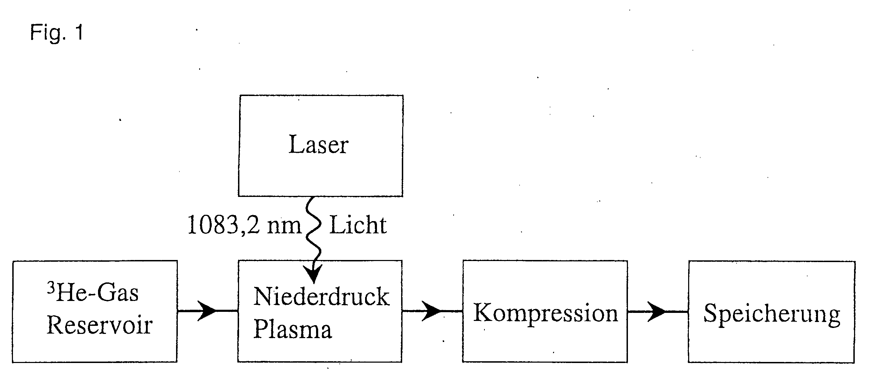

Fig. 1 skizziert die Bereitstellung große kernspinpolarisierter $^3$He-Gasmengen nach der Methode des direkten Optischen Pumpens von $^3$He. $^3$He-Gas wird einem Reservoir entnommen und und bei einem Druck von ca. 1 mb in einer Plasmaentladung durch Absorption von zirkularpolarisiertem 1083,2 nm Laserlicht kernspinpolarisiert. Anschließende Kompression und Speicherung stellen das polarisierte Gas für eine Reihe sehr verschiedener Zwecke in der physikalischen Grundlagenforschung wie in der medizinischen Anwendung zur Verfügung.

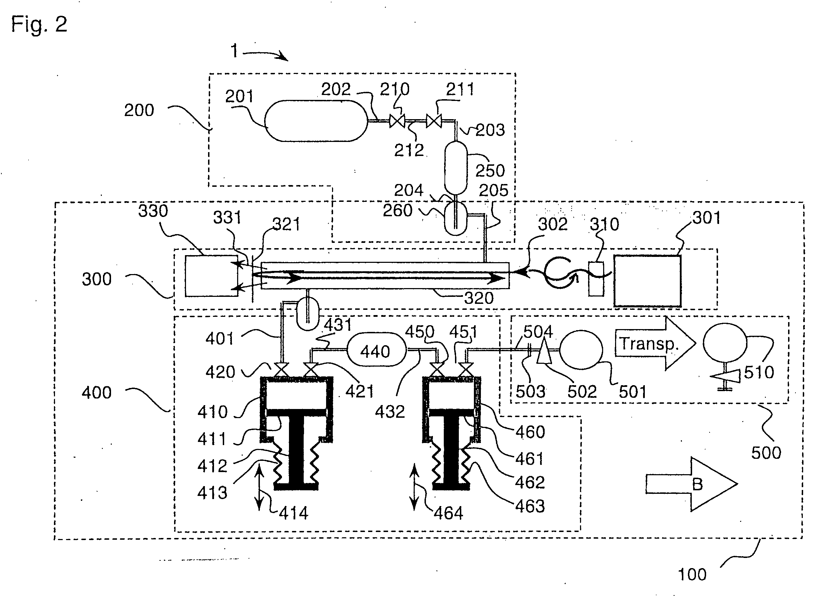

Fig. 2 skizziert den Polarisator und Kompressor (1), wie nach dem Stand der Technik bekannt ist. Er umfaßt 5 Baugruppen: Gas wird aus einem Gasversorgungssystem 200 entnommen, in einer Polarisationseinheit 300 bei Drücken zwischen 0,5 und 5 mb kernspinpolarisiert, in einer Kompressionseinheit 400 auf Enddrücke von bis zu 10 bar verdichtet und schließlich in einer angeschlossenen Speichereinheit 500 für längere Zeiträume zwecks Transports und Anwendung aufbewahrt. Eine Vorraussetzung zur Polarisationserhaltung über längere Zeiträume ist die Einbettung der polarisationsführenden Systeme 300, 400, 500 in einem homogenen Magnetfeld, der Baugruppe 100. Es werden Feldstärken von typischer Weise 0,8 mT verwendet.

Das Magnetfeld 100 besteht aus 5 Ringspulen gleichen Durchmessers mit geeigneter Amper-Windungszahl und Abständen zueinander. Die jeweils beiden äußeren Spulenringe sind symmetrisch zur mittleren Spule axial angeordnet. Alle 5 Spulenringe sind in Serie geschaltet, sodass ein einheitlicher Strom alle Spulen durchfließt. Die benötigte Ampere-Windungszahl wird dann für jede Spule anhand geeignet gewählter Drahtwicklungszahlen eingestellt. Dies hat den Vorteil, den relativen Beitrag einjeder Spule zum Gesamtfeld trotz evt. kleiner Stromdrifts konstant zu lassen und damit verlässlich ein homogenen Feldbereich innerhalb des Spulenvolumens für die Apparatur aufzubauen.

Das Gasversorgungssystem umfaßt ein $^3$He-Reservoir 201, das über eine Leitung 202 mit einem ersten Ventil 210 verbunden ist. Dieses bildet mit Volumen 212 und Ventil 211 eine Schleuse, die periodisch Gas aus dem Reservoir 201 entnimmt und in Leitung 203 mit niedrigerem Druck entläßt. Damit wird ein $^3$He-Fluß erzeugt, der in der Reinigungsstufe 250 von Fremdgasen außer Edelgasen gesäubert wird. Hierin wird hochporöses Gettermaterial St 707 der Firma SAES, Milano, verwendet, das in einem ersten Teil bei etwa 250°C vermehrt Moleküle adsorbiert, sie in ihre atomaren Bestandteile zerlegt und diese festhält. In einem zweiten Teil senkt Gettermaterial bei Raumtemperatur den Wasserstoffpartialdruck auf sehr kleine Werte ab, d.h. Wasserstoff wird effektiv im Getter adsorbiert. Nachfolgend wird das gereinigte $^3$He Gas durch eine Kapillare 204 und eine Flüssigstickstoffkühlfalle 260 über Leitung 205 in den Bereich 300 geleitet. Die Kapillare 204 formt über ein Druckgefälle einen kontinuierlichen Fluß in den Bereich 300 und verhindert andererseits eine Rückdiffusion von polarisiertem Gas aus Bereich 300 in die Reinigungseinheit 250 und Gebiete außerhalb des Magnetfeldes 100, die die Polarisation schnell zerstören würden.

In der Polarisationseinheit 300 wird in 4 zylinderförmigen, 1 Meter langen und 75 mm durchmessenden Zellen 320 bei Drücken zwischen 0,1 und 3 mbar über das Einstrahlen von elektrischen Hochfrequenzfeldern eine Gasent-ladung gezündet und metastabiles $^3$He Gas erzeugt ($^3$He$^*$). Dieses $^3$He$^*$ absorbiert resonant 1083,2 nm Licht 302 aus einer geeigneten Lichtquelle 301 (beispielsweise eines cw Nd:LMA Festkörperlasers), das über Polarisationsoptiken 310 zirkular polarisiert und entlang des äußeren Magnetfeldes 100 eingestrahlt wird. Der über die Lichtabsorption übertragene Drehimpuls richtet den Kernspin in Abhängigkeit der Rechts- oder Linkshändigkeit des Lichtes parallel (oder antiparallel) zum Magnetfeld aus.

Das Laserlicht wird nach einmaligem Durchgang durch eine Zelle 320 an einem dichroitischen Spiegel 321 reflektiert, nichtabsorbiertes Licht steht damit für einen 2. Durchlauf zur Verfügung. 668 nm Fluoreszenzlicht kann dagegen durch diesen Spiegel hindurchtreten. In einem optischen Polarisationsnachweis 330 wird der Zirkularpolarisationsgrad dieses Fluoreszenzlichtes gemessen und über zum Teil druckabhängige Eichfaktoren in die gerade vorherrschende, absolute Kernspinpolarisation umgerechnet.
Die Zellen sind seriell miteinander verbunden, so dass das Gas langsam durch diese hindurchströmt und stetig an Polarisation gewinnt.

Über eine weitere Flüssigstickstoff-Kühlfalle und Verbindungsrohr 401 wird das polarisierte $^3$He Gas peri-odisch z.B. alle 10 s über Ventil 420 in den Kompressionsraum der ersten Kompressionsstufe 410 eingelassen, mittels Vorschub des Kolbens 411 verdichtet und über Ventil 421 in eine Sammelzelle 440 ausgestoßen. Der Antrieb des Kolbens erfolgt über einen Pressluft-Linearmotor (schematisiert durch 414), der über die Endplatte des Balges 413 und die Kolbenstange 412 die Bewegung auf den Kolben im ultrahochvakuumdichten Kompressor überträgt. Alle Komponenten 410 bis 412 sind aus Titan gefertigt, um Polarisationsverluste durch Wandkontakt mit magnetischem Material zu minimieren und andererseit ein Ultrahochvakuum taugliches und abriebfestes Material zu verwenden. Der Kolben mit Durchmesser 140 mm und Hub 110 mm ist durch einen "Quadring" der Fa. Busak & Shamban gedichtet und gelagert. Die Sammelzelle 440 wird standardmäßig auf etwa 200 mb gefüllt bevor eine im Prinzip baugleiche zweite Kompressionsstufe 460 mit Ventilen 450 und 451, Kolben 461 mit Durchmesser 60 mm und Hub 94 mm, Kolbenstange 462, Balg 463 und Antrieb 464 das Gas auf Enddrücke von 1 bis 10 bar verdichtet. Diese führt pro Sammelzellenfüllung 6 Kompressionszyklen aus mit einer Periode von ebenfalls 10 s und entleert damit die Sammelzelle auf einen Mini-maldruck $p_{min}$.
Das von der Kompressionsstufe 460 hochverdichtete Gas wird über Leitung 504 in eine, an Flansch 503 angeschlossene Speicherzelle 501 gepumpt. Diese Speicherzelle kann über Ventil 502 verschlossen und anschließend für Transportzwecke vom Polarisator (1) gelöst werden. Solche transportable Zellen 510 werden auch zur langen Aufbewahrung des Gases eingesetzt. Eine beispielhafte Ausführung für den medizinischen Einsatz ist daher z.B. aus geeignetem eisenarmen und diffusionsfestem Supremax Glas der Fa. Schott Glas gefertigt, worin Relaxationzeiten von bis zu 100 Stunden erreicht werden können. Weiterhin können auch durch geeignete Innenbeschichtungen die Relaxationszeit verlängert und Speicherzeiten von bis zu 200 Stunden erreicht werden.

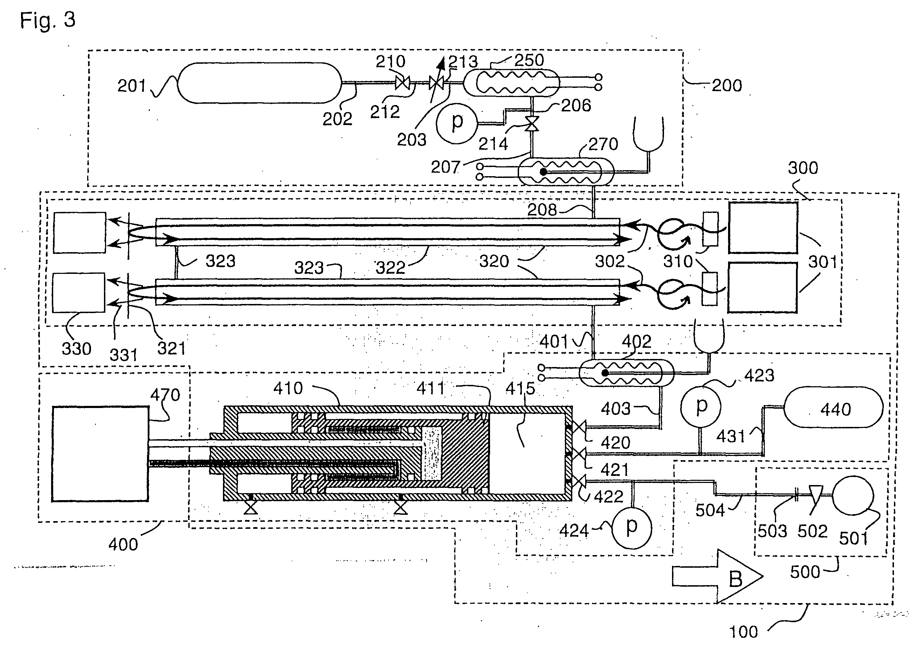

Fig. 3 zeigt die für den Polarisationsprozess wesentlichen Elemente des erfindungsgemäßen Polarisators und Kompressors auf Basis der Methode des direkten Optischen Pumpens von $^3$He. Das Gasversorgungssystem umfaßt ein $^3$He-Reservoir 201, das über eine Leitung 202 mit einem ersten Ventil 210 verbunden ist. Dieses ist über die Leitung 212 mit einem Dosierventil 213 verbunden, welches einen Fluß f regeln kann, so dass über eine Zeitspanne $\tau = q / f$ eine gewünschte Gasmenge $q = p^*V$ in das Volumen V 250 bis zum Erreichen des gewünschten Druckes p in der Polarisationseinheit 300 eingefüllt werden kann. Volumen 250 enthält eine Gasreinigungseinheit in Form eines Titanverdampfergetters. Von einem Heizfaden wird bei Gasdrücken zwischen 0 und 100 mb reines Titan auf eine Oberfläche abgedampft, die Fremdgase außer Edelgasen bindet. Über ein Ventil kann ein Druckausgleich mit dem Volumen 320 hergestellt werden und aufgrund des geringeren Drucks in 320 ein Großteil der Gasmenge q in die Polarisationseinheit 300 eingebracht werden. Zwischen Ventil 213 und der Polarisationseinheit befindet sich eine weitere Gasreinigungseinheit 270 im ständigen Kontakt mit Volumen 320.

Die Polarisationseinheit 300 umfaßt in der beispielhaften Ausführung mehrere ca. 2,5 Meter lange und seriell, über Verbindungsrohre 323 miteinander verbundene Zellen 320 aus Glas, in denen bei Drücken von 0,5 bis 5 mbar, vorzugsweise um 1 mbar, über das Einstrahlen von elektrischen Hochfrequenzfeldern eine Gasentladung gezündet und metastabiles $^3$He Gas erzeugt ($^3$He$^*$) wird . Dieses $^3$He$^*$ absorbiert resonant Licht 302 der Wellenlänge $\lambda$=1083,2 nm aus einer geeigneten Lichtquelle 301, beispielsweise einem cw Nd:LMA Festkörperlasers, das über Polarisationsoptiken 310 zirkular polarisiert und entlang des äußeren Magnetfeldes 100 eingestrahlt wird. Der über die Lichtabsorption übertragene Drehimpuls richtet den Kernspin in Abhängigkeit der Rechts- oder Linkshändigkeit des Lichtes parallel oder antiparallel zum Magnetfeld aus. Das Laserlicht wird nach einmaligem Durchgang durch eine Zelle 320 an einem dichroitischen Spiegel 321 reflektiert. Nichtabsorbiertes Licht steht damit für einen 2. Durchlauf zur Verfügung. Fluoreszenzlicht der Wellenlänge $\lambda$=668 nm kann dagegen durch diesen Spiegel hindurchtreten. In einem optischen Polarisationsnachweis 330 wird der Zirkularpolarisationsgrad dieses Fluoreszenzlichtes gemessen und über Eichfaktoren in die in der Zelle herrschende, absolute Kernspinpolarisation momentan umgerechnet.

Über Verbindungsrohre 401, 403 einer weiteren Gasreinigungeinheit 402 und Ventil 420 kann Gas in den evakuierten Kompressionsraum 415 des Kolben-Kompressor 410 eingelassen werden. Durch Vorschub des Kolbens 411 wird das Gas verdichtet und über Ventil 421 oder 422 über Leitung 431 oder Leitung 504 in die Sammelzelle 440 oder Speicherzelle 501 ausgestoßen. Letztere kann für Transport- und/oder Anwendungszwecke mit Hahn 502 verschlossen und von Flansch 503 getrennt werden. Der Kolben wird hydraulisch bewegt, wofür ein Öldruck von einem Aggregat 470 zur Verfügung gestellt wird.

Bei ausreichender Relaxationszeit der Polarisation in der Speicherzelle, worunter die Zeitkonstante des exponentiellen Abfalls der Hyperpolarisation in Richtung der Boltzmann Polarisation $P_B$ (GI.(1)) verstanden wird, hat die Trennung der Speicherzelle von der hier vorgestellten Abfüllapparatur denVorteil, dass die Präparation und Anwendung des Gases räumlich und zeitlich getrennt werden können. Dies ist von großem Vorteil für die Anwendung des Gases, da am Anwendungsort i.A. die Bedingungen für eine effektive Polarisierung des Gases nicht vorhanden sind (z.B. Investitionsmittel, qualifiziertes Personal, entsprechend installierte Räumlichkeiten, etc.). Eine Trennung bietet auch die Möglichkeit, sehr effizient an einem Ort das Gas zu präparieren, während die Anwender sich nur auf den Einsatz des Gases konzentrieren müssen. Die Qualitätssicherung läßt sich so am Ort der Herstellung bündeln, ohne die Anwendung des Gases einzuschränken.

Mit Vorteil sind nichtmagnetische Druckmessgeräte 423, 424 in der beispielhaften Ausführung in den Kopf des Kompressors zusammen mit den Ventilen integriert. Druckmessgeräte in eigenständigen Gehäusen würden ansonsten nur unnötig zum Totvolumen der Leitungen beitragen.

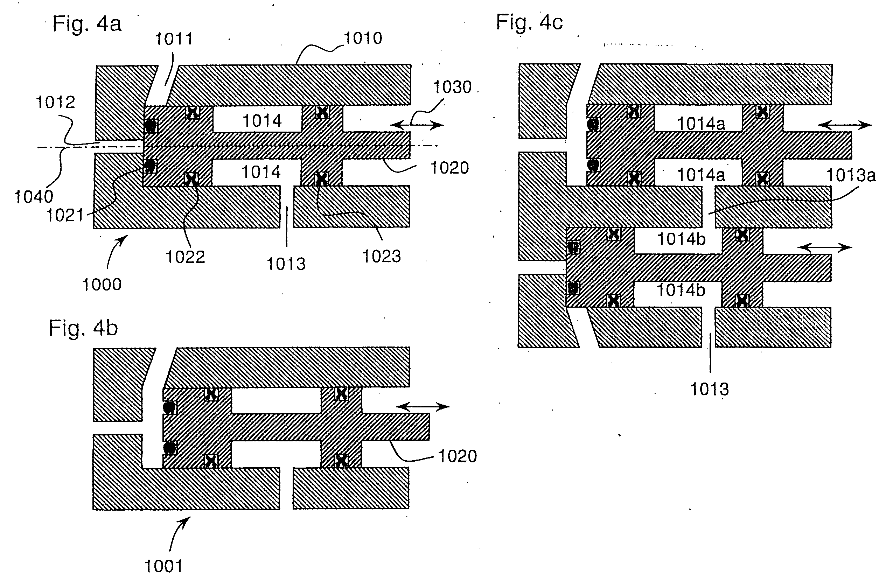

Fig. 4a und 4b illustrieren ein kostengünstiges, platzsparendes und insbesondere für alle Dimensionen zuverlässig arbeitendes Prinzip der UHV-tauglichen Linearbewegungsdurchführung an Hand eines Ventilschemas. Fig. 4a zeigt den Schnitt durch ein geschlossenes Ventil (1000) und Fig. 4b den durch ein geöffnetes Ventil (1001) zeigt. In einer bevorzugten, einfach herzustellenden Bauform ist das Ventil bis auf die Ventilbohrungen 1011 und 1013 rotationssymetrisch um die Symetrieachse 1040 ausgeführt.

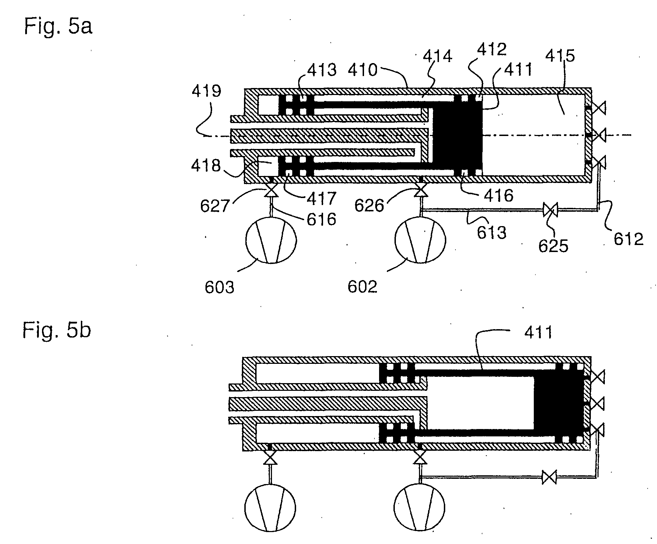

Innerhalb des Ventilgehäuses 1010 wird ein Ventilstößel 1020 von einem externen Antrieb 1030 linear bewegt. Der Stößel verschließt in der geschlossenen Stellung die Verbindung zwischen Ventilbohrungen 1011 und 1012. Mit Vorteil sorgt eine Gleitdichtung, z.B. ein Dichtring 1021 aus z.B. Gummi in Form eines O-Rings, vorzugsweise aus Viton, für ausreichende Dichtung auch bei Druckdifferenzen von bis zu 10 bar. Weitere Dichtringe 1022 und 1023 trennen den eigentlichen Ventilbereich 1011 und 1012 von einem Ventilrückraum 1014 und der äußeren Atmosphäre mit Ventilantrieb 1030 ab. Diese Dichtringe können mit Vorteil als Doppellippendichtringe ausgeführt werden. Über Öffnung 1013 wird der Rückraum 1014 evakuiert. Dies erfolgt beispielsweise über Leitung 612, Ventil 625 und die Pumpe 602 (Fig. 5a). Dieses Dichtungsprinzip mit selektivem Bepumpen von Zwischenvolumen wird im Folgenden "fraktioniertes Pumpen" genannt.

Mit Vorteil ist Pumpe 602 als ölfreie Turbomolekularpumpe ausgeführt. Dichtring 1022 hat zusätzlich den Vorzug, das eigentliche Ventilvolumen zwischen Bohrung 1011 und 1012 zu minimieren, was für den Transport hyperpolarisierten Gases von besonderer Bedeutung ist, wie weiter unten ausgeführt wird. Beide Dichtringe 1022 und 1023 übernehmen zusätzlich die Führung des Ventilstößels.

Diese Konstruktion vermeidet einen aufwendigen und anfälligen Balg als UHV-Abtrennung zwischen Atmosphäre und hochreinem Gasraum. Über den Dichtring 1023 einleckende Luft wird über Pumpe 602 abgepumpt. Eine restliche Diffusion von Restgasmolekülen aus Rückraum 1014 über Dichtring 1022 in den eigentlichen Ventilraum 1011, 1012 ist vernachlässigbar und deutlich geringer als die Desorption aus den Metalloberflächen und Dichtringen 1021 und 1022. Ein wichtiges Merkmal dieser Konstruktion im Zusammenhang mit den genannten Desorptionsraten liegt darin, dass der Abstand der Dichtringe, die das Zwischenvakuum begrenzen, größer ist, als der Hub des Stößels. Auf diese Weise wird verhindert, dass Teile der Ventilinnenoberfläche, die sich außerhalb des Vakuums durch Adsorption mit Gasen beladen, auch Teil des hochreinen Innenraums werden können und dort die adsorbierten Gase wieder desorbieren. Vielmehr wird dieser Gastransport via dieses Adsorptions- Desorptionszyklus im Zwischenvakuum abgefangen.

Die Ventilkonstruktion ist wartungsarm, ein Austausch der Ringe ist erheblich kostengünstiger als der Tausch von Bälgen. Der Aufwand des Einsatzes einer eigens dafür installierten Vakuumpumpe 602 fällt für alle Ventile nur einmal an. Mit ihr kann eine beliebige Menge an solchermaßen gestalteten Ventilen evakuiert werden. Die Überwachung des gemeinsamen Zwischenvakuums durch eine geeignete, in eine Sicherheitskette integrierten Vakuumanzeige garantiert auch die Betriebssicherheit der Anlage bezüglich störenden Fremdgaseintrags, während ein sich entwikkeltes Leck in einem ermüdenden Balg zu einer Betriebsstörung führt, ohne als solches erkannt werden zu können.

Wie in Fig. 4c schematisch gezeigt werden solche Ventil mit Vorteil zu Blöcken integriert,. Dabei werden die Rückräume beispielsweise 1014a und 1014b über eine Stichbohrung 1013a verbunden und zusammen über Leitung 1013 evakuiert. Ein solcher Ventilblock wird mit Vorteil im Kopf des Kompressors integriert. Weitere Ventile, wie sie in der Anlage eingesetzt werden, beispielsweise Ventil 624, 627, 629, 210, werden ebenfalls mit Vorteil zu Blöcken zusammengefaßt. So kann das Ventilprinzip ohne ein Vielzahl an Leitungen wie beispielsweise 612 an Bohrung 1013 platz-, pumpweg- und materialsparend realisiert werden.

Fig. 5a zeigt das prinzipielle Schema des Kompresors, der als Kolbenkompressor ausgeführt ist. Auch hier wird das Prinzip des Zwischenvakuums realisiert, wie schon bei der vorstehenden Ventilkonstruktion besprochen. Der Kolben 411 benutzt Dichtringe in Nut 412 und 413 am Kopf und am Ende, die einen Zwischenraum 414 einschließen. Aus dem Rückraum 418 in den Zwischenraum 414 einleckende Fremdgase oder -dämpfe werden über Ventil 626 und Pumpe 602 abgepumpt, so dass der Transport von solchen Restgas- oder Dampfmolekülen über Dichtring in Nut 412 in den Kompressionsraum 415 nicht signifikant ist.

Mit weiterem Vorteil wird das Zwischenvolumen 414 über eine Bohrung im Zylinder und Ventil 626 abgepumpt und nicht intern über eine mögliche Bohrung im Kolben 411 mit nachfolgender Vakuumleitung (beispielsweise einem flexiblen Schlauch). Die hier vorgeschlagene Konstruktion bedingt zwar eine Kolbenlänge entsprechend des Hubs des Kolbens, mit Vorteil überlappen aber die Fahrwege der Dichtringe in Nut 412 und 413 nicht. Damit ist auch klar, dass der Abstand der Kolbenringe größer als der Kolbenhub ist.

Dieses Konstruktionsmerkmal stellt sicher, dass Oberflächen, die im Lauf der Bewegung in Kontakt mit z.B. Außenluft geraten (beispielsweise in Rückraum 418), durch den Zwischenvakuumbereich strikt vom hochreinen Innenraum getrennt bleiben. Auf diese Weise wird der besagte Gaseintrag in Form eines Adsorptions-Desorptionszyklus verhindert. Fremdgasatome, - moleküle oder Dämpfe im Rückraum 418 können in vorgeschobener Kolbenstellung (vgl. Fig. 5b) an der Wand adsorbieren und nach Zurückziehen des Kolbens von der Wand desorbieren. Ist beim Zurückziehen der Dichtring in Nut 413 über die Position der adsorbierten Verunreinigung gefahren, so erfolgt die Desorption im Zwischenvolumen und nicht im eigentlichen Kompressionsvolumen 415. Vor einer weiteren Absorption bei vorgefahrenem Kolben und Desorption bei rückgefahrenem Kolben und einem damit verbunden Transport in den Kompressionsraum besteht die Chance, die Verunreinigung über Ventil 625 abzupumpen.

Die Dichtung des Kolbens sind vorzugsweise als Doppellippendichtringe ausgeführt sind. Sie dichten den Kompresionsraum 415 gegen das Zwischenvolumen 414 und das Rückraumvolumen 418 ab. Mit Vorteil ist Pumpe 602 als ölfreie Turbomolekularpumpe ausgeführt.

Dieses Prinzip des Abfangens von Fremdgaseinträgen, bei bewegten Vakuumdurchführungen durch Zwischenvakua wird im folgenden als "fraktioniertes Pumpen" bezeichnet. Es charakterisiert den hier vorgestellten Kompressor als einen "Reinstkompressor". Gleiches gilt für die Ventile und Dichtungen 1022 und 1023 äquivalent.

Mit Vorteil werden die von dem Antriebsystem in den Rückraum 418 abgegebenen Gase und Dämpfe bereits über Ventil 627 und Pumpe 603 abgepumpt und haben kaum eine Chance, über weitere Absorption-Desorptionsschritte oder Diffusion durch die Dichtringe in den hochreinen Innenraum vorzudringen. Die bepumpten Rückraum- 418 und Zwischenvolumina 414 erfüllen daher beide das oben definierte Prinzip des fraktionierten Pumpens und bilden daher bzgl. der Dichtstrecke über Dichtringe in Nut 413 und 412 eine doppelte fraktionierte Pumpstrecke. Mit Vorteil ist auch Pumpe 603 als ölfreie Turbomolekularpumpe ausgeführt.

Tatsächlich werden im Ruhezustand des Kompressors in Rückraumvolumen 418 10$^{-3}$ mb und im Zwischenvolumen 414 5,3$^{*}$10$^{-5}$ mb Druck erreicht. Im Betrieb, d.h. beim Fahren des Kolbens 411 stellen sich in Rückraumvolumen 418 etwa 10$^{-2}$ mb und im Zwischenvolumen 1,1$^{*}$10$^{-3}$ mb ein. Selbst bei diesem Zwischenvolumendruck von 1,1$^{*}$10$^{-3}$ mb ist die Diffusion von Restgas- oder -dampfmolekülen aus Zwischenvolumen 414 in das Kompressionsvolumen 415 über Dichtring in Nut 412 vernachlässigbar gegenüber der typischen Ausgasrate von Dichtringen selbst. Die Gasverunreinigung im Kompressionsraum 415 wird durch diese Anordnung auf ein Mindestmaß (Ausgasraten aus dem Kolbendichtring in Nut 412 und Kompressormaterial im Raum 415 selbst ) reduziert ohne eine aufwendige Balgkonstruktion zu verwenden.

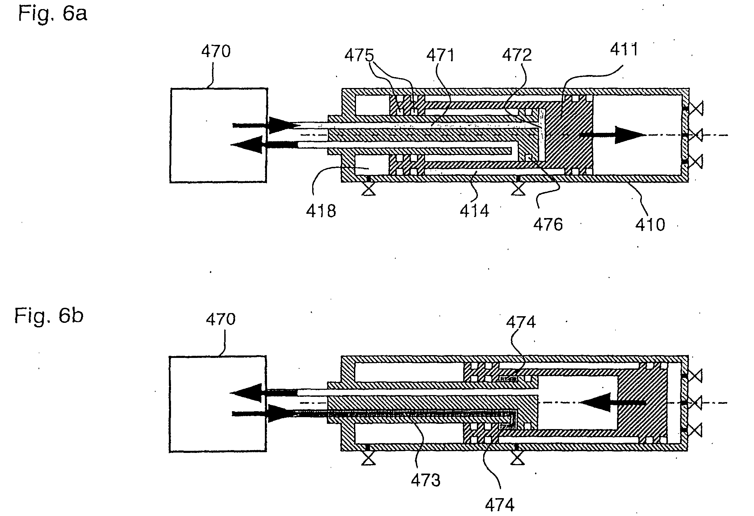

Fig. 6a und 6b illustrieren das Kompressorschema, wobei Fig. 6a den Schnitt durch den Kompressor mit Kolben 411 in Ruhestellung und maximalem Kompressionsvolumen 415 zeigt. In Fig. 6b ist der Kolben 411 vorgefahren, entsprechend höchster Kompression, wobei er den Kompressionsraum vollständig ausfüllt. In einer bevorzugten, einfach herzustellenden Bauform ist der Kompressor - ausgenommen die Anordnung der Ventile im Zylinderkopf (beispielsweise Ventile 420, 421, 422 in Fig. 3 und Ventile 626, 627 in Fig. 5a) mit zugehörigen Bohrungen - im Zylinder rotationssymetrisch um die Symetrieachse 419 ausgeführt.

Der Kolbenkompressors und sein Antrieb dürfen das homogene Magnetfeld nicht verzerren. Für platzsparende Apparaturen muß daher der Antrieb aus nichtmagnetischen Materialien dicht am eigentlichen Kompressor aufgebaut sein. Mit Vorteil wurde für die beispielhafte Ausführung ein integrierter, hydraulischer Antrieb mit zwei Druckkammern konstruiert, wie in Fig. 6a und Fig. 6b gezeichnet.

Der funktionsbedingt lange Kolben - er muß länger als sein Hub sein - führt nun im allgemeinen zu einer erheblichen Verlängerung des gesamten Kompressors. Das ist besonders bei großen Hubwegen, wie sie in der hier besprochenen Anwendung bevorzugt werden, aufwendig. Dieser Nachteil kann durch den nachfolgend beschriebenen speziellen Hydraulikantrieb des Kompressors durch Integration der Druckkammern in den Innenraum des Kolbens selbst überwunden werden.

Zunächst steht Kolben 411 in rückwärtiger Position, wie in Fig. 6a gezeigt. Für die Kompression wird Öl aus dem Aggregat 470 in Leitung 471 in die erste Kammer 472 eingepresst. Daraufhin bewegt sich Kolben 471 in Richtung Zylinderkopf des Kompressors 410. Dabei wird Ol aus der zweiten Kammer 474 über Leitung 473 (Fig. 6b) in das Aggregat zurückgedrückt. Ist der Kompresssionsvorgang abgeschlossen (Fig. 6b), so wird von dem Aggregat 470 Öl in Leitung 473 und zweite Kammer 474 eingepresst und damit Kolben 411 wieder in rückwärtige Position gefahren. Nuten 475 enthalten Hydraulilkdichtringe, die den Ölraum 474 besonders dicht gegenüber dem Rückraum 418 abschließen. Nut 476 enthält einen Hydraulikdichtring, der die erste Kammer 472 gegen die zweite Kammer 474 abdichtet.

Um bei gegebenem Kolbendurchmesser den gewünschten Enddruck im Kompressionsvolumen aufbauen zu können muß der Antrieb genügend Kraft aufbringen. In der beispielhaften Ausführung wird eine Kraft von mehr als 15400 N benötigt, um bei einem Kolbendurchmesser von 14 cm einen Enddruck von 10 bar zu erreichen. Mit Vorteil werden hydraulische Antriebe eingesetzt, da bei Öl-Drücken von typischerweise bis zu 250 bar nur eine kleine Stirnfläche für das Volumen 472 notwendig ist, um diese Kraft zu realisieren (15,9 cm$^2$ in der beispielhaften Ausführung). Beim Zurückfahren müssen nur Reibungskräfte der Dichtringe überwunden werden, wofür eine kleinere Stirnfläche von 5,7 cm$^2$ in der beispielhaften Ausführung ausreicht. Daher kann mit Vorteil der Antrieb innerhalb des Kolbens untergebracht werden, womit bei vorgegebener maximaler, äußerer Kompressorbaulänge der Hub des Kolbens maximiert werden kann.

Gleichzeitig lassen sich platzsparend, da parallel neben dem Kolben, zwei fraktionierte Pumpstrecken 418 und 414 realisieren, weshalb auf Bälge als Hochvakuumabdichtungen verzichtet werden kann.

Mit Vorteil enthält diese Anordnung nur ein einziges bewegtes Teil, den Kolben 411 selbst. Dieser wird über Ringe in den Nuten 416, 418 geführt und über Ringe in den Nuten 412, 413, 475, 476 gedichtet. Die Ausfallsmöglichkeiten aufgrund von Verbindungselementen zu externen Antrieben ist damit ausgeschaltet.

Weiterhin ermöglicht ein hydraulischer Antrieb eine beliebige Plazierung des Kolbens 411 innerhalb des Kompressors 410. Damit können reduzierte Hubvolumina $V_{red}$ eingestellt werden, um beispielsweise nach Druckausgleich mit dem Sammelzellenvolumen 440 einen bestimmten Teil des Gases in eine Speicherzelle 501 zu komprimieren. Somit kann Zeit gewonnen werden, indem ein große Sammelzelle bis zu geeignet gewähltem Druck aufgefüllt wird, und dann nacheinander mehrere kleine Speicherzellen mit angepaßter Gasmenge gefüllt werden, wobei die Gasmenge je nach Sammelzellendruck durch die Wahl von $V_{red}$ über die hintere Kolbenstellung eingestellt wird. Da bei der unten beschriebenen Betriebsart zum Füllen mehrerer, parallelgeschalteter Speicherzellen gleichzeitig Zeit für das Abpumpen gespart wird, führt diese Maßnahme zu einer Steigerung der mittleren Produktionsrate.

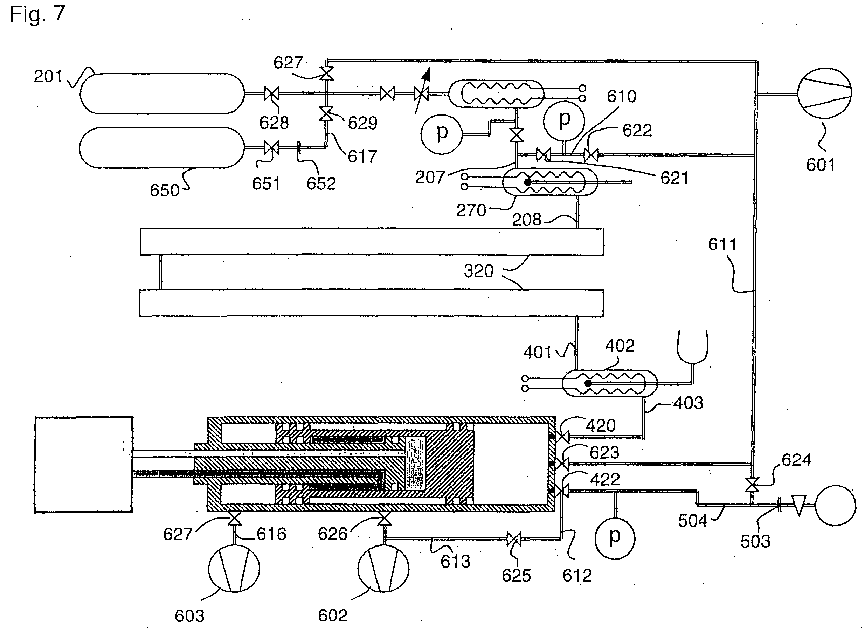

Fig. 7 zeigt das Schema zur Erzeugung guter Vakua, die Vorraussetzung für hohe Gasreinheiten sind. Mit Vorteil werden drei Turbopumpen verwendet. Pumpe 601 dient zur Vorbereitung der Apparatur. Dies sind Evakuierung vor dem Einfüllen von $^3$He und dem Füllen einer Speicherzelle sowie das Präparieren des optischen Pumpvolumens.

Zum Einfüllen von $^3$He wird eine Flasche 650 mit Ventil 651 an Flansch 652 verbunden. Eingeschlossene Luft in Volumen 617 wird nun über Ventil 629 und Ventil 627 mit der Pumpe 601 über das Leitungssystem 611 abgepumpt. Anschließend kann über Ventil 651, Leitung 617, Ventil 629 und schließlich Ventil 628 frisches Gas aus der Flasche 650 in das Reservoir 201 eingefüllt werden.

Ebenso kann nach dem Verbinden der Speicherzelle an Flansch 503 die Luft in der Leitung 504 bei geschlossenem Ventil 422 und geöffnetem Ventil 622 über das Leitungssystem 611 abgepumpt werden.

Vor der Inbetriebnahme der Apparatur muß das optische Pumpvolumen speziell präpariert werden. Dafür wird das Volumen 320 über Leitung 208, Reinigungseinheit 270, Leitung 207, Ventil 621, Leitung 610, Ventil 622 und Leitungssystem 611 von Pumpe 601 evakuiert. Für das effektive Reinigen von Volumen 320 wird dieses gleichzeitig auf über 200° Celsius geheizt.

Während die Volumina 617 und 504 möglichst klein gebaut sind (etwa 70 und 1 cm$^3$) wurde das Leitungssystem 611 mit einem großen Querschnitt versehen. Bei Leitwerten von 3,2 l/s (Leitung mit Länge 2,2m und Durchmesser d=3,9cm), 0,72 l/s respektive und 0,2 l/s lassen sich bei Pumpansaugdrücken von <3$^*$10$^{-8}$ mb und einer Saugleistung von 68 l/s (für N$_2$) der Pumpe in den Volumina 617, 504 und 320 Drücke von unter 1$^*$10$^{-5}$, 1$^*$10$^{-5}$, 3$^*$10$^{-6}$ mb erreichen.

Die besprochenen Leitungssysteme sind zwecks Minimierung ihrer Länge ebenfalls in dem homogenen Magnetfeld (Fig. 3 100) untergebracht. Dieses darf nicht verzerrt werden, um in dem geforderten Bereich die für die Polarisationsspeicherung notwendige Homogenität mit relativen, transversalen Gradienten von beispielsweise

$$\partial Br/\partial r / B_0 = G_r < 5 * 10^{-4} / cm \qquad (20)$$

aufzuweisen.

Um solche Gradienten in einem zylindrischen Volumen von 2,8 m Länge und 48 cm Druchmesser zu unterschreiten wurde mit Vorteil ein System von 7 Magnetfeldspulen mit gleichen Durchmesser von 160 cm errichtet. Die jeweils äußeren 3 Spulen sind symmetrisch zur mittleren axial ausgerichtet und werden durch Vermeidung relativer Feldschwankungen zueinander elektrisch in Serie betrieben. Notwendige individuelle Ampere-Windungszahlen wurden über die Zahl der Windungen eingestellt.

Alle Vakuumleitungen sind in dem Magnetfeld 100 untergebracht und, um die Feldhomogenität zu erhalten, aus nichteisenhaltigen Materialien gefertigt. Allgemein sind außer für die polarisationführenden Leitungen 208, 323, 401 und 431 Aluminiumrohre eingesetzt. Als Dichtmaterialien werden Alu-Schneidkantringe eingesetzt. Alu-Schneidkantringe haben gegenüber normalen Dichtungen aus Polymeren (Kunststoff, Gummi wie Viton, NBR etc.) den Vorteil, nicht mehr als das Rohrmaterial selbst auszugasen, so dass Vakua von unter 10$^{-6}$ mb leicht erreicht werden. Im Niederdruckbereich 320 werden Glas-Metall Übergänge ebenfalls metallisch mit weichem Indium gedichtet. Auf der Hochdruckseite (Ausgang Ventil 420 und Ventil 422) werden Viton Dichtringe eingesetzt. Viton eignet sich von allen Polymeren am besten aufgrund seiner dichtesten Matrix und den damitverbunden relativ geringen Ausgasraten an Fremdgasen wie z.B. N$_2$, O$_2$, CO$_2$, oder Kohlenwasserstoffen.

In einem solchermaßen gefertigten und gedichteten System lassen sich vor dem Pumpstutzen Vakua von < 5$^*$10$^{-9}$ mb erreichen. Dies ist gegenüber dem Stand der Technik, wo alle Vakuumsysteme aus Glas gefertigt waren und nur Glashähne mit kleinem Querschnitt (max. 5 mm Durchmesser) und folglich geringem Leitwert eingesetzt werden konnten, ein entscheidender Vorteil.

Fig. 7 zeigt auch die Ausweitung des Prinzip des "Fraktionierten Pumpens" auf den $^3$He-Plasmaraum. Mit Vorteil werden auch hier fraktionierte Pumpstufen eingesetzt. Nachdem das Volumen 320 unter Vakuum ausgeheizt und mit Gasentladungen und mehrfachem Austausch des Helium-Spülgases letzte Verunreinigungen von der Wand gelöst wurden, wird Helium-3 Gas aus Reservoir 201 über Leitungen 202, 212, 203 sowie Ventile 210 und 213 in die Reinigungseinheit 250 eingelassen. Nach ausreichender Verweildauer wird Ventil 214 geöffnet und das Gas in das Volumen 320 über Leitung 206, 207, einer weiteren Gasreinigungseinheit 270 sowie Leitung 208 eingelassen. Am Ausgang von Volumen 320 befindet sich mit Vorteil ebenfalls eine, mit 270 identische Gasreinigungseinheit 402.

Gasreinigungseinheit 270 und 402 haben nun die Aufgabe, beispielsweise aus Ventil 213 oder Ventil 420 eindringende Verunreinigungen (Fremdgase) zurückzuhalten und das Gas nachzureinigen. Während Ventil 214 in Ultrahochvakuum-Ausführung gefertigt ist, und daher prkatisch keine Verunreinigungen abgibt, enthält Ventil 420 Vitondichtringe und der Kompressor ein Schmiermittel, die Fremdgase abgeben. Desweiteren dienen die Gasreinigungseinheiten 270 und 402 über die kurzen Verbindungsleitungen 208 und 401 (Leitwerte 4,3 l/s bzw. 7,6 l/s) als Getterpumpen im Volumen 320 und können innerhalb des Volumens 320 abgegebene Fremdgase absorbieren.

Die Gasreinigungseinheiten 270 und 402 enthalten sowohl einen Titan-Sublimationsgetter wie auch eine flüssig Stickstoff Kühlfalle.

Um das fraktionierte Pumpen und die Apparaturpräparation vakuumtechnisch zu trennen, werden mit Vorteil für die beschriebenen Evakuationaufgaben unterschiedliche Pumpen 601, 602 und 603 eingesetzt. Damit wird die Diffusion von Fremdgasen oder -dämpfen zwischen den jeweiligen Vakuumbereichen der Apparatur vermieden und höchste Gasreinheiten in den Volumen für Optische Polarisierung von $^3$He und dessen Speicherung erreicht. Zu unterscheiden sind die Bereiche, die im regulären Betrieb der Anlage $^3$He enthalten, Rückraumbereiche der Ventile und Öldämpfen ausgesetzte Volumen im Bereich des Kompressorantriebs. Zweckmäßigerweise wird der vierte Bereich, das Zwischenvolumen 414 des Kompressors zusammen mit den Rückräumen der Ventile von einer Pumpe 602 abgepumpt. Diese Anordnung erlaubt es zusätzlich, Rück- und Zwischenräume der Ventile und des Kompressors ständig zu bepumpen, unabhängig vom Bedarf der Evakuierung relevanter $^3$He Arbeitsvolumen. Weiterhin werden das Leitungssystem für die Evakuation des $^3$He Arbeitsvolumen niemals mit Fremdgasen aus den Rück- und Zwischenräumen der Ventile und des Kompressors verunreinigt. Dies steigert die Zuverlässigkeit, mit der die erforderliche Reinheit des $^3$He im $^3$He-Arbeitsvolumen erreicht und auf längere Sicht aufrecht erhalten werden kann, was Vorraussetzung für lange, störungsfreie Betriebsphasen und damit für hohe mittlere Produktionraten ist.

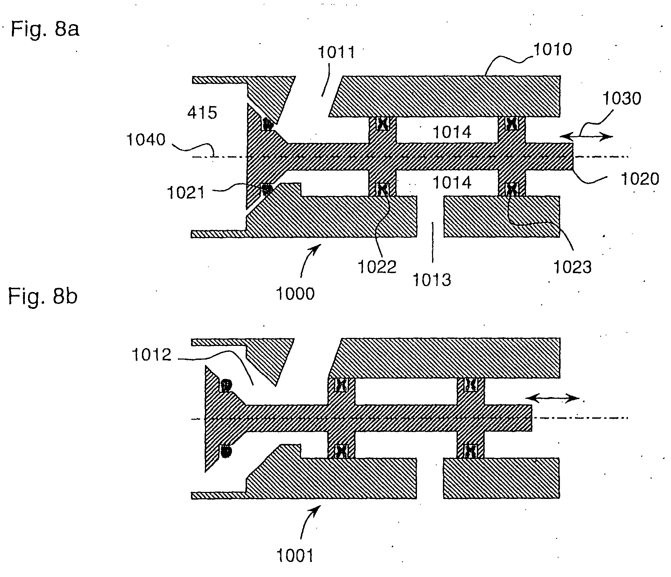

Fig. 8a,b zeigt ein querschnittsoptimierte Einlaß- oder Evakuationsventil für den Kompressor gemäß der Erfindung. Bei der Auslegung des Kompressors für die Kompression von kernspinpolarisiertem $^3$He-Gas muß auf die Niederdruckeigenschaften einerseits und auf die Hochdruckeigenschaften andererseits geachtet werden. Niederdruckeigenschaften betreffen gute Evakuierbarkeit, d.h. große Ventilquerschnitte zur Pumpe 601 und zum Volumen für Optisches Pumpen 320. Hochdruckeigenschaften bedeuten u.a. kleine Toträume in Ventilen 421 zur Sammelzelle und Ventil 422 zur Speicherzelle. Weiterhin muß das Totvolumen zwischen Ventilen im Kompressorkopf und eigentlichem Kompressionsraum 415 minimiert werden.

Die beispielhafte Ausführung umfaßt diese Eigenschaften. Ventil 420 und 623 sind von der Art gemäß Fig. 8a,b. Ein um Achse 1040 rotationssymmetrischer Ventilstößel 1020 öffnet in den Kompresionsraum 415. Dabei kann Gas über eine Leitung 1011 und großer Öffnung 1012 über einen großen Querschnitt in den Kompressionsraum einströmen bzw. der Kompressionsraum gut evakuiert werden. Im geschlossenen Zustand füllt der Kopf des Stößels den Durchlaß 1012 vollständig aus, so dass seine Oberfläche plan mit der Innenseite des Kompressionsraumes abschließt (Fig. 8a). So können großer Querschnitt 1012 und geringes Totvolumen erfolgreich gepaart werden.

Die restlichen Ventile sind in der Art gemäß Fig. 4 aufgebaut. Kleine Durchmesser der Bohrungen 1011, 1012 erlauben kleine Toträume sowohl zum Kompressionsvolumen als auch für Leitung 413 und 504. Der Ventilstößel 1020 füllt zusätzlich das Ventilvolumen weitestgehend aus, was zur Minimierung der Totvolumina beiträgt.

In der beispielhaften Ausführung betragen die Querschnitten 300 mm$^2$ für Ventil 420 und 623. Der Querschnitt von Ventil 421 beträgt 20 mm$^2$ und von Ventil 422 sind es 7 mm$^2$.

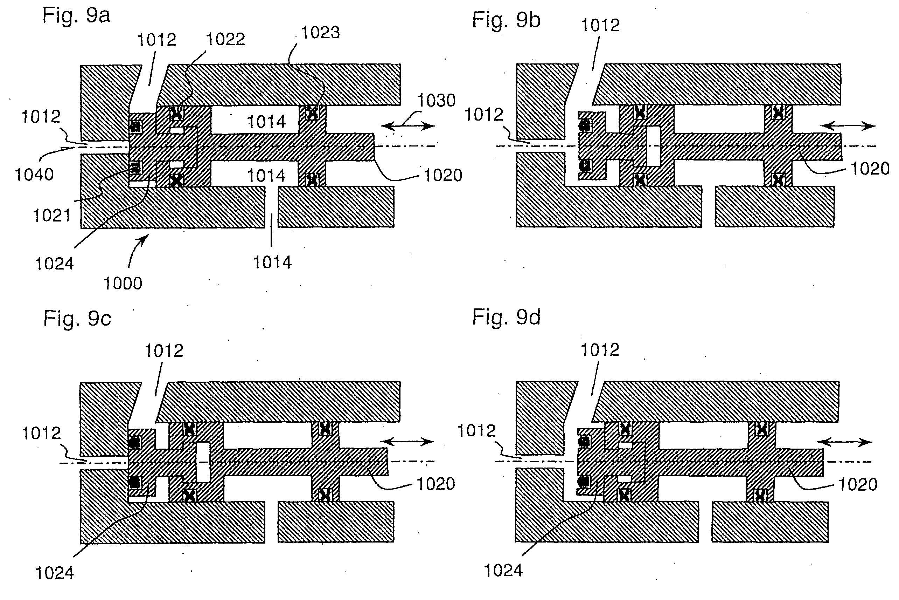

Fig. 9a bis 9d stellt Ventil 421 dar, dass mit Vorteil als automatisch öffnendes aufgebaut ist. Antrieb 1030 kennt dafür 3 Stellungen. Antrieb 1030 bewegt den Stößel 1020 in die garantiert geschlossene Stellung (Fig. 9a). Dabei drückt Stößel 1020 einen Hilfsstößel 1024 gegen die Öffnung 1012. In mittlerer Stellung von Stößel 1020 (Fig. 9c und 9d) kann der Hilfsstößel 1024 sich sowohl in geschlossener Stellung (Fig. 9c) als auch in geöffneter Stellung (Fig. 9d) befinden. Fig. 9b zeigt schließlich Stößel 1020 in der garantiert geöffneten Stellung.

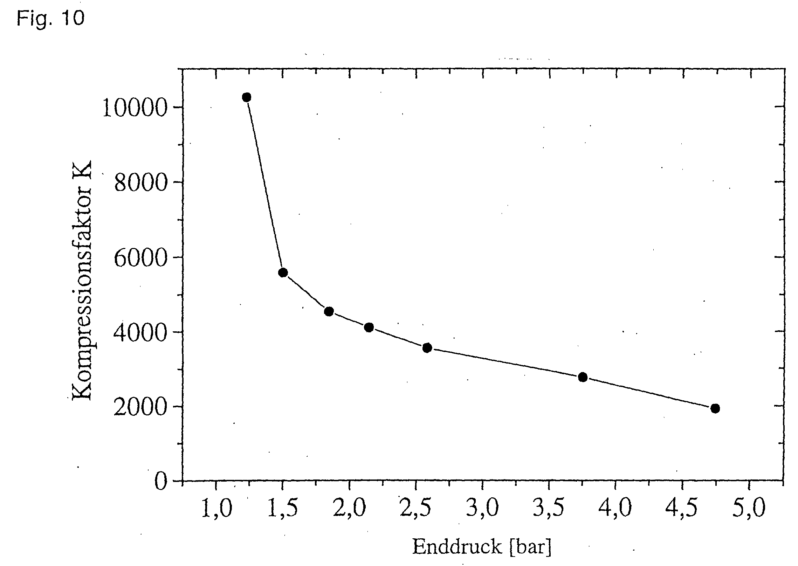

Fig. 10 zeigt beispielhaft die damit maximal erreichbare Kompression $K_0 = V_{hub} / V_{tot}$ (s. Gl. (7)) in Abhängigkeit des Ausgangsdrucks $p_{501}$, dem Druck in der Speicherzelle 501. Bzgl. $K_0$ sind $p_{501}$ in der Speicherzelle 501 und $p_{440}$ in der Sammelzelle 440 äquivalent. Der Abfall von $K_0 = 10000$ bei $p_{501} = 1,5$ bar auf $K_0 = 2000$ bewi $p_{501} = 5$ bar ist auf das elastische Zurückweichen des Dichtringes in Nut 412 zurückzuführen, wodurch sich $V_{tot}$ nach Gl. (12) proportional zu $\alpha \propto p_{501}$ vergrößert. Das erklärt nach Gl. (7) den hyperbolischen Abfall von $K_0$ in Fig. 10. Aus dem Diagramm ersieht man, dass in der ersten Kompression in die Sammelzelle 440 hinein mit der Verdichtung von $\approx 1$ mbar auf 200 mbar ein $K_{eff} = 200$ (entsprechend Gl. (6)) in der Regel nicht überschritten wird und damit $K_0 > 10000$ beträgt..

Somit wird mindestens ein Polarisationstransferfaktor $\eta_1 = 1 - 200 / 10000 = 98$ % (Gl. (9) udn (11)) rreicht (d.h. bei vollständiger Relaxation im $V_{tot}$). In der Tat wurde in der beispielhaften Ausführung innerhalb der Meßfehler von $\pm 2$ % kein Polarisationsverlust beim Transfer beobachtet. Bei einer nachfolgenden Verdichtung von 200 mbar auf 3 bar in die Speicherzelle 501 ergibt sich aus Fig. 10 ein $K_0 = 3000$ und unter Berücksichtigung der Entspannung des Gases von der Sammelzelle 440 mit Volumene $V_{440 = 3,5 \text{ liter}}$ in den Hubraum mit Volumen $V_{hub} = 15,4$ liter ein

$$K_{eff2} = 3 \text{ bar} / ( 0,2 \text{ bar} \cdot V_{440} / (V_{440} + V_{hub})) \approx 80 \, .$$

Daraus berechnet sich unter Annahme vollständiger Relaxation in $V_{tot}$ ein $\in_2 = K_{eff2} / K_o$ von 2,7 % und ein $\eta_2$-Wert (Gl. (11) )von 1- $\in_2 = 97,3$ %. Tatsächlich wurde auch im zweiten Kompressionschritt innerhalb des Meßfehlers von $\pm$ 2 % kein Transferverlust der Polarisation beobachtet. Insgesamt bleiben die Polarisationsverluste bei der Kompression aufgrund des großen $K_0$-Werts auf höchstens 1 - $\eta_1 \cdot \eta_2 = 4,7$ % beschränkt, bei der offensichtlich nur teilweisen Relaxation des in $V_{tot}$ gefangenen Gases sogar auf weniger. Dahingegen galt bei der Vorgänger-Version mit K = 800 und $K_{eff} = 200$ schon $\eta_1 \leq 0,75$. Die beispielhafte Ausführung komprimiert also das polarisierte $^3$He sehr viel effektiver.

Fig. 10 zeigt andererseits die Notwendigkeit einer schrittweisen Verdichtung über die hier mit Vorteil eingeführte Sammelzelle 440 als Zwischenspeicher. Bei einer Verdichtung in einem Schritt von beispielsweise 1 mbar auf 2 bar würden ca. 50 % des komprimierten Gases im Totvolumen verbeleiben und es müßte mit Polarisationstransferverlusten bis zu 50 % gerechnet werden.

In einer zweistufigen Verdichtung dagegen kann, ausgehend von einem Druck von ca. 1 mbar, ein Enddruck von bis zu 10 bar angestrebt werden, ohne größere Polarisationstrasnferverluste befürchten zu müssen. Eine Verdichtung auf mehr als 10 bar ist nicht sinnvoll, da nach der Veröffentlichung

N.R. Newbury, A.S. Barton, G.D. Cates, W. Happer, H. Middleton; "Gaseous $^3$He-$^3$He magnetic dipolar spin relaxation"; Phys. Rev. A 48:6 (1993) 4411 - 4420

bei hohen Drücken eine gegenseitige, sogenannte dipolare Relaxation der $^3$He-Atome relevant wird mit einer bei konstanter Temperatur T = 273 K dem Druck proportionalen Rate

$$\Gamma_{1,dip(23°C)} = (p \, / \, bar) \, / \, (744 \, h) \, . \tag{21}$$

Sie verkürzt bei 10 bar die Relaxationszeit auf höchstens 75 h unabhängig von anderen, zusätzlichen Relaxationsquellen.

Mit Vorteil wird in der hier vorgestellten Methode und beispielhaften Ausführung eine Sammelzelle 440 verwendet, die eine ausreichend lange Relaxationszeit aufweist. In der beispielhaften Ausführung handelt es sich um einen 3,5 liter Kolben aus Duranglas mit $T_1 \approx 19$ h. Bei einer typischen Sammelzeit von ca. 20 min resultiert hieraus ein zusätzlicher relativer Polarisationsverlust von 2,4 %. Die oben besprochenen Verluste im Totvolumen hinzugerechnet, ergibt sich ein Gesamtpolarisationstransferfaktor von mindestens $\eta = 93$ %, bei offensichtlich nur teilweiser Relaxation im Totvolumen sogar ein noch höherer Wert.

Es sei angemerkt, dass in der beispielhaften Ausführung der Polarisationsverlust während des Aufenthalts des Gases im vollen Hubvolumen des Kompressors vernachlässigbar ist. Die Messung ergab eine Relaxationszeit von $T_1 = 13$ h, woraus sich nach Gl. (4) ein sehr kleiner oberflächenspezifischer Relaxationskoeffizient von $\gamma = 0,26$ cm / h errechnet im Vergleich zu dem ungünstigeren Wert von 4 cm/h in einer Kompressionsstufe gemäß dem Stand der Technik, wie in

J. Becker, W. Heil, B. Krug, M. Leduc, M. Meyerhoff, P.J. Nacher, E.W. Otten, Th. Prokscha, L.D. Schearer, R.Surkau; "Study of mechanical compression of spin-polarized $^3$He gas"; Nuc. Instr. & Meth. A 346 (1994) 45 - 51 beschrieben.

Mit Vorteil wurde in der Vorrichtung gemäß der Erfindung die abschließende Innenbearbeitung des Kompressionsraumes mit nichtmagnetischen Werkzeugen vorgenommen. Normale Drehstähle hinterlassen Abbrandspuren im Titan, die nach Ultraschallbadreinigung als Eisenoxydflecken sichtbar werden. Sollte nicht alles Eisen oxydiert sein, so sind damit ferromagnetische Bezirke in der Oberfläche eingebracht, die eine starke Relaxation verursachen Im übrigen ist der Kompressorraum mit einem beständigen, sehr niedrig ausgasendem Spezialfett geschmiert worden, das gute Relaxationseigenschaften besitzt.

Der Einsatz nur einer Kolbenpumpe gemäß der Erfindung, die Zwischenspeicher und Speicher bedient, reduziert den Aufwand an Beschaffungs- und Wartungskosten mit Vorteil. Die statistisch gesehen geringere Ausfallswahrscheinlichkeit aufgrund der verminderten Anzahl mechanischer Teile gewährt eine höhere Zuverlässigkeit und damit längere Betriebsphasen. Dies wirkt sich letztendlich auf eine höhere Produktionsrate zwischen den notwendigen Serviceintervallen für die Apparatur aus.

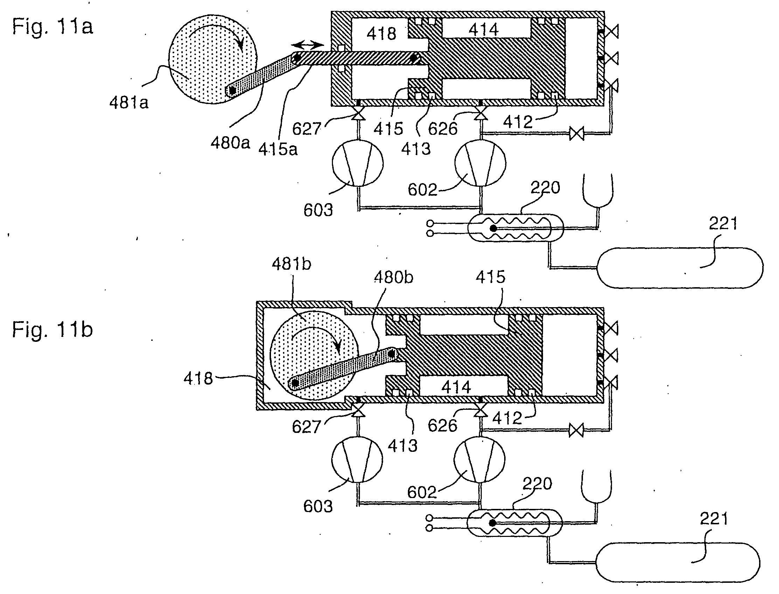

Fig. 11a und 11b zeigt eine schnelllaufende Variante des hier vorgestellten Prinzips eines Reinstgaskompressors mit kleinerem Hubvolumen und kürzerem Hubweg bei gleicher Förderleistung. Dabei wird das Prinzip des fraktionierten Pumpens über Zwischenraumvolumina, beispielsweise nach Fig. 5a, beibehalten. Der Antrieb des Kolbens 415 kann dann mit Vorteil auf traditionelle Weise über Kurbelwelle 481a,b und Pleuel 480a,b in zwei Versionen mit außen- (Fig. 11 a) und innenliegendem (Fig. 11b) Antrieb erfolgen.

Die durch den Dichtring 412, 413 aus dem Arbeitsraum in das Zwischenvakuum 418 einleckende Edelgasmenge wird über den Ventil 627 die Hochvakuumpumpe 603 abgepumpt und in einem Reservoir 221 gespeichert. Nach Abtrennung der Fremdgase, beispielsweise kryotechnisch, wie in DE 199 27 773.7 beschrieben, steht das gereinigte Edelgas wieder zur Verfügung. Aufgrund der erhöhten Leckraten der Dichtringe bei schnellaufendem Kolben wird dem Zwischenvolumen 418 mit Vorteil ein weiterer Zwischenraum 414 vorgelagert, der über den Saugstutzen 626 evakuiert wird.

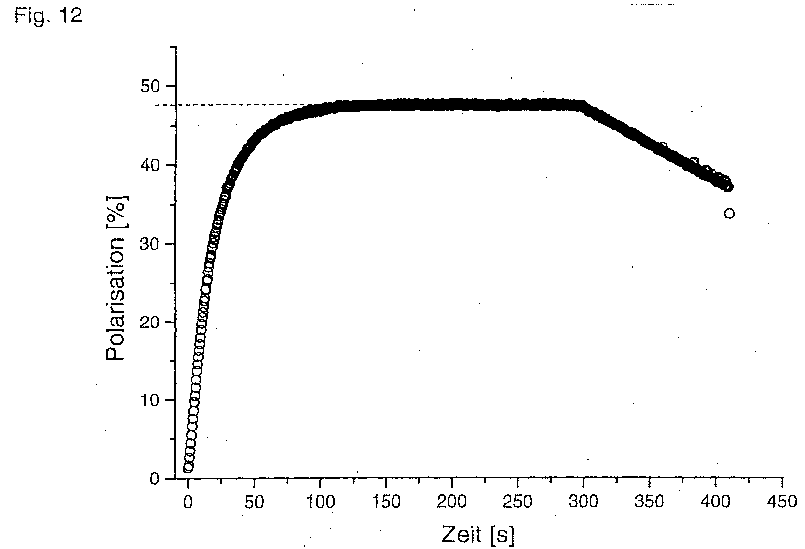

Fig. 12 zeigt den zeitlichen Verlauf der Polarisationsentwicklung in einer der Zellen aus Volumen 320, wenn zunächst unpolarisiertes Helium-3 Gas eingefüllt wurde und dann das resonante, zirkular polarisierte 1083 nm Laserlicht zugeschaltet wurde. Um lange Absorptionstrecken zu erhalten und möglichst viel Laserlicht zu absorbieren. In der beispielhaften Ausführung wurde das Volumen auf 5 Zellen mit je 2,4 m verteilt. Die resonante Laserleistung betrug etwa 5 Watt, die Aufpolarisations in dem langgestreckte Gasvolumen von 5,5 liter benötigt bei typischen Drücken etwa 1 Minute, um 93 % der Gleichgewichtspolarisation zu erreichen. Der zunächst sehr steile Anstieg der Polarisation wird mit steigender Polarisation zunehmend flacher und läuft schließlich in einen Gleichgewichtswert (strichlinierte Linie), der aufgrund konkurierender, bereits oben besprochenen Relaxationsprozesse unterhalb von 100 % liegt.

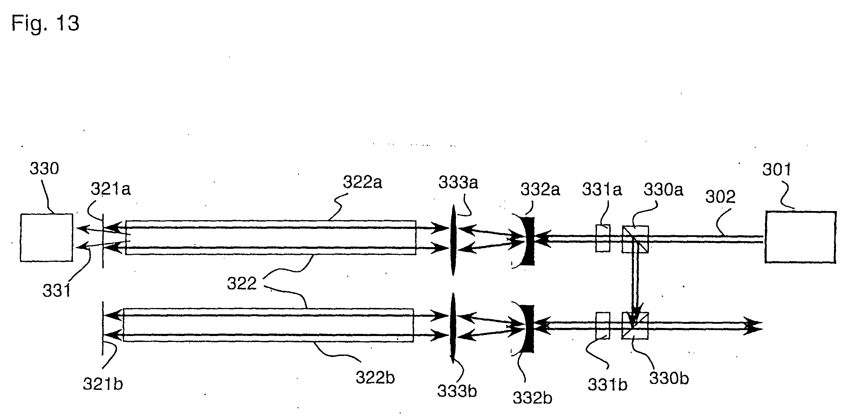

Fig. 13 zeigt das Aufweiten und Parallelisieren des Laserlichts 302, welches vor Eintritt in die Volumen zum Optischen Pumpen beispielsweise 322a, 322b vollständig zirkular polarisiert sein muß. Mit Vorteil wird der Laserstrahl zunächst durch einen Polarisationsstrahlteilerwürfel 330a von hoher Qualität linear polarisiert und anschließend mittels einer passend justierter λ/4-Platte 331a zirkularpolarisiert. Ein nachfolgendes Teleskop 332a, 333a weitet den Strahl auf den geforderten Durchmesser auf und bildet den Zwischenfokus auf den gegenüberliegenden Spiegel 321a ab, dabei das Zellenvolumen möglichst gut ausfüllend, d.h. schwach konvergent. Bei langen Wegstrecken durch Zellen 322a, 322b, wie die in der beispielhaften Ausführung mit als 2$^*$ 240 cm gewählt wurden, ist es vorteilhaft, den Strahl nach Hin und Herspiegelung durch eine Zelle wieder durch Linsen 333a, 332a zu kollimieren, bevor er in eine weitere Zelle eingespiegelt wird. Durch eine solche Linsenstraße 333a, 332a wird trotz an die Zelldurchmesser angepaßter Strahldurchmesser eine Berührung des Lichts mit den Zellwänden 322a bzw. 322b vermieden. Mit Vorteil wird dabei eine ebenfalls möglichst weite Strahltaille an den Ort des rückwärtigen Spiegels 321a bzw. 321b gelegt. Ansonsten würde Reflexion des Lichts an dem Zellenrohr den Zirkularpolarisationsgrad vermindern und zusätzlich relaxierend wirken. Vorteilhafterweise besteht das Teleskop aus einer Konkavlinse 332a und einer Konvexlinse 333a, was die Baulänge des Teleskops verkürzt und die Divergenz auf relativ kleine Winkel beschränkt. Auf letztere ist zwecks Erhaltung der Zirkularpolarisation trotz Entspiegelung der Linsen zu achten.

Vorteilhafterweise wird der Laserstrahl 302 nacheinander durch alle Zellen (in desem Beispiel 322a und 322b) eines Bereichs 322 justiert, um die angebotene Absorptionslänge wie oben erläutert voll auszunutzten. So wird möglichst viel Licht genutzt und insbesonderen dem Bereich mit der höchsten Polarisation und der damit verbunden geringsten Absorptionswahrscheinlichkeit zu einer relativ höchsten Polarisationsrate verholfen. Nach dem zweiten Durchgang durch das Teleskop 332a, 333a und zurück durch die Polarisationsoptik 331a, 330a ist der Strahl schließlich wieder linearpolarisiert mit kleinem Durchmesser. Aufgrund seiner um 90° gedrehten Linearpolarisation wird er senkrecht zur Einfallsrichtung aus dem Strahlteilerwürfel 330a herausreflektiert. Durch eine gleich aufgebaute Optik, bestehend aus Polarisationsstrahlteilerwürfel 330b, λ/4-Platte 331 b und Teleskop 332b, 333b wird der Strahl in Richtung der nächsten Zelle 323 umgelenkt, wieder zirkular polarisiert und aufgeweitet.

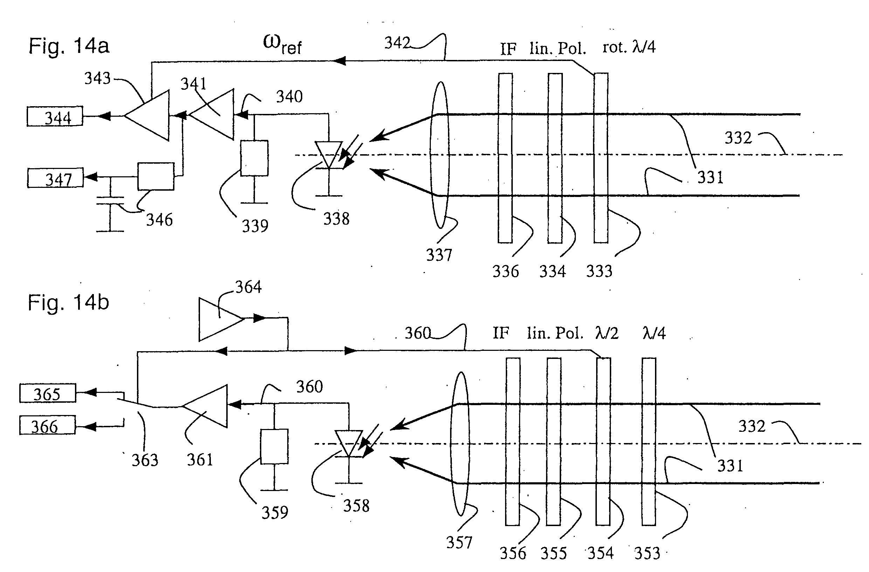

Fig. 14a,b zeigt Meßschemata, mit denen der Kernspinpolarisationsgrad des $^3$He in den OP-Zellen bestimmt wrden kann. Dafür ist der Spiegel 321a als dichroitischer ausgeführt; hoch reflektierend für 1083 nm, hochtransmittierend für 668 nm. Er wirft einerseits das Pumplicht hinter der Zelle 322a zurück, transmittiert aber Fluoreszenzlicht welches entlang der optischen Achse 332 aus den Zellen zum Optischen Pumpen emittiert wird. Sein Zirkulationsgrad läßt sich über Eichfaktoren in eine Kernspinpolarisation des $^3$He-Gases im Pumpvolumen umrechnen.

Der Zirkulationpolarisationsgrad des austretenden Fluoreszenzlichts wird dafür mittels einer mit Frequenz f rotierenden λ/4-Platte 333 in linear polarisiertes Licht umgesetzt, dessen Polarisationsrichtung ebenfalls mit Frequenz f rotiert. Nach Durchgang durch einen statischen Linearpolarisator 334, ein Inteferenzfilter 336 und einen Kollimator 337 wird das Licht in einem Photodetektor 338, beispielsweise einer Photodiode, in ein mit Frequenz 2f amplitudenmoduliertes Stromsignal umgewandelt. Nach Verstärkung im Verstärker 341 wird der Modulationshub von einem Lock-In Verstärker 343 nachverstärkt, phasenempfindlich gleichgerichtet und im Speicher 344 aufgezeichnet. Das aufgrund des unpolarisierten Fluoreszenzlichtanteils vorhandene, von Null verschiedene Mittelwertsignal wird mittels eines Tiefpasses 346 gemessen und in Speicher 347 aufgezeichnet. Der Quozient aus den Werten in Speicher 344 und 347 ist der gesuchte Zirkularpolarisationsgrad und gibt, dividiert durch einen bekannten druckabhängigen Eichfaktor, den Wert der Kernspinpolarisation an.

Fig. 14b beschrieb eine neuen Variante des Verfahrens, die mit Vorteil einen Nachweis ohne mechanisch rotierende Elemente angewendet. Der Aufbau generiert in Abhängigkeit der Fluoreszenzzirkularpolarisation einen maximalen und einen minimalen Spannungswert, deren Differenz, geteilt durch ihre Summe und den Druckeichfaktor wieder die Kernspinpolarisation ergibt. Dazu durchquert das 668 nm Licht zunächst eine statische λ/4-Platte 353, anschießend einen Flüssigkristallelement 354, einen Linearpolarisator 355, ein 668 nm Interferenzfilter 356 und einen Kollimator 357 bevor es in einem Photodetektor 358 nachgewiesen und im Verstärker 361 verstärkt wird. Ein Rechtecksignalgenerator 364 erzeugt ein positives oder negatives Spannungssignal auf Leitung 360, dass das Flüssigkristallelement 354 bei einer der beiden Spannungen veranlaßt, als λ/2-Platte zu wirken, bei der anderen nicht. Dadurch wird die Polarisationsebene periodisch um 90° gedreht und vom Analysator 355 abwechselnd der links- und der rechtspolarisierte Anteil des Fluoreszenzlichtes transmittiert und in Abhängigkeit des Steuerspannungssignals auf Leitung 360 über einen davon abhängigen Schalter einmal in Speicher 365 und das anderemal in den Speicher 366 abgelegt. Aus diesen beiden Werten kann nun das Kerspinpolarisationssignal wie oben angegeben berechnet werden.

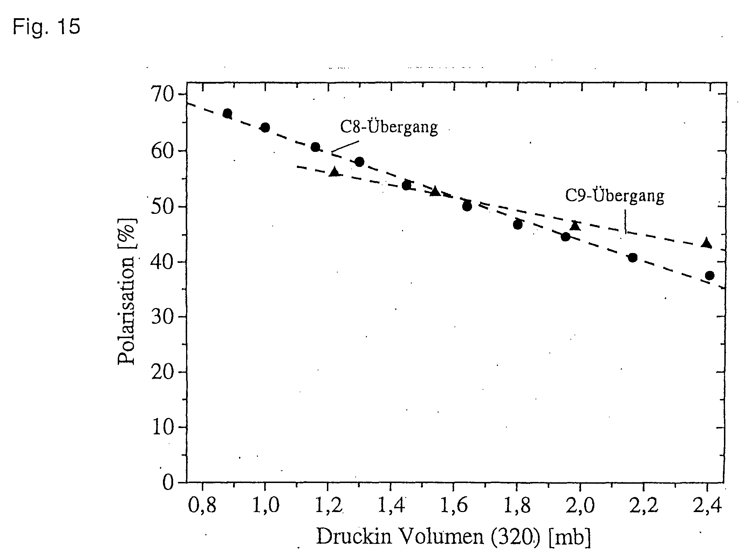

Fig. 15 zeigt die beispielhaft für eine Optische Pump-Konfiguration erreichbare Gleichgewichtspolarisation, wie z.B. in durch die strichlinierte Linie dargestellt, in Abhängigkeit des Gasdruckes. So erreicht man bei minimalen Druck maximale Gleichgewichtspolarisationen. Mit stetiger Zunahme des Drucks fällt diese ab. Weiterhin ist die unterschiedliche Effizient der beiden optischen Übergänge C9 und C9 des $^3$He$^*$-Atoms gezeigt. Bei niedrigeren Drücke können mit dem C8-Übergang höhere Endpolarisationen erreicht werden.

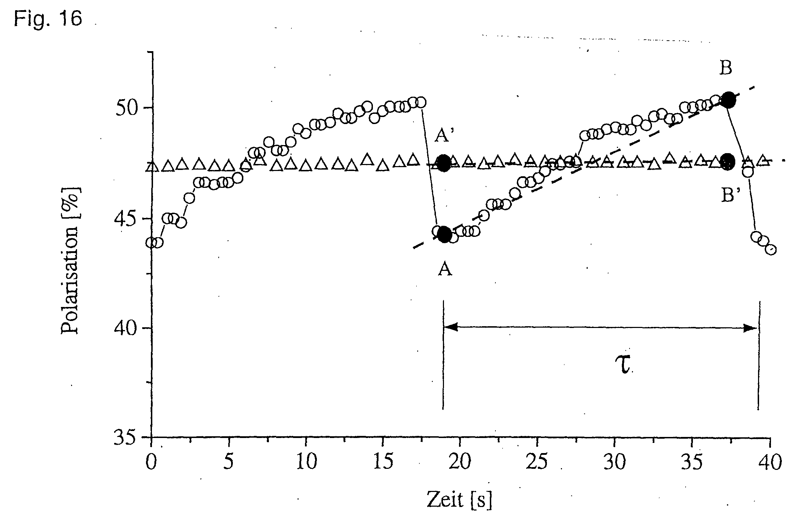

Fig. 16 illustriert beispielhaft, dass die erfinderische Anordnung zum Optischen Pumpen von $^3$He nach Fig.

3 und 13 es nun erlaubt, durch diskontinuiertliche Gaszufuhr höhere Polarisationsraten zu erreichen, als bei kontinuierlicher. Bei letzterem fließt kontinuierlich geringer polarisiertes Gas in die OP-Zellen nach. In der Fig. 3 illustrierten Anordnung erfolgt dagegen sowohl die Gasversorgung des Volumens 320 für das Optische Pumpen als auch der Gastransport in den Kompressor 410 diskontinuierlich. Dafür wird aus Reservoir 201 über Leitungen 202, 212 und Ventile 210, 213 gerade soviel Gas in die Reinigungseinheit 250 eingestellt, wie beim anschließendem Öffnen von Ventil 214 und Ventil 420 aus dem Optischen Pumpvolumen 320 mit bestimmten Druck in den evakuierten Kompressionsraum 415 einfließt. Der Gasaustausch erfolgt quais momentan, so dass sich sofort in jeder Zelle 323 die neuen, niedrigen Polarisationswerte einstellen. Folglich beginnt, wie in Fig. 16 dargestellt, nach dem Gasaustausch mit Vorteil die Aufpolarisierung mit der für die nun deutlich niedrigere Polarisation typischen hohen Polarisationsraten (vergleiche den steilen Polarisationsanstieg bei Punkt A in Fig. 16). Sie wird zusätzlich im ganzen steiler durch den Wegfall der strömungsbedingten Realxationsrate $\Gamma_{1,\text{ström}}$. Dadurch ist bei diesem "fraktioniertem Optischen Pumpen" der Polarisationszuwachs des Gases während der Zykluszeit im ganzen größer als bei kontinuierlicher Gaszufuhr. Sie führt vom Punkt A zu höherer Endpolarisation B, während bei

kontinuierlicher Gaszufuhr die Polarisation sich von A' nach B' entwickelt.

Mit Vorteil führt daher der oben beschriebene, diskontinuierliche Gasaustausch in Volumen 320 zu höheren Endpolarisationen. Für eine vorbestimmte Förderrate muß der Kompressor mit einer bestimmten Gasmenge gefüllt werden, entsprechend einem durch den Hubraum bestimmten Druck $p_{\text{ein}}$. In der Ausführung der Stand der Technik, wie in Surkau 1995 und Surkau et.al. 1997 beschrieben, stellt sich dieser Druck nach Entpannen des Gases aus dem Volumen zum Optischen Pumpen ein. Der Druck beim Polarisieren ist daher größer, als der Startdruck bei der Kompression. Wie aus Fig. 15 zu entnehmen, werden damit Einbußen in der Endpolarisation in Kauf genommen. In dem hier beschrieben neuen Verfahren bleibt nach der recht kurzen Ausströmphase in den Kompressor bei gleichzeitigem raschen Nachströmen aus der Reinigungseinheit 250 der Druck in den optischen Pumpzellen 323 stabil beim Wert $p_{\text{ein}}$ und wächst so während der Polarisationsphase nicht darüber an. Dadurch werden bei gleicher Förderrate höhere Endpolarisationen erreicht.

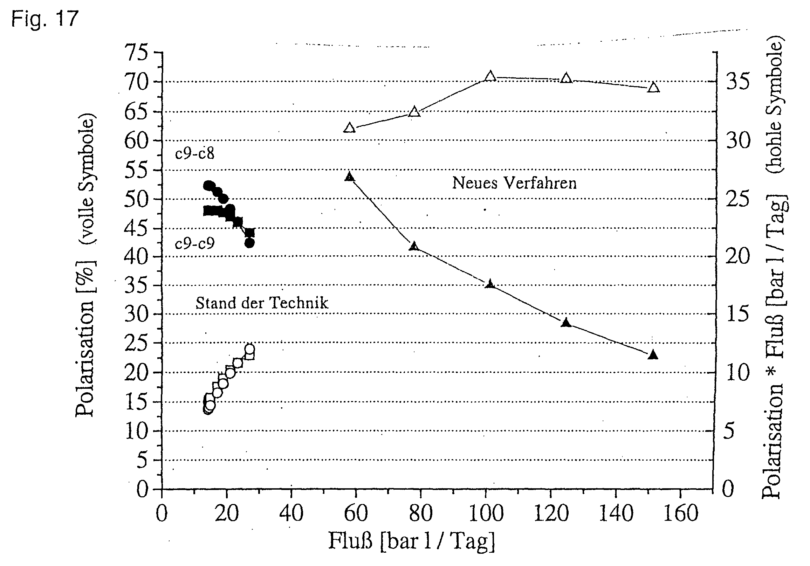

Fig. 17 zeigt im Vergleich die Leistungsfähigkeit des Verfahrens gemäß dem Stand der Technik und gemäß der Erfindung, wobei letzteres in einer vorläufigen Betriebsweise ohne aktivierte Reinigungseinheiten (270) und (402) beschrieben wird. Mit einem Kompressionszykluszeit von 20 s und bei Einsatz von etwa 8 Watt an resonantem Laserlicht auf dem optischen $^3$He$^*$-Übergang C9 wird $^3$He-Gas mit Produktionsraten von 60 bar litern / Tag über 50 % polarisiert. Mit steigender Rate fällt die erreichbare Polarisation gemäß Fig. 17 ab. Das Produkt aus Rate und Endpolarisation steigt aber sogar leicht an und ist ein Maß für die absolute Zahl an polarisierten Atomen, die in nichtpolarisiertem $^3$He gelöst sind. Benötigt die Anwendung also eine möglichst große Zahl an polarisierten $^3$He Atomen, die in beliebieger Menge an Puffergas verdünnt sein kann, dann sind die dargestellten Produktionsraten bei höherem Druck vorteilhaft.

Vergleicht man diese Produktionsraten mit denen der Stand der Technik so ergibt sich in der vorläufigen Betriebsweise bei gleichhoher Endpolarisation von über 50 % bereits eine Flußsteigerung um einen Faktor 5 (Fig. 17). Sieht man es auf höchste Produktionsraten ab, so wurde der Fluß um über eine Größenordnung gesteigert und die Produktionsrate, ausgedrückt in % bar liter / Tag um einen Faktor 5. Bei Einsatz der Reinigungseinheiten (270) und (402) läßt sich zusätzlich ein vorteilhafte Steigerung der Endpolarisation auf Werte über 60 % erreichen.

**Anhang: Formelübersicht**

$$P_{\text{Boltzmann}} = \tanh(\mu_I \, B_T \, / \, kT) \tag{1}$$

$$\partial Br/\partial r/B_0 = G_r < 5 * 10^{-4} \, / \, cm \tag{2}$$

$$T_1 = p \, / \, (G_r^2 * 17000) \, h \, / \, (\text{bar cm}^2) \tag{3}$$

$$\Gamma_{1,W} = 1 \, / \, T_1 = \gamma_W \, O \, / \, V \tag{4}$$

$$P(\tau) = P_0 \cdot \exp(-\tau \, / \, T_1) \approx P_0 \tag{5}$$

$$K_{eff} = P_{aus} / P_{ein}. \tag{6}$$

$$K_0 = V_{hub} / V_{tot}. \tag{7}$$

$$K_{eff} \leq K_0. \tag{8}$$

$$\in = K_{eff}\, V_{tot} / V_{hub} = K_{eff} / K_0. \tag{9}$$

$$\kappa_A = V_L/V_G = V_L / V_{hub} \cdot (1/K_{eff} - 1/K_0)^{-1}. \tag{10}$$

$$\eta = P_{aus} / P_{ein} = 1 - \in\, < 1. \tag{11}$$

$$V_{tot} = 2\pi\, r\, \alpha. \tag{12}$$

$$\in = K_{eff}\, 2\,\pi\, r\, \alpha / V_{hub}. \tag{13}$$

$$\beta = 2\,\pi\, r / V_{hub} = (K_0\, \alpha)^{-1} \tag{14}$$

$$T_{diff} = l\, V / A\, D \tag{15}$$

$$d(\Delta P) / dt = -\, \Delta P / \tau_{diff}. \tag{16}$$

$$\Gamma_{1,diff} = 1/P_2\, dP_2/dt = -\, 1/(2\, P_2)\, d(\Delta P)/dt = \Delta P/(2\, P_2)\, 1/\tau_{diff}. \tag{17}$$

$$f = \pi\, d^4\, (p_2^2 - p_1^2) / (\, 256\, \eta\, l) \tag{18}$$

$$\Gamma_{1,O2} = 1 / T_1 = p_{O2} / (bar\, 2{,}25\, s). \tag{19}$$

$$\partial Br/\partial r / B_0 = G_r < 5 * 10^{-4} / cm \tag{20}$$

$$\Gamma_{1,dip(23°C)} = (p / bar) / (744\, h). \tag{21}$$

**Patentansprüche**

1. Element zur UHV-tauglichen Bewegungsdurchführung, insbesondere in der Ausführungsform als Kompressoren oder Ventile für eine Vorrichtung zur Erzeugung polarisierter Gase, insbesondere von $^3$He-Gas umfassend

   1.1 wenigstens ein in einem Gehäuse bewegliches Bauteil,
   dadurch gekennzeichnet, daß das bewegliche Bauteil derart ausgeführt ist, daß

1.2 das vom Außenraum bewegte Bauteil über einen Zwischenraum in den zu dichtenden Innenraum führt, so daß das Eindringen von flüchtigen Stoffen vom Außenraum in den Innenraum durch Dichtringe weitgehend vermieden wird.

**2.** Element nach Anspruch 1,
dadurch gekennzeichnet, daß der Zwischenraum einen Pumpanschluß umfasst.

**3.** Element nach Anspruch 1 oder 2,
dadurch gekennzeichnet, daß die im Gehäuse beweglichen Bauteile Kolben oder Kolbenstangen von Kompressoren oder Stössel von Ventilen sind.

**4.** Element gemäß einem der Ansprüche 1 - 3,
dadurch gekennzeichnet, daß das Element als Auslaßventil derart ausgebildet ist, daß die Dichtung über einen Hilfsstößel erfolgt, der mit einem gewissen freien Bewegungsspielraum an einen Stößel nach Anspruch 3 gekoppelt ist, derart, daß

4.1. in einer mittleren Stellung des Stößels der Hilfsstößel das Ventil nur durch einen Überdruck auf der Ventilausgangsseite dichtet,
4.2. in einer eingefahrenen Stellung des Stößels der Hilfsstößel zwangsweise das Ventil schließt unabhängig von den Druckverhältnissen,
4.3. in ausgefahrener Stellung des Stößels der Hilfsstößel zwangsweise das Ventil öffnet unabhängig von den Druckverhältissen.

**5.** Element nach einem der Ansprüche 1 bis 4,
dadurch gekennzeichnet, daß der Zwischenraum eine derartige Länge aufweist, daß der Hub des Kolbens oder des Stössels des Elementes stets geringer als die Längserstreckung des Zwischenraumes ist.

**6.** Element nach einem der Ansprüche 1 bis 5,
dadurch gekennzeichnet, daß das Element mehrere nebeneinander angeordnete bewegliche Bauteile umfasst, wobei die den jeweiligen Bauteilen zugeordneten Zwischenräume in leitender Verbindung miteinander stehen.

**7.** Element nach einem der Ansprüche 1 bis 6,
dadurch gekennzeichnet, daß der Zwischenraum mit Hilfe von in einer Nut verlaufenden Gleitdichtungen abgedichtet ist.

**8.** Element nach einem der Ansprüche 1 bis 7,
dadurch gekennzeichnet, daß alle Bauteile des Elementes, die sich in unmittelbarem Kontakt mit dem polarisierten $^3$He oder in dessen Nähe befinden, gering ausgasende und / oder nur schwach magnetische Werkstoffe umfassen.

**9.** Element nach einem der Ansprüche 1 bis 8,
dadurch gekenzeichnet, daß die bewegten, mechanisch beanspruchten Bauteile gering ausgasendes Titan oder Bronze umfassen.

**10.** Element gemäß einem der Ansprüche 1-9,
dadurch gekennzeichnet, daß das Element ein Kompressor ist, der derart ausgebildet ist, daß bei vorgezogenem Kolben das Totvolumen im Zylinderkopf minimiert wird.

**11.** Element gemäß einem der Ansprüche 1-10,
dadurch gekennzeichnet, daß der Antrieb des Kolbens des Kompressors oder des Stössels des Ventiles hydraulisch erfolgt.

**12.** Element gemäß Anspruch 11,
dadurch gekennzeichnet, daß der hydraulische Antrieb des Kolbens zwei im Innern des Kolbens gelegene Druckkammern umfasst.

**13.** Element gemäß einem der Ansprüche 1-10,
dadurch gekennzeichnet, daß der Antrieb des Kompressors einen schnelllaufenden Kurbelwellenantrieb mit Pleuel umfasst.

14. Vorrichtung zur Erzeugung von kernspinpolarisierten Medien, insbesondere 3He-Gas mit

14.1. einer Einrichtung zum optischen Pumpen in einem Niederdruckplasma
14.2. einer Kompressoreinrichtung zur Kompression des polarisierten Mediums sowie
14.3 einem Speichervolumen
    dadurch gekennzeichnet, daß
14.4 die Kompressoreinrichtung wenigstens ein Element gemäß einem der Ansprüche 1-13 umfasst.

15. Vorrichtung gemäß Anspruch 14,
dadurch gekennzeichnet, daß die Ventile der Vorrichtung zur Erzeugung polarisierter Medien Elemente gemäß einem der Ansprüche 1-13 umfassen

16. Vorrichtung gemäß Anspruch 15,
dadurch gekennzeichnet, daß mehrere Ventile der Vorrichtung zur Erzeugung polarisierter Medien zu Ventilblöcken zusammengefasst sind.

17. Vorrichtung gemäß Anspruch 16,
dadurch gekennzeichnet, daß die Ventilblöcke ein gemeinsames Zwischenvakuum für eine Vielzahl von Ventilen umfassen und die einzelnen Zwischenvakuua über Bohrungen miteinander verbunden sind.

18. Vorrichtung gemäß Anspruch 17,
dadurch gekennzeichnet, daß ein oder mehrere Ventilblöcke das Einlaßventil von der optischen Pumpeinrichtung in die Kompressoreinrichtung und/oder das Auslaßventil von der Kompressoreinrichtung in das Speichervolumen umfassen.

19. Vorrichtung gemäß Anspruch 17 oder 18,
dadurch gekennzeichnet, daß ein oder mehrere Ventilblöcke die Ventile für die Evakuierung der optischen Pumpeinrichtung und/oder für die Steuerung der Gasflüsse sowie die Druckmonitore umfassen.

20. Vorrichtung gemäß einem der Ansprüche 14-19,
dadurch gekennzeichnet, daß das Leitungssystem der Vorrichtung für Gastransport und Evakuierung metallringgedichtete, gering ausgasende Aluminiumrohre umfasst.

21. Vorrichtung gemäß einem der Ansprüche 14-20,
dadurch gekennzeichnet, daß die Vorrichtung in Strömungsrichtung des Gases vor und/oder innerhalb und/oder nach der optischen Pumpeinrichtung selektiv absorbierende Getter umfasst.

22. Vorrichtung nach Anspruch 21,
dadurch gekennzeichnet, daß die Getter Verdampfungsgetter sind und schwach relaxierende Substanzen, wie Titan oder Wismut umfassen.

23. Vorrichtung gemäß einem der Ansprüche 14-22,
dadurch gekennzeichnet, daß die Vorrichtung derart ausgebildet ist, daß das Totvolumen im Zylinderkopf, dem Auslaßventil der Kompressoreinrichtung zum Speichervolumen und den Zuleitungen zum Speichervolumen minimiert ist, so daß ein schneller und möglichst vollständiger Gasdurchtritt von der Kompressoreinrichtung zum Speichervolumen erfolgt.

24. Vorrichtung gemäß Anspruch 23,
dadurch gekennzeichnet, daß das Hubvolumen des Kompressors derart gewählt wird, daß der Anteil des im Totvolumen verbleibenden und dort relaxierenden Gases minimiert wird.

25. Vorrichtung gemäß einem der Ansprüche 14-24,
dadurch gekennzeichnet, daß die Kompressoreinrichtung einen Kompressorzylinder umfasst und das Verhältnis des Umfanges des Kompressorzylinders zum Hubvolumen des Kompressors $<1/(30\ cm^2)$, bevorzugt $<1/(100\ cm^2)$, besonders bevorzugt $<1/(300\ cm^2)$ ist.

**26.** Vorrichtung gemäß einem der Ansprüche 14-25
dadurch gekennzeichnet, daß die optische Pumpeinrichtung mindestens eine langgestreckte Zelle für das optisch gepumpte Niederdruckplasma umfasst.

**27.** Vorrichtung gemäß Anspruch 26,
dadurch gekennzeichnet, daß die Zelle die Zirkularpolarisation erhaltende Spiegel umfasst, so daß der Absorptionsweg des Pumplichtes im der Zelle verdoppelt wird.

**28.** Vorrichtung gemäß Anspruch 27,
dadaurch gekennzeichnet, daß die Spiegel als für bestimmte $^3$He-Spektrallinien transmittierende Spiegel ausgebildet sind.

**29.** Vorrichtung gemäß einem der Ansprüche 26-28,
dadurch gekennzeichnet, daß die optische Pumpeinrichtung abbildende optische Elemente umfasst, die außerhalb der langgestreckten Zellen derart angeordnet sind, daß der Strahl der Lichtquelle derart gebündelt wird, daß er innerhalb der Zelle einen möglichst großen und konstanten Querschnitt aufweist, der jedoch stets geringer als der Querschnitt der Zelle ist, so daß keine depolarisierende Reflexion beispielsweise an den Zellwänden auftritt.

**30.** Vorrichtung gemäß einem der Ansprüche 26 - 29,
dadurch gekennzeichnet, daß ein Element zur Bestimmung des Zirkularpolarisationsgrades von Licht vorgesehen ist und der Zirkularpolaristionsgrad aus der Differenz eines maximalen und eines minimalen Spannungswertes, geteilt durch Ihre Summe bestimmt wird, derart, daß das Licht zunächst eine statische $\lambda$ /4-Platte, anschließend ein Flüssigkristallelement und schließlich einen Linearpolarisator durchquert bevor es in einem Photodetektor in Abhängigkeit eines positiven oder negativen Spannungssignales eines der beiden Spannungssignale generiert, wobei das Flüssigkristallelement in Abhängigkeit des positiven oder negativen Spannungssignals als Verzögerungsplatte mit geradzahligen oder ungeradzahligen Vielfachen der Verzögerung der halben Lichtwellenlänge $\lambda$ /2 oder vice versa arbeitet.

**31.** Vorrichtung gemäß einem der Ansprüche 14-30,
dadurch gekennzeichnet, daß die optische Pumpvorrichtung zum Aufrechterhalten des Plasmas mindestens eine Hochfrequenzelektrode umfasst.

**32.** Vorrichtung gemäß einem der Ansprüche 14-31,
dadurch gekennzeichnet, daß die Vorrichtung mindestens ein Pumpsystem umfasst, an das die Zwischenvakuua der Elemente mit beweglichen Bauteilen angeschlossen sind.

**33.** Vorrichtung gemäß Anspruch 32, dadurch gekennzeichnet, daß die Vorrichtung eine Reinigungseinrichtung zum Reinigen des aus den Zwischenvakuua abgepumpten Gases umfaßt, um dieses der Vorrichtung erneut zuführen zu können.

**34.** Verfahren zur Herstellung kernspinpolarisierter Gase, insbesondere $^3$He-Gas, umfassend die Schritte:

    34.1. des optischen Pumpens in einem Niederdruckplasma und der
    34.2. anschließenden mechanischen Kompression des Gases sowie
    34.3. der Zufuhr zu einem Speichervolumen,

dadurch gekennzeichnet, daß bei der mechanischen Kompression das fraktionierte Pumpen angewandt wird.

**35.** Verfahren nach Anspruch 34, dadurch gekennzeichnet, daß das fraktionierte Pumpen mit einem Element gemäß einem der Ansprüche 1-13 durchgeführt wird.

**36.** Verfahren nach einem der Ansprüche 34-35, dadurch gekennzeichnet, daß, die Herstellung der kernspinpolarisierten Gase mit einer Vorrichtung gemäß einem der Ansprüche 14-33 durchgeführt wird.

**37.** Verfahren gemäß einem der Ansprüche 34-36, dadurch
gekennzeichnet, daß die Kompression auf einen Druck < 10 bar mit ein und derselben Kompressoreinrichtung und Zwischenspeichern erfolgt.

**38.** Verfahren gemä einem der Ansprüche 34 - 37, dadurch gekennzeichnet, daß die Zwischenvauua aktiv bepumpt werden.

**39.** Verfahren gemäß einem der Ansprüche 34 - 38, dadurch gekennzeichnet, daß das Gals vor dem optischen Pumpen und/oder während des optischen Pumpens und/oder der Kompression mit Hilfe von Gettereinrichtungen von Störstoffen gereinigt wird.

**40.** Verfahren gemäß einem der Ansprüche 34 - 39, dadurch gekennzeichnet, daß das aus den aktiv bepumpten Zwischenvakuua abgepumpte Edelgas gereinigt und der Vorrichtung zur Erzeugung polarisierter Edelgasse zu-geführt wird.

Fig. 1

Laser

1083,2 nm ⌇ Licht

| | | | |
|---|---|---|---|
| $^3$He-Gas Reservoir | Niederdruck Plasma | Kompression | Speicherung |

EP 1 116 690 A2

Fig. 2

Fig. 3

Fig. 4a

Fig. 4c

Fig. 4b

EP 1 116 690 A2

Fig. 5a

Fig. 5b

Fig. 6a

Fig. 6b

Fig. 7

Fig. 8a

Fig. 8b

EP 1 116 690 A2

Fig. 9b

Fig. 9a

Fig. 9d

Fig. 9c

1012 1020 1030 1023 1022 1012 1040 1021 1024 1000 1014 1024

Fig. 10

Fig. 11a

Fig. 11b

Fig. 12

EP 1 116 690 A2

Fig. 13

Fig. 14a

IF lin.Pol. rot. λ/4

332
331
333
334
336
337
342
$\omega_{ref}$
338
340
339
341
343
346
344
347

Fig. 14b

IF lin.Pol. λ/2 λ/4

332
331
353
354
355
356
357
360
358
360
359
361
364
363
365
366

Fig. 15

Fig. 16

EP 1 116 690 A2

Fig. 17